# EUROPEAN PATENT APPLICATION

(11) **EP 1 671 957 A1**
(43) Date of publication of application: **21.06.2006**
(21) Application number: 04792510.2
(22) Date of filing: 08.10.2004
(51) Int. Cl.: C07D 285/12, C07D 417/04, C07D 417/06, C07D 417/12, A61K 31/433, A61K 31/497, A61K 31/4439, A61P 43/00, A61P 35/00

(54) **THIADIAZOLINE DERIVATIVES**

(30) Priority: 10.10.2003 JP 2003351872; 21.10.2003 JP 2003360263
(71) Applicant: KYOWA HAKKO KOGYO CO., LTD., Chiyoda-ku, Tokyo 100-8185 (JP); FUJI PHOTO FILM CO., LTD., Minamiashigara-shi, Kanagawa-ken 250-0193 (JP)
(72) Inventor: MURAKATA, Chikara Kyowa Hakko Kogyo Co., Ltd., Sunto-gun, Shizuoka 411-8731 (JP); AMISHIRO, Nobuyoshi Kyowa Hakko Kogyo Co., Ltd., Sunto-gun, Shizuoka 411-8731 (JP); INO, Yoji, Kyowa Hakko Kogyo Co., Ltd., Chiyoda-ku, Tokyo 100-8185 (JP); YAMAMOTO, Junichiro Kyowa Hakko Kogyo Co., Ltd., Sunto-gun, Shizuoka 411-8731 (JP); ATSUMI, Toshiyuki Kyowa Hakko Kogyo Co., Ltd., Sunto-gun, Shizuoka 411-8731 (JP); NAKAI, Ryuichiro Kyowa Hakko Kogyo Co., Ltd., Sunto-gun, Shizuoka 411-8731 (JP); NAKANO, Tomohisa Kyowa Hakko Kogyo Co., Ltd., Chiyoda-ku, Tokyo 100-8185 (JP)
(74) Representative: Sternagel, Fleischer, Godemeyer & Partner
(86) International application number: PCT/JP2004/015293
(87) International publication number: WO 2005/035512

(57) **Abstract**

An antitumor agent comprising a thiadiazoline derivative represented by the general formula (I), or a pharmacologically acceptable salt thereof as an active ingredient: 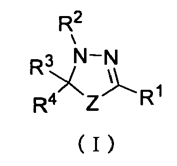 (wherein Z represents a sulfur atom and the like, R¹ represents substituted or unsubstituted lower alkynyl and the like, R² represents a hydrogen atom and the like, R³ represents substituted or unsubstituted lower alkyl and the like, and R⁴ represents substituted or unsubstituted aryl and the like), and the like are provided.

## Description

### Technical Field

The present invention relates to a therapeutic and/or preventive agent for a diseases involving cell proliferation such as tumors, restenosis, cardiac hypertrophy, and immunologic diseases, which comprises a thiadiazoline derivative or a pharmacologically acceptable salt thereof as an active ingredient, and the like.

### Background Art

Pharmaceutical agents such as vinca alkaloids and taxanes, which are clinically important antitumor agents, have an action of binding to microtubules to inhibit the functions of spindles comprising microtubules as structural units. It is known that the functions of spindles are indispensable to localization of centromeres and correct separation of chromosomes at the time of cell division (mitotic phase of cell cycle), and inhibition of the functions thereof leads to inhibition of normal cell division and induce cell death of cancer cells [Biochem. Biophys. Res. Commun., Vol. 263, p.398 (1999)].

The microtubules are involved in maintenance of cell morphology, intracellular substance transport, and axonal transport of nerve fibers, as well as serve as molecular components of mitotic spindles. Accordingly, anticancer agents acting on the microtubule not only have an effect on cancer cells but also adversely affect on normal cells. For example, as side effects unique to the agents acting on the microtubule, peripheral nerve disorders due to the inhibition of the axonal transport of the nerve fibers have been recognized as clinical problems. Therefore, an agent that acts on a molecule, other than the microtubule, which is important for regulation of the spindle function during the mitotic phase of the cell cycle and inhibits the spindle functions in the same manner as existing microtubule-acting anticancer agents, is expected to be a potential novel anticancer agent which avoids the aforementioned side effects derived from the action on the microtubules observed for the existing anticancer agents.

The mitotic kinesins are proteins that are involved in the mitotic spindle regulation, and play an essential role for progression of the mitotic phase in cell cycle. These proteins have a function of moving proteins along microtubules using the energy produced by ATP hydrolysis, and belong to a class of functional proteins generally called "molecular motors". In the mitotic phase, the proteins are deeply involved in extension and maintenance of mitotic spindles, as well as formation of structure called spindle pole body, and further, they regulate progression of normal cell division through the movement of chromosomes along the spindle microtubules.

The mitotic kinesin Eg5 is one of the mitotic kinesins constituting an evolutionarily conserved subfamily. It is known that Eg5 has a function as a bipolar homotetramer molecule, and is involved in the formation of the bipolar spindle structure by crosslinking two of microtubules of the same direction and moving them in the direction toward the + (plus) end to cause sliding of two of the antiparallel microtubules, thereby keep - (minus) ends of microtubules at a distance and separate spindle pole bodies. The above functions of Eg5 were elucidated on the basis of the analysis of the human cells treated with anti-Eg5 antibody and a specific inhibitor [Cell, Vol. 83, p.1159 (1995); J. Cell Biol., Vol. 150, p.975 (2000); Jikken Igaku (Experimental Medicine), Vol. 17, p.439 (1999)].

The gene of human Eg5 was cloned in 1995, and the expression of a full-length human Eg5 recombinant protein by using an insect cell and functional analysis using the resulting protein were reported [Cell, Vol. 83, p.1159 (1995)]. The gene was registered in a public database as GenBank accession numbers: X85137, NM004523 and U37426. A biochemical analysis and structure analysis by crystallization of Eg5 utilizing an N-terminus portion of human Eg5, expressed by using *Escherichia coli* cells, were reported [J. Biological Chemistry, Vol. 276, p.25496 (2001); Chemistry & Biology, Vol. 9, p.989 (2002)], which applied a technique similar to the analysis utilizing Eg5 derived from *Xenopus laevis* having a high homology to the human Eg5 [Proc. Natl. Acad. Sci. USA, Vol. 96, p.9106 (1999); Biochemistry, Vol. 35, p.2365 (1996)].

It is known that the expression of Eg5 in human normal tissues are limited to testis, thymus and the like, and it has been reported, on the basis of results of analysis of tissues from cancer patients, that human Eg5 is more intensely expressed in tumor tissues compared with normal tissues [Proc. Natl. Acad. Sci. USA, Vol. 99, p.4465 (2002), US6414121B1].

As described above, the mitotic kinesin Eg5 is important as a target molecule of a novel mitotic phase acting agent and it is considered that an inhibitor against said molecule is promising as an agent for therapeutic treatment of diseases involving cell proliferation (e.g., tumors, restenosis, cardiac hypertrophy, arthritis, immunologic diseases and the like) [International Patent Publications WO01/98278, WO02/56880, WO02/57244; Trends in Cell Biology, Vol. 12, p.585 (2002)].

As compounds having inhibitory activity against the human Eg5 enzyme, monastrol [Science, Vol. 286, p.971 (1999)], quinazoline derivatives (WO01/98278), phenathiazine derivatives (WO02/57244), triphenylmethane derivatives (WO02/56880), dihydropyrimidine derivatives (WO02/79149; WO02/79169), and the like were reported.

Thiadiazoline derivatives having inhibitory activity against a transcription factor STAT6 activation or those having integrin antagonistic action are known (Japanese Patent Unexamined Publication (KOKAI) No. 2000-229959; WO01/56994), and further, those having an antibacterial activity, ACE inhibitory activity or the like (WO93/22311; Japanese Patent Unexamined Publication (KOKAI) No. 62-53976; J. Bangladesh Chem. Soc., Vol. 5, p.127 (1992)), and those having antitumor activity (WO03/051854, J. Med. Chem., Vol. 44, p.4416 (2001)) are also known.

Further, thiadiazoline derivatives having an alkynyl group or an aromatic heterocyclic group at the 2-position (WO01/56994) and thiadiazoline derivatives having an aryl group at the 2-position (Japanese Patent Unexamined Publication (KOKAI) No. 2000-159756) are known. Furthermore, thiadiazoline derivatives having benzyl group at the 2-position are also known (Chemistry of Heterocyclic Compounds, Vol. 35, p.87 (1999)).

### Disclosure of the Invention

An object of the present invention is to provide a therapeutic and/or preventive agent for a diseases involving cell proliferation such as tumors, restenosis, cardiac hypertrophy, and immunologic diseases, which comprises a thiadiazoline derivative or a pharmacologically acceptable salt thereof as an active ingredient. Another object of the present invention is to provide a thiadiazoline derivative or a pharmacologically acceptable salt thereof which is useful for therapeutic treatment of the aforementioned diseases involving cell proliferation.

The present invention relates to the following (1) to (56).
(1) An antitumor agent comprising a thiadiazoline derivative represented by the general formula (I), or a pharmacologically acceptable salt thereof as an active ingredient: 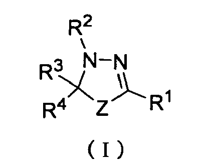 <wherein Z represents a sulfur atom or -S(=O)-, R¹ represents substituted or unsubstituted lower alkyl, substituted or unsubstituted lower alkenyl, substituted or unsubstituted lower alkynyl, substituted or unsubstituted aryl, a substituted or unsubstituted aromatic heterocyclic group, or -C(=W)R⁵ {wherein W represents an oxygen atom or a sulfur atom, and R⁵ represents a hydrogen atom, substituted or unsubstituted lower alkyl, substituted or unsubstituted lower alkenyl,
   -YR⁶ (wherein Y represents an oxygen atom or a sulfur atom, and R⁶ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group), or
   -NR⁷R⁸ [wherein R⁷ and R⁸ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group,
   -OR⁹ (wherein R⁹ has the same meaning as that of the aforementioned R⁶), or -NR¹⁰R¹¹ (wherein R¹⁰ and R¹¹ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group, or R¹⁰ and R¹¹ are combined together with the adjacent nitrogen atom to form a substituted or unsubstituted heterocyclic group), or R⁷ and R⁸ are combined together with the adjacent nitrogen atom to form a substituted or unsubstituted heterocyclic group]},
   R² represents a hydrogen atom, substituted or unsubstituted lower alkyl, or
   -C(=W¹)R¹² [wherein W¹ represents an oxygen atom or a sulfur atom, R¹² represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, -Y¹R¹³ (wherein Y¹ represents an oxygen atom or a sulfur atom, and R¹³ represents substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group), or -NR¹⁴R¹⁵ (wherein R¹⁴ and R¹⁵ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group, or R¹⁴ and R¹⁵ are combined together with the adjacent nitrogen atom to form a substituted or unsubstituted heterocyclic group)],
   R³ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group, and
   R⁴ represents substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group,
   or R³ and R⁴ are combined together to represent
   -(CR^{16A}R^{16B})ₘ₁-Q-(CR^{16C}R^{16D})ₘ₂- {wherein Q represents a single bond, substituted or unsubstituted phenylene, or cycloalkylene, m1 and m2 are the same or different, and each represents an integer of 0 to 4, with proviso that m1 and m2 are not 0 at the same time,
   R^{16A}, R^{16B}, R^{16C} and R^{16D} are the same or different, and represent a hydrogen atom, halogen, substituted or unsubstituted lower alkyl, -OR¹⁷ [wherein R¹⁷ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, -CONR¹⁸R¹⁹ (wherein R¹⁸ and R¹⁹ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group, or R¹⁸ and R¹⁹ are combined together with the adjacent nitrogen atom to form a substituted or unsubstituted heterocyclic group),
   -SO₂NR²⁰R²¹ (wherein R²⁰ and R²¹ have the same meanings as those of the aforementioned R¹⁸ and R¹⁹, respectively), or -COR²² (wherein R²² represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group)], -NR²³R²⁴ [wherein R²³ and R²⁴ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, -COR²⁵ (wherein R²⁵ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, substituted or unsubstituted lower alkoxy, substituted or unsubstituted aryloxy, amino, substituted or unsubstituted lower alkylamino, di-(substituted or unsubstituted lower alkyl)amino, or substituted or unsubstituted arylamino), or -SO₂R²⁶ (wherein R²⁶ represents substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group), or R²³ and
   R²⁴ are combined together with the adjacent nitrogen atom to form a substituted or unsubstituted heterocyclic group], or -CO₂R²⁷ (wherein R²⁷ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group), or R^{16A} and R^{16B}, or R^{16C} and R^{16D} are combined together to represent an oxygen atom, and when m1 or m2 is an integer of 2 or more, any two of R^{16A}, R^{16B}, R^{16C} and
   R^{16D} may be the same or different, and any two of R^{16A}, R^{16B}, R^{16C} and R^{16D} which are bound to the adjacent two carbon atoms may combine together to form a bond}>.
(2) The antitumor agent according to (1), wherein R¹ is substituted or unsubstituted lower alkynyl, substituted or unsubstituted aryl, or a substituted or unsubstituted aromatic heterocyclic group.
(3) The antitumor agent according to (1), wherein R¹ is substituted or unsubstituted lower alkyl, substituted or unsubstituted lower alkenyl, or -C(=W)R⁵ (wherein W and R⁵ have the same meanings as those mentioned above).
(4) The antitumor agent according to (1), wherein R¹ is substituted or unsubstituted aryl, or a substituted or unsubstituted aromatic heterocyclic group.
(5) The antitumor agent according to (1), wherein R¹ is substituted or unsubstituted aryl.
(6) The antitumor agent according to (1), wherein R¹ is substituted or unsubstituted lower alkynyl.
(7) The antitumor agent according to (1), wherein R¹ is substituted or unsubstituted lower alkyl, or substituted or unsubstituted lower alkenyl.
(8) The antitumor agent according to any one of (1) to (7), wherein R² is a hydrogen atom, substituted or unsubstituted lower alkyl, or -C(=W¹)R¹² (wherein W¹ and R¹² have the same meanings as those mentioned above, respectively).
(9) The antitumor agent according to any one of (1) to (7), wherein R² is -C(=W¹)R¹² (wherein W¹ and R¹² have the same meanings as those mentioned above, respectively).
(10) The antitumor agent according to (8) or (9), wherein R¹² is substituted or unsubstituted lower alkyl, substituted or unsubstituted lower alkenyl, substituted or unsubstituted lower alkynyl, or substituted or unsubstituted cycloalkyl.
(11) The antitumor agent according to (8) or (9), wherein R¹² is substituted or unsubstituted lower alkyl.
(12) The antitumor agent according to (8) or (9), wherein R¹² is lower alkyl.
(13) The antitumor agent according to any one of (8) to (12), wherein W¹ is an oxygen atom.
(14) The antitumor agent according to any one of (1) to (13), wherein R³ is substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group.
(15) The antitumor agent according to any one of (1) to (13), wherein R³ is substituted or unsubstituted lower alkyl.
(16) The antitumor agent according to any one of (1) to (13), wherein R³ is substituted lower alkyl.
(17) The antitumor agent according to any one of (1) to (16), wherein R⁴ is substituted or unsubstituted cycloalkyl, substituted or unsubstituted aryl, or a substituted or unsubstituted heterocyclic group.
(18) The antitumor agent according to any one of (1) to (16), wherein R⁴ is substituted or unsubstituted aryl, or a substituted or unsubstituted heterocyclic group.
(19) The antitumor agent according to any one of (1) to (16), wherein R⁴ is substituted or unsubstituted phenyl, or substituted or unsubstituted thienyl.
(20) The antitumor agent according to any one of (1) to (13), wherein R³ and R⁴ are combined together to represent -(CR^{16A}R^{16B})ₘ₁-Q-(CR^{16C}R^{16D})ₘ₂- (wherein Q, R^{16A}, R^{16B}, R^{16C}, R^{16D}, m1 and m2 have the same meanings as those mentioned above, respectively).
(21) The antitumor agent according to any one of (1) to (13), wherein R³ and R⁴ are combined together to represent -(CH₂)ₘ₁-Q-(CH₂)m₂- (wherein Q, m1 and m2 have the same meanings as those mentioned above, respectively).
(22) The antitumor agent according to (20) or (21), wherein Q is substituted or unsubstituted phenylene.
(23) A mitotic kinesin Eg5 inhibitor containing the thiadiazoline derivative or a pharmacologically acceptable salt thereof according to any one of (1) to (22) as an active ingredient.
(24) A thiadiazoline derivative represented by the formula (IA) or a pharmacologically acceptable salt thereof: 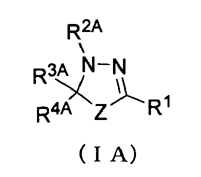 {wherein Z has the same meaning as that mentioned above, R¹ has the same meaning as that mentioned above,
   (A) when R¹ is substituted or unsubstituted lower alkyl, substituted or unsubstituted lower alkenyl, or -C(=W)R⁵ (wherein W and R⁵ have the same meanings as those mentioned above, respectively), R^{2A}, R^{3A} and R^{4A} have the same meanings as those of the aforementioned R², R³ and R⁴ (with proviso that Z^{A} is a sulfur atom, R¹ is benzyl, R^{2A} is acetyl, one of R³ and R^{4A} is methyl, and the other of R³ and R^{4A} is not 2-oxopropyl), respectively
   (B) when R¹ is substituted or unsubstituted lower alkynyl, or a substituted or unsubstituted aromatic heterocyclic group, R^{2A} and R^{3A} have the same meanings as those of the aforementioned R² and R³, respectively, and R^{4A} represents substituted or unsubstituted aryl, or a substituted or unsubstituted heterocyclic group, and
   (C) when R¹ is substituted or unsubstituted aryl, R^{2A} represents -C(=W)R¹² (wherein W and R¹² have the same meanings as those mentioned above, respectively), R^{3A} represents -(CH₂)ₖNHSPO₂R^{3B} [wherein k represents an integer of 1 to 6, and R^{3B} represents substituted or unsubstituted lower alkyl, substituted or unsubstituted lower alkenyl, substituted or unsubstituted lower alkynyl, or -NR^{7B}R^{8B} (wherein R^{7B} and R^{8B} have the same meanings as those of the aforementioned R⁷ and R⁸, respectively)], -(CH₂)ₖNR^{7C}R^{8C} (wherein k has the same meaning as that mentioned above, and R^{7C} and R^{8C} have the same meanings as those of the aforementioned R⁷ and
      R⁸, respectively), or -(CH₂)ₖNHC(=O)R^{7D} (wherein k has the same meaning as that mentioned above, and R^{7D} has the same meaning as that of the aforementioned R⁷), and R^{4A} has the same meaning as that of the aforementioned R⁴}.
(25) The thiadiazoline derivative or a pharmacologically acceptable salt thereof according to (24), wherein Z is a sulfur atom.
(26) The thiadiazoline derivative or a pharmacologically acceptable salt thereof according to (24) or (25), wherein R¹ is substituted or unsubstituted lower alkynyl, substituted or unsubstituted aryl, or a substituted or unsubstituted aromatic heterocyclic group.
(27) The thiadiazoline derivative or a pharmacologically acceptable salt thereof according to (24) or (25), wherein R¹ is substituted or unsubstituted aryl.
(28) The thiadiazoline derivative or a pharmacologically acceptable salt thereof according to (24) or (25), wherein R¹ is substituted or unsubstituted phenyl.
(29) The thiadiazoline derivative or a pharmacologically acceptable salt thereof according to (24) or (25), wherein R¹ is substituted or unsubstituted lower alkynyl.
(30) The thiadiazoline derivative or a pharmacologically acceptable salt thereof according to (24) or (25), wherein R¹ is substituted lower alkyl.
(31) The thiadiazoline derivative or a pharmacologically acceptable salt thereof according to (24) or (25), wherein R¹ is -C(=W)R⁵ (wherein W and R⁵ have the same meanings as those mentioned above, respectively).
(32) The thiadiazoline derivative or a pharmacologically acceptable salt thereof according to (31), wherein W is an oxygen atom.
(33) The thiadiazoline derivative or a pharmacologically acceptable salt thereof according to (31) or (32), wherein R⁵ is -NR⁷R⁸ (wherein R⁷ and R⁸ have the same meanings as those mentioned above, respectively).
(34) The thiadiazoline derivative or a pharmacologically acceptable salt thereof according to any one of (24) to (33), wherein R^{2A} is -C(=O)R¹² (wherein R¹² have the same meanings as those mentioned above).
(35) The thiadiazoline derivative or a pharmacologically acceptable salt thereof according to (34), wherein R¹² is lower alkyl.
(36) The thiadiazoline derivative or a pharmacologically acceptable salt thereof according to any one of (24) to (35), wherein R^{3A} is substituted or unsubstituted lower alkyl.
(37) The thiadiazoline derivative or a pharmacologically acceptable salt thereof according to any one of (24) to (35), wherein R^{3A} is -(CH₂)ₖNHSO₂R^{3B} (wherein k and R^{3B} have the same meanings as those mentioned above, respectively),
   -(CH₂)ₖNR^{7C}R^{8C} (wherein k, R^{7C} and R^{8C} have the same meanings as those mentioned above, respectively), or -(CH₂)ₖNHC(=O)R^{7D} (wherein k and R^{7D} have the same meanings as those mentioned above, respectively).
(38) The thiadiazoline derivative or a pharmacologically acceptable salt thereof according to any one of (24) to (35), wherein R^{3A} is -(CH₂)ₖNHSO₂R^{3B} (wherein k and R^{3B} have the same meanings as those mentioned above, respectively).
(39) The thiadiazoline derivative or a pharmacologically acceptable salt thereof according to any one of (24) to (38), wherein R^{4A} is substituted or unsubstituted aryl, or a substituted or unsubstituted aromatic heterocyclic group.
(40) The thiadiazoline derivative or a pharmacologically acceptable salt thereof according to any one of (24) to (38), wherein R^{4A} is substituted or unsubstituted aryl.
(41) The thiadiazoline derivative or a pharmacologically acceptable salt thereof according to any one of (24) to (38), wherein R^{4A} is substituted or unsubstituted phenyl, or substituted or unsubstituted thienyl.
(42) The thiadiazoline derivative or a pharmacologically acceptable salt thereof according to any one of (24) to (38), wherein R^{4A} is phenyl.
(43) A medicament comprising the thiadiazoline derivative or a pharmacologically acceptable salt thereof according to any one of (24) to (42) as an active ingredient.
(44) A mitotic kinesin Eg5 inhibitor comprising the thiadiazoline derivative or a pharmacologically acceptable salt thereof according to any one of (24) to (42) as an active ingredient.
(45) A therapeutic agent for a disease involving cell proliferation comprising the thiadiazoline derivative or a pharmacologically acceptable salt thereof according to any one of (24) to (42) as an active ingredient.
(46) An antitumor agent comprising the thiadiazoline derivative or a pharmacologically acceptable salt thereof according to any one of (24) to (42) as an active ingredient.
(47) A method for therapeutic and/or preventive treatment of a malignant tumor which comprises administering an effective amount of the thiadiazoline derivative or a pharmacologically acceptable salt thereof according to any one of (1) to (22).
(48) A method for inhibiting a mitotic kinesin Eg5 which comprises administering an effective amount of the thiadiazoline derivative or a pharmacologically acceptable salt thereof according to any one of (1) to (22).
(49) Use of the thiadiazoline derivative or a pharmacologically acceptable salt thereof according to any one of (1) to (22) for the manufacture of the antitumor agent.
(50) Use of the thiadiazoline derivative or a pharmacologically acceptable salt thereof according to any one of (1) to (22) for the manufacture of the mitotic kinesin Eg5 inhibitor.
(51) A method for inhibiting a mitotic kinesin Eg5 which comprises administering an effective amount of the thiadiazoline derivative or a pharmacologically acceptable salt thereof according to any one of (24) to (42).
(52) A method for therapeutic and/or preventive treatment of a disease involving cell proliferation which comprises administering an effective amount of the thiadiazoline derivative or a pharmacologically acceptable salt thereof according to any one of (24) to (42).
(53) A method for therapeutic and/or preventive treatment of a malignant tumor which comprises administering an effective amount of the thiadiazoline derivative or a pharmacologically acceptable salt thereof according to any one of (24) to (42).
(54) Use of the thiadiazoline derivative or a pharmacologically acceptable salt thereof according to any one of (24) to (42) for the manufacture of the mitotic kinesin Eg5 inhibitor.
(55) Use of the thiadiazoline derivative or a pharmacologically acceptable salt thereof according to any one of (24) to (42) for the manufacture of the therapeutic agent for a disease involving cell proliferation.
(56) Use of the thiadiazoline derivative or a pharmacologically acceptable salt thereof according to any one of (24) to (42) for the manufacture of the antitumor agent.

### Best Mode for Carrying out the Invention

Hereinafter, compounds represented by the general formulas (I) and (IA) are referred to as "Compound (I)" and "Compound (IA)", respectively. The compounds having the other formula numbers are referred to in the same manner.

In the definition of each group of the general formula (I) and the general formula (IA),
(i) Examples of the lower alkyl moiety in the lower alkyl, the lower alkoxy, the lower alkylamino, and the di-(lower alkyl)amino include straight or branched chain alkyl having 1 to 10 carbon atoms, for example, methyl, ethyl, propyl, isopropyl, butyl, isobutyl, sec-butyl, tert-butyl, pentyl, isopentyl, neopentyl, hexyl, heptyl, octyl, nonyl, decyl and the like. The two lower alkyl moieties in the di-(lower alkyl)amino may be the same or different.
(ii) Examples of the lower alkenyl include straight or branched chain alkenyl having 2 to 10 carbon atoms, for example, vinyl, allyl, 1-propenyl, butenyl, pentenyl, hexenyl, heptenyl, octenyl, nonenyl, decenyl and the like.
(iii) Examples of the lower alkynyl include straight or branched chain alkynyl having 2 to 10 carbon atoms, for example, ethynyl, propynyl, butynyl, pentynyl, hexynyl, heptynyl, octynyl, nonynyl, decynyl and the like.
(iv) Examples of the cycloalkyl include cycloalkyl having 3 to 8 carbon atoms, for example, cyclopropyl, cyclobutyl, cyclopentyl, cyclohexyl, cycloheptyl, cyclooctyl and the like.
(v) Examples of the aryl and the aryl moiety in the aryloxy and arylamino include phenyl, naphthyl and the like.
(vi) Examples of the aromatic heterocyclic group include a 5- or 6-membered monocyclic aromatic heterocyclic group containing at least one atom selected from a nitrogen atom, an oxygen atom and a sulfur atom, and a bicyclic or tricyclic condensed aromatic heterocyclic group comprising 3- to 8-membered rings and containing at least one atom selected from a nitrogen atom, an oxygen atom and a sulfur atom, and the like, for example, furyl, thienyl, benzothienyl, pyrrolyl, pyridyl, pyrazinyl, imidazolyl, pyrazolyl, triazolyl, thiazolyl, isothiazolyl, thiadiazolyl, oxazolyl, oxadiazolyl, pyrimidinyl, indolyl, isoindolyl, benzothiazolyl, benzimidazolyl, benzotriazolyl, quinolyl, isoquinolyl, quinazolinyl, pyranyl, and the like.
(vii) Examples of the heterocyclic group include an aliphatic heterocyclic group, the aforementioned aromatic heterocyclic group and the like. Examples of the aliphatic heterocyclic group include a 5- or 6-membered monocyclic aliphatic heterocyclic group containing at least one atom selected from a nitrogen atom, an oxygen atom and a sulfur atom, and a bicyclic or tricyclic condensed aliphatic heterocyclic group comprising 4- to 8-membered rings and containing at least one atom selected from a nitrogen atom, an oxygen atom and a sulfur atom and the like, for example, pyrrolidinyl, imidazolidinyl, piperidinyl, piperazinyl, morpholinyl, thiomorpholinyl, piperidino, morpholino, oxazolinyl, dioxolanyl, tetrahydropyranyl, and the like.
(viii) Examples of the heterocyclic group formed together with the adjacent nitrogen atom include an aliphatic heterocyclic group containing at least one nitrogen atom, and the like. Said aliphatic heterocyclic group containing at least one nitrogen atom may contain an oxygen atom, a sulfur atom or another nitrogen atom, and examples thereof include, for example, pyrrolidinyl, morpholino, thiomorpholino, pyrazolidinyl, piperidino, piperazinyl, homopiperazinyl, aziridinyl, azetidinyl, azolidinyl, perhydroazepinyl, perhydroazocinyl, succinimido, phthalimido, pyrrolidonyl, glutarimido, piperidonyl, and the like.
(ix) Examples of the cycloalkylene include a cycloalkylene having 3 to 8 carbon atoms, for example, cyclopropylene, cyclobutylene, cyclopentylene, cyclohexylene, cycloheptylene, cyclooctylene, and the like, and examples of the phenylene include 1,2-phenylene, 1,3-phenylene, and 1,4-phenylene.
(x) The halogen means each atom of fluorine, chlorine, bromine, and iodine.
(xi) The substituent in the substituted lower alkyl, the substituted lower alkoxy, the substituted lower alkenyl, the substituted lower alkynyl, the substituted cycloalkyl, the substituted lower alkylamino, and the substituted di-(lower alkyl)amino may be the same or different in number of 1 to substitutable number, preferably 1 to 3 substituent(s), and includes halogen, hydroxy, oxo, nitro, azido, cyano,
   substituted or unsubstituted cycloalkyl [the substituent (b) in said substituted cycloalkyl may be the same or different in number of 1 to 3 substituent (s), and includes
   halogen, hydroxy, oxo, carboxy, cyano,
   substituted or unsubstituted lower alkoxy (the substituent (a) in said substituted lower alkoxy may be the same or different in number of 1 to 3 substituent (s), and includes
   halogen, hydroxy, oxo, carboxy, lower alkoxy, lower alkanoyloxy, amino, lower
   alkylamino, di-(lower alkyl)amino, aryl, a heterocyclic group, and the like), lower alkanoyloxy,
   substituted or unsubstituted lower alkylthio (the substituent in said substituted lower alkylthio has the same meaning as that of the aforementioned substituent (a) in the substituted lower alkoxy),
   aryl, aryloxy, a heterocyclic group, amino,
   substituted or unsubstituted lower alkylamino (the substituent in said substituted lower alkyl has the same meaning as that of the aforementioned substituent (a) in the substituted lower alkoxy),
   di-(substituted or unsubstituted lower alkyl)amino (the substituent in said substituted lower alkyl has the same meaning as that of the aforementioned
   substituent (a) in the substituted lower alkoxy), and the like], substituted or unsubstituted aryl (the substituent in said substituted aryl has the same meaning as that of the after-mentioned substituent (xii) in the substituted aryl), a substituted or unsubstituted heterocyclic group (the substituent in said substituted heterocyclic group has the same meaning as that of the after-mentioned substituent (xiii) in the substituted heterocyclic group),
   -CONR²⁸R²⁹ <wherein R²⁸ and R²⁹ are the same or different, and represent
   a hydrogen atom, hydroxy,
   substituted or unsubstituted lower alkyl {the substituent (c) in the substituted lower alkyl may be the same or different in number of 1 to substitutable number,
   preferably 1 to 3 substituent(s), and includes
   halogen, hydroxy, oxo, carboxy, cyano,
   substituted or unsubstituted lower alkoxy (the substituent in said substituted lower alkoxy has the same meaning as that of the aforementioned substituent (b) in the substituted cycloalkyl),
   substituted or unsubstituted lower alkylthio (the substituent in said substituted lower alkylthio has the same meaning as that of the aforementioned substituent (b) in the substituted cycloalkyl),
   substituted or unsubstituted lower alkylsulfonyl (the substituent in said substituted lower alkylsulfonyl has the same meaning as that of the aforementioned substituent (b) in the substituted cycloalkyl),
   substituted or unsubstituted aryl (the substituent in said substituted aryl has the same meaning as that of the after-mentioned substituent (xii) in the substituted aryl),
   a substituted or unsubstituted heterocyclic group (the substituent in said substituted heterocyclic group has the same meaning as that of the
   after-mentioned substituent (xiii) in the substituted heterocyclic group), substituted or unsubstituted aryloxy (the substituent in said substituted lower aryloxy has the same meaning as that of the after-mentioned substituent (xii) in the substituted aryl), -NR³⁰R³¹ [wherein R³⁰ and R³¹ are
   the same or different, and represent
   a hydrogen atom,
   substituted or unsubstituted lower alkyl (the substituent in said
   substituted lower alkyl has the same meaning as that of the aforementioned substituent (b) in the substituted cycloalkyl), substituted or unsubstituted lower alkenyl (the substituent in said substituted lower alkenyl has the same meaning as that of the aforementioned substituent (b) in the substituted cycloalkyl), substituted or unsubstituted lower alkynyl (the substituent in said substituted lower alkynyl has the same meaning as that of the aforementioned substituent (b) in the substituted cycloalkyl), substituted or unsubstituted cycloalkyl (the substituent in said substituted cycloalkynyl has the same meaning as that of the aforementioned substituent (b) in the substituted cycloalkyl), substituted or unsubstituted substituted aryl (the substituent in said substituted aryl has the same meaning as that of the after-mentioned substituent (xii) in the substituted aryl),
   a substituted or unsubstituted heterocyclic group (the substituent in said substituted heterocyclic group has the same meaning as that of the after-mentioned substituent (xiii) in the substituted heterocyclic group), or substituted or unsubstituted lower alkylsulfonyl (the substituent in said substituted lower alkylsulfonyl has the same meaning as that of the aforementioned substituent (b) in the substituted cycloalkyl), or
   R³⁰ and R³¹ are combined together with the adjacent nitrogen atom to form a substituted or unsubstituted heterocyclic group (the substituent in said substituted heterocyclic group formed together with the adjacent nitrogen atom has the same meaning as that of the after-mentioned substituent (xiii) in the substituted heterocyclic group formed together with the adjacent nitrogen atom)], and the like},
   substituted or unsubstituted lower alkenyl (the substituent in said substituted lower alkenyl has the same meaning as that of the aforementioned substituent (b) in the substituted cycloalkyl),
   substituted or unsubstituted lower alkynyl (the substituent in said substituted lower alkynyl has the same meaning as that of the aforementioned substituent (b) in the substituted cycloalkyl),
   substituted or unsubstituted cycloalkyl (the substituent in said substituted cycloalkyl has the same meaning as that of the aforementioned substituent (b) in the substituted cycloalkyl),
   substituted or unsubstituted aryl (the substituent in said substituted aryl has the same meaning as that of the after-mentioned substituent (xii) in the substituted aryl), or
   a substituted or unsubstituted heterocyclic group (the substituent in said substituted heterocyclic group has the same meaning as that of the
   after-mentioned substituent (xiii) in the substituted heterocyclic group), or R²⁸ and R²⁹ are combined together with the adjacent nitrogen atom to form a substituted or unsubstituted heterocyclic group (the substituent in said substituted heterocyclic group formed together with the adjacent nitrogen atom has the same meaning as that of the after-mentioned substituent (xiii) in the substituted heterocyclic group formed together with the adjacent nitrogen atom)>,
-CO₂R³² [wherein R³² represents
a hydrogen atom,
substituted or unsubstituted lower alkyl (the substituent in said substituted lower alkyl has the same meaning as that of the aforementioned substituent (c) in the substituted lower alkyl),
substituted or unsubstituted lower alkenyl (the substituent in said substituted lower alkenyl has the same meaning as that of the aforementioned substituent (c) in the substituted lower alkyl),
substituted or unsubstituted lower alkynyl (the substituent in said substituted lower alkynyl has the same meaning as that of the aforementioned substituent (c) in the substituted lower alkyl),
substituted or unsubstituted cycloalkyl (the substituent in said substituted cycloalkyl has the same meaning as that of the aforementioned substituent (c) in the substituted lower alkyl),
substituted or unsubstituted aryl (the substituent in said substituted aryl has the same meaning as that of the after-mentioned substituent (xii) in the substituted aryl), or
a substituted or unsubstituted heterocyclic group (the substituent in said substituted heterocyclic group has the same meaning as that of the after-mentioned substituent (xiii) in the substituted heterocyclic group)],
-COR³³ (wherein R³³ has the same meaning as that of the aforementioned R³²), -NR³⁴R³⁵ <wherein R³⁴ and R³⁵ may be the same or different, and represent
a hydrogen atom,
substituted or unsubstituted lower alkyl {the substituent (d) in said substituted lower alkyl may be the same or different in number of 1 to substitutable number,
preferably 1 to 3 substituent (s), and includes
halogen, hydroxy, oxo, carboxy, cyano,
substituted or unsubstituted lower alkoxy (the substituent in said substituted lower alkoxy has the same meaning as that of the aforementioned substituent (c) in the substituted alkyl),
substituted or unsubstituted lower alkylthio (the substituent in said substituted lower alkylthio has the same meaning as that of the aforementioned substituent (c) in the substituted alkyl),
substituted or unsubstituted aryl (the substituent in said substituted aryl has the same meaning as that of the after-mentioned substituent (xii) in the substituted aryl),
a substituted or unsubstituted heterocyclic group (the substituent in said substituted heterocyclic group has the same meaning as that of the
after-mentioned substituent (xiii) in the substituted heterocyclic group), substituted or unsubstituted aryloxy (the substituent in said substituted aryloxy has the same meaning as that of the after-mentioned substituent (xii) in the substituted aryl),
-O (CH₂CH₂O)ₙR³⁶ (wherein n represents an integer of 1 to 15, and R³⁶ represents lower alkyl),
·SO₂R³⁷ [wherein R³⁷ represents
substituted or unsubstituted lower alkyl (the substituent in said substituted lower alkyl has the same meaning as that of the aforementioned substituent (c) in the substituted alkyl), lower alkenyl, lower alkynyl, substituted or unsubstituted aryl (the substituent in said substituted aryl has the same meaning as that of the after-mentioned substituent (xii) in the substituted aryl), a substituted or unsubstituted heterocyclic group (the substituent in said substituted heterocyclic group has the same meaning as that of the after-mentioned substituent
(xiii) in the substituted heterocyclic group), amino, lower alkylamino, or di-(lower alkyl)amino],
-NR³⁸R³⁹ (wherein R³⁸ and R³⁹ have the same meanings as those of the aforementioned R³⁰ and R³¹, respectively), and the like},
substituted or unsubstituted lower alkenyl (the substituent in said substituted lower alkenyl has the same meaning as that of the aforementioned substituent (c) in the substituted lower alkyl),
substituted or unsubstituted lower alkynyl (the substituent in said substituted lower alkynyl has the same meaning as that of the aforementioned substituent (c) in the substituted lower alkyl),
substituted or unsubstituted cycloalkyl (the substituent in said substituted lower cycloalkyl has the same meaning as that of the aforementioned substituent (c) in the substituted lower alkyl),
substituted or unsubstituted aryl (the substituent in said substituted aryl has the same meaning as that of the after-mentioned substituent (xii) in the substituted aryl),
a substituted or unsubstituted heterocyclic group (the substituent in said substituted heterocyclic group has the same meaning as that of the after-mentioned substituent (xiii) in the substituted heterocyclic group), -COR⁴⁰ {wherein R⁴⁰ represents
a hydrogen atom,
substituted or unsubstituted lower alkyl (the substituent in said substituted lower alkyl has the same meaning as that of the aforementioned substituent (c) in the substituted lower alkyl),
substituted or unsubstituted lower alkenyl (the substituent in said substituted lower alkenyl has the same meaning as that of the aforementioned substituent (c) in the substituted lower alkyl),
substituted or unsubstituted lower alkynyl (the substituent in said substituted lower alkynyl has the same meaning as that of the aforementioned substituent (c) in the substituted lower alkyl),
substituted or unsubstituted cycloalkyl (the substituent in said substituted cycloalkyl has the same meaning as that of the aforementioned substituent (c) in the substituted lower alkyl),
substituted or unsubstituted aryl (the substituent in said substituted aryl has the same meaning as that of the after-mentioned substituent (xii) in the substituted aryl),
a substituted or unsubstituted heterocyclic group (the substituent in said substituted heterocyclic group has the same meaning as that of the after-mentioned substituent (xiii) in the substituted heterocyclic group),
-NR⁴¹R⁴² (wherein R⁴¹ and R⁴² have the same meanings as those of the aforementioned R³⁰ and R³¹, respectively), or
-OR⁴³ [wherein R⁴³ represents
a hydrogen atom,
substituted or unsubstituted lower alkyl (the substituent in said substituted lower alkyl has the same meaning as that of the aforementioned substituent (c) in the substituted lower alkyl), substituted or unsubstituted lower alkenyl (the substituent in said substituted lower alkenyl has the same meaning as that of the aforementioned substituent (c) in the substituted lower alkyl), substituted or unsubstituted lower alkynyl (the substituent in said substituted lower alkynyl has the same meaning as that of the aforementioned substituent (c) in the substituted lower alkyl), substituted or unsubstituted cycloalkyl (the substituent in said substituted cycloalkyl has the same meaning as that of the aforementioned substituent (c) in the substituted lower alkyl), substituted or unsubstituted aryl (the substituent in said substituted aryl has the same meaning as that of the after-mentioned substituent (xii) in the substituted aryl), or
a substituted or unsubstituted heterocyclic group (the substituent in said substituted heterocyclic group has the same meaning as that of the after-mentioned substituent (xiii) in the substituted heterocyclic group)]}, or
-SO₂R⁴⁴ (wherein R⁴⁴ has the same meaning as that of the aforementioned R⁴⁰), or R³⁴ and R³⁵ are combined together with the adjacent nitrogen atom to form a heterocyclic group, or a substituted heterocyclic group (the substituent in said substituted heterocyclic group formed together with the adjacent nitrogen atom has the same meaning as that of the after-mentioned substituent (xiii) in the substituted heterocyclic group formed together with the adjacent nitrogen atom)>,
-N+R⁴⁵R⁴⁶R⁴⁷X- (wherein R⁴⁵ and R⁴⁶ may be the same or different, and represent lower alkyl, or R⁴⁵ and R⁴⁶ are combined together with the adjacent nitrogen atom to form a heterocyclic group, R⁴⁷ represents lower alkyl, and X represents each atom of chlorine, bromine and iodine),
-OR⁴⁸ [wherein R⁴⁸ represents
substituted or unsubstituted lower alkyl (the substituent in said substituted lower alkyl has the same meaning as that of the aforementioned substituent (d) in the substituted lower alkyl),
substituted or unsubstituted lower alkenyl (the substituent in said substituted lower alkenyl has the same meaning as that of the aforementioned substituent (c) in the substituted lower alkyl),
substituted or unsubstituted lower alkynyl (the substituent in said substituted lower alkynyl has the same meaning as that of the aforementioned substituent (c) in the substituted lower alkyl),
substituted or unsubstituted cycloalkyl (the substituent in said substituted cycloalkyl has the same meaning as that of the aforementioned substituent (c) in the substituted lower alkyl),
substituted or unsubstituted aryl (the substituent in said substituted aryl has the same meaning as that of the after-mentioned substituent (xii) in the substituted aryl), or
a substituted or unsubstituted heterocyclic group (the substituent in said substituted heterocyclic group has the same meaning as that of the
after-mentioned substituent (xiii) in the substituted heterocyclic group)],
-SR⁴⁹ (wherein R⁴⁹ has the same meaning as that of the aforementioned R⁴⁸),
-SO₂R⁵⁰ [wherein R⁵⁰ represents
substituted or unsubstituted lower alkyl (the substituent in said substituted lower alkyl has the same meaning as that of the aforementioned substituent (d) in the substituted lower alkyl),
substituted or unsubstituted lower alkenyl (the substituent in said substituted lower alkenyl has the same meaning as that of the aforementioned substituent (c) in the substituted lower alkyl),
substituted or unsubstituted lower alkynyl (the substituent in said substituted lower alkynyl has the same meaning as that of the aforementioned substituent (c) in the substituted lower alkyl),
substituted or unsubstituted cycloalkyl (the substituent in said substituted cycloalkyl has the same meaning as that of the aforementioned substituent (c) in the substituted lower alkyl),
substituted or unsubstituted aryl (the substituent in said substituted aryl has the same meaning as that of the after-mentioned substituent (xii) in the substituted aryl),
a substituted or unsubstituted heterocyclic group (the substituent in said substituted heterocyclic group has the same meaning as that of the
after-mentioned substituent (xiii) in the substituted heterocyclic group), substituted or unsubstituted lower alkoxy (the substituent in said substituted lower alkoxy has the same meaning as that of the aforementioned substituent (c) in the substituted lower alkyl), or
-NR⁵¹R⁵² (wherein R⁵¹ and R⁵² have the same meanings as those of the aforementioned R³⁰ and R³¹, respectively)],
-OSO₂R⁵³ (wherein R⁵³ has the same meaning as that of the aforementioned R⁵⁰) and the like.

Herein, the lower alkyl moiety in the lower alkyl, the lower alkoxy, the lower alkylthio, the lower alkylsulfonyl, the lower alkylamino, the di-(lower alkyl)amino and the lower alkanoyloxy, the lower alkenyl, the lower alkynyl, the cycloalkyl, the aryl moiety in the aryl and the aryloxy, the heterocyclic group, the heterocyclic group formed together with the adjacent nitrogen atom and the halogen have the same meanings as those of the aforementioned lower alkyl (i), lower alkenyl (ii), lower alkynyl (iii), cycloalkyl (iv), aryl (v), a heterocyclic group (vii), a heterocyclic group formed together with the adjacent nitrogen atom (viii) and halogen (x), respectively, and two of the lower alkyl moieties in the di-(lower alkyl)amino may be the same or different.
(xii) The substituent in the substituted aryl, the substituted aryloxy, the substituted arylamino and the substituted phenylene may be the same or different in number of 1 to 3 substituent(s), and includes halogen, hydroxy, carboxy, formyl, nitro, cyano, methylenedioxy, substituted or unsubstituted lower alkyl [the substituent (e) in said substituted lower alkyl may be the same or different in number of 1 to 3 substituent(s), and includes
   halogen, hydroxy, oxo, carboxy,
   substituted or unsubstituted lower alkoxy (the substituent (f) in said substituted lower alkoxy may be the same or different in number of 1 to 3 substituent(s), and includes
   halogen, hydroxy, oxo, carboxy, lower alkoxy, amino, lower alkylamino,
   di-(lower alkyl)amino, aryl, a heterocyclic group and the like), amino,
   substituted or unsubstituted lower alkylamino (the substituent in said substituted lower alkylamino has the same meaning as that of the aforementioned substituent (f) in the substituted lower alkoxy), di-(substituted or unsubstituted lower alkyl)amino (the substituent in said di-(substituted lower alkyl)amino has the same meaning as that of the aforementioned substituent (f) in the substituted lower alkoxy),
   aryl,
   a heterocyclic group, and the like],
   substituted or unsubstituted lower alkenyl (the substituent in said substituted lower alkenyl has the same meaning as that of the aforementioned substituent (e) in the substituted lower alkyl),
   substituted or unsubstituted lower alkynyl (the substituent in said substituted lower alkynyl has the same meaning as that of the aforementioned substituent (e) in the substituted lower alkyl),
   substituted or unsubstituted cycloalkyl (the substituent in said substituted cycloalkyl has the same meaning as that of the aforementioned substituent (e) in the substituted lower alkyl),
   substituted or unsubstituted lower alkoxy (the substituent in said substituted lower alkoxy has the same meaning as that of the aforementioned substituent (e) in the substituted lower alkyl),
   substituted or unsubstituted lower alkylthio (the substituent in said substituted lower alkylthio has the same meaning as that of the aforementioned substituent (e) in the substituted lower alkyl),
   amino,
   substituted or unsubstituted lower alkylamino (the substituent in said substituted lower alkylamino has the same meaning as that of the aforementioned substituent (e) in the substituted lower alkyl),
   di-(substituted or unsubstituted lower alkyl)amino (the substituent in said di-(substituted lower alkyl)amino has the same meaning as that of the aforementioned substituent (e) in the substituted lower alkyl),
   substituted or unsubstituted aryl [the substituent (g) in said substituted aryl may be the same or different in number of 1 to 3 substituent(s), and includes
   halogen, hydroxy, carboxy, cyano, nitro,
   substituted or unsubstituted lower alkyl (the substituent in said substituted lower alkyl has the same meaning as that of the aforementioned substituent (f) in the substituted lower alkoxy),
   substituted or unsubstituted lower alkoxy (the substituent in said substituted lower alkoxy has the same meaning as that of the aforementioned substituent (f) in the substituted lower alkoxy),
   amino,
   substituted or unsubstituted lower alkylamino (the substituent in said substituted lower alkylamino has the same meaning as that of the aforementioned substituent
   (f) in the substituted lower alkoxy), di-(substituted or unsubstituted lower alkyl)amino (the substituent in said di-(substituted lower alkyl)amino has the same meaning as that of the aforementioned substituent (f) in the substituted lower alkoxy) and the like],
   a substituted or unsubstituted heterocyclic group (the substituent in said substituted heterocyclic group has the same meaning as that of the aforementioned substituent (g) in the substituted aryl),
   substituted or unsubstituted aryloxy (the substituent in said substituted aryloxy has the same meaning as that of the aforementioned substituent (g) in the substituted aryl),
   substituted or unsubstituted arylamino (the substituent in said substituted arylamino has the same meaning as that of the aforementioned substituent (g) in the substituted aryl),
   substituted or unsubstituted arylthio (the substituent in said substituted arylthio has the same meaning as that of the aforementioned substituent (g) in the substituted aryl),
   substituted or unsubstituted arylsulfonyl (the substituent in said substituted arylsulfonyl has the same meaning as that of the aforementioned substituent (g) in the substituted aryl),
   substituted or unsubstituted heterocyclic oxy (the substituent in said substituted heterocyclic oxy has the same meaning as that of the aforementioned substituent (g) in the substituted aryl),
   substituted or unsubstituted heterocyclic amino (the substituent in said substituted heterocyclic amino has the same meaning as that of the aforementioned substituent (g) in the substituted aryl),
   substituted or unsubstituted heterocyclic thio (the substituent in said substituted heterocyclic thio has the same meaning as that of the aforementioned substituent (g) in the substituted aryl), and the like.

Herein, the lower alkyl moiety in the lower alkyl, the lower alkoxy, the lower alkylthio, the lower alkylamino and the di-(lower alkyl)amino has the same meaning as that of the aforementioned lower alkyl (i). The lower alkenyl, the lower alkynyl, the cycloalkyl and the halogen have the same meanings as those of the lower alkenyl (ii), the lower alkynyl (iii), the cycloalkyl (iv), and the halogen (x), respectively, and two of the lower alkyl moieties of the di-(lower alkyl)amino may be the same or different. Further, herein, the aryl moiety in the aryl, the aryloxy, the arylthio, the arylamino and the arylsulfonyl has the same meaning as that of the aforementioned aryl (v), and the heterocyclic group moiety of the heterocyclic group, the heterocyclic amino, the heterocyclic oxy and the heterocyclic thio has the same meaning as that of the aforementioned heterocyclic group (vii).
(xiii) The substituent in the substituted aromatic heterocyclic group, and the substituted aromatic heterocyclic group among the substituted heterocyclic group has the same meaning as that of the aforementioned substituent (xii) in the substituted aryl, and the substituent in the substituted aliphatic heterocyclic group among the substituted heterocyclic group, and the substituted heterocyclic group formed together with the adjacent nitrogen atom includes oxo and the like as well as the groups mentioned in the definition of the aforementioned substituent (xii) in the substituted aryl.

Example of the pharmacologically acceptable salt of Compound (I) include pharinacoiogically acceptable acid addition salts, metal salts, ammonium salts, organic amine addition salts, amino acid addition salts and the like. Examples of the pharmacologically acceptable acid addition salt of Compound (I) include an inorganic acid addition salt such as hydrochloride, sulfate and phosphate, an organic acid addition salt such as acetate, maleate, fumarate and citrate, and the like. Examples of the pharmacologically acceptable metal salt include an alkali metal salt such as a sodium salt and a potassium salt, an alkaline-earth metal salt such as a magnesium salt and a calcium salt, an aluminium salt, a zinc salt and the like. Examples of the pharmacologically acceptable ammonium salt include a salt of ammonium, tetramethylammonium or the like. Examples of the pharmacologically acceptable organic amine addition salt include an addition salt of morpholine, piperidine or the like. Examples of the pharmacologically acceptable amino acid addition salt include an addition salt of lysine, glycine, phenylalanine, aspartic acid, glutamic acid or the like.

Methods for preparing the Compound (I) will be described below.

In the preparing methods as shown below, when the defined group changes under the conditions of the method carried out, or is inappropriate for carrying out the methods, the desired compound can be obtained by using the protection and deprotection methods which are ordinarily used in the organic synthetic chemistry [e.g., Protective Groups in Organic Synthesis, T. W. Greene, John Wiley & Sons Inc. (1981)] and the like. In addition, the order of the steps for introducing a substituent and the like may be changed, if necessary.

Compound (I) can be prepared according to the following preparing methods. Preparing method 1

Among Compound (I), Compound (Ia) wherein Z is a sulfur atom, and R¹ is substituted or unsubstituted lower alkyl, substituted or unsubstituted lower alkenyl, substituted or unsubstituted lower alkynyl, substituted or unsubstituted aryl, or a substituted or unsubstituted aromatic heterocyclic group among the definitions of R¹ can also be prepared in accordance with the following steps from Compound (II) that can be obtained by the method described in WO03/051854, or the methods similar to the foregoing method. 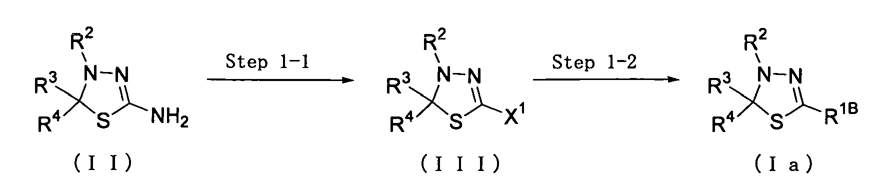 [wherein X¹ represents each atom of chlorine, bromine and iodine, R², R³ and R⁴ have the same meanings as those mentioned above, and R^{1B} represents substituted or unsubstituted lower alkyl, substituted or unsubstituted lower alkenyl, substituted or unsubstituted lower alkynyl, substituted or unsubstituted aryl, or a substituted or unsubstituted aromatic heterocyclic group among the definitions of R¹]

### Step 1-1

Compound (III) can be prepared by the methods described in J. Chem. Soc. Chem. Commun., Vol. 8, p. 873 (1998) and the like, or the methods similar to the foregoing methods.

Specifically, Compound (III) can be prepared by reacting Compound (II) with 1 to 30 equivalents of a nitrite compound such as sodium nitrite, and tert-butyl nitrite without solvent or in an appropriate solvent, if necessary, in the presence of 0.1 to 50 equivalents of an appropriate acid at a temperature between -50°C and 100°C for 5 minutes to 48 hours to prepare a corresponding diazonium salt, and then reacting the diazonium salt with 1 to 30 equivalents of, for example, copper halide, iodine or the like in an appropriate solvent, if necessary, with addition of 1 to 30 equivalents of potassium iodide at a temperature between -50°C and 200°C for 5 minutes to 48 hours.

Examples of the appropriate solvent used for each reaction include methanol, ethanol, dichloromethane, chloroform, acetonitrile, toluene, ethyl acetate, tetrahydrofuran (THF), 1,4-dioxane, N,N-dimethylformamide (DMF), N-methylpyrrolidone (NMP), water and the like, and they can be used alone or as a mixture thereof. Examples of the appropriate acid include hydrochloric acid, hydrobromic acid, sulfuric acid, acetic acid, trifluoroacetic acid and the like. Examples of the copper halide include, for example, copper chloride, copper bromide, copper iodide and the like. These copper halides can be prepared by, for example, adding sodium chloride, sodium bromide, or the like to an aqueous solution of copper sulfate and then reducing the resultant by sodium nitrite, and they can also be used for this step as the mixture per se without isolation.

Further, Compound (III) may also be prepared by reaction of the diazonium salt with copper halide in one pot without isolation of the diazonium salt. Specifically, Compound (III) can also be prepared by reacting a mixture of Compound (II), 1 to 30 equivalents of the nitrite compound exemplified above, and 1 to 30 equivalents of copper halide, iodine, potassium iodide, or the like exemplified above in an appropriate solvent exemplified above at a temperature between -50°C and 200°C for 5 minutes to 48 hours.

### Step 1-2

Compound (Ia) can be prepared by reacting Compound (III) obtained in Step 1-1 mentioned above with 1 to 30 equivalents of (R^{1B})ₚM_{q}(R^{A})ᵣ (wherein R^{1B} has the same meaning as that mentioned above, M represents each atom of tin, zinc, boron, silicon, aluminum, zirconium, copper, and mercury, R^{A} represents hydroxy, halogen having the same meaning as defined above, lower alkyl having the same meaning as defined above, lower alkoxy having the same meaning as defined above, aryl having the same meaning as defined above, or aryloxy having the same meaning as defined above, p and q are the same or different, and represent 1 or 2, and r represents an integer of 0 to 3) in an appropriate solvent in the presence of 0.001 to 1 equivalent of a transition metal catalyst at a temperature between -50°C and 200°C for 5 minutes to 80 hours. In this reaction, 0.01 to 30 equivalents of an appropriate additive may also be added to accelerate the reaction.

Examples of the appropriate solvent include methanol, ethanol, dichloromethane, chloroform, acetonitrile, toluene, ethyl acetate, THF, 1,4-dioxane, DMF, NMP, water and the like, and they can be used alone or as a mixture thereof. Examples of the transition metal catalyst include palladium catalysts such as palladium acetate, tetrakis(triphenylphosphine)palladium, palladium chloride, palladium bromide, bis(triphenylphosphine)palladium chloride, dichlorobis(acetonitrile)palladium and bis(dibenzylideneacetone)palladium, and nickel catalysts such as nickel chloride, nickel acetylacetonate, bis(1,5-cyclooctadiene)nickel, and nickel bromide. Examples of the appropriate additive include triphenylphosphine, tri(o-tolyl)phosphine, 1,1'-bis(diphenylphosphino)ferrocene, 1,2-bis(diphenylphosphino)propane, 2,2'-bis(diphenylphosphino)-1,1'-binaphthyl, 1,2-bis(diphenylphosphino)ethane, silver oxide, copper iodide, lithium chloride, cesium fluoride, triethylamine, diethylamine, sodium hydroxide, potassium hydroxide, sodium carbonate, tetrabutylammonium fluoride, and the like, and they can be used alone or as a mixture thereof.

### Preparing method 2

Among Compound (Ia), Compound (Ib) wherein R¹ is -C≡C-R^{1C} [wherein R^{1C} represents substituted or unsubstituted lower alkyl (said lower alkyl has the same meaning as that mentioned above, and the substituent in said substituted lower alkyl has the same meaning as that of the aforementioned substituent (xi)in the substituted lower alkyl)] can also be prepared in accordance with the following step. 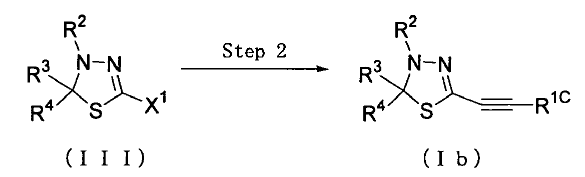 (wherein X¹, R^{1C}, R², R³ and R⁴ have the same meanings as those mentioned above, respectively)

### Step 2

Compound (Ib) can be prepared by reacting Compound (III) obtained in the preparing Step 1-1 of method 1 with 1 to 50 equivalents of HC≡C-R^{1C} (wherein R^{1C} has the same meanings as that mentioned above) in the presence of 0.01 to 1 equivalent of a palladium catalyst without solvent or in an appropriate solvent at a temperature between -50°C and 200°C for 5 minutes to 80 hours. In this reaction, 0.01 to 20 equivalents of an appropriate additive may also be added to accelerate the reaction.

Examples of the appropriate solvent include methanol, ethanol, dichloromethane, acetonitrile, toluene, ethyl acetate, THF, 1,4-dioxane, DMF, NMP, water and the like, and they can be used alone or as a mixture thereof. Examples of the palladium catalyst include palladium acetate, tetrakis(triphenylphosphine)palladium, palladium chloride, palladium bromide, bis(triphenylphosphine)palladium chloride, dichlorobis(acetonitrile)palladium, and the like. Examples of the appropriate additive include triphenylphosphine, tri(o-tolyl)phosphine, 1,1'-bis(diphenylphosphino)ferrocene, 2,2'-bis(diphenylphosphino)-1,1'-binaphthyl, 1,2-bis(diphenylphosphino)ethane, 1,2-bis(diphenylphosphino)propane, copper iodide, silver oxide, lithium chloride, cesium fluoride, triethylamine, diethylamine, sodium hydroxide, potassium hydroxide, sodium carbonate, and the like, and they can be used alone or as a mixture thereof. Preparing method 3

Among Compound (I), Compound (Ic) wherein Z is a sulfur atom, R² is a hydrogen atom, and R¹ is substituted or unsubstituted aryl, or a substituted or unsubstituted aromatic heterocyclic group among the definitions of R¹ can also be prepared by the methods described in Japanese Patent Unexamined Publication (KOKAI) No. 2000-159756, and the like, or the methods similar to the foregoing methods.

### Preparing method 4

Among Compound (I), Compound (Id) wherein Z is a sulfur atom, R² is substituted or unsubstituted lower alkyl, or -C(=O)R¹² (wherein R¹² has the same meanings as that defined above) among the definitions of R² can be prepared from Compound (Ie) wherein R² is a hydrogen atom among Compound (I), which is obtained in the preparing methods 1 to 3, or the after-mentioned preparing methods 5 to 14, in accordance with the following step. 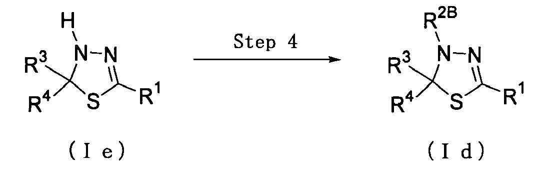 [wherein R¹, R³ and R⁴ have the same meanings as those mentioned above, R^{2B} is substituted or unsubstituted lower alkyl, or -C(=O)R¹² (wherein R¹² has the same meaning as those mentioned above) among the definitions of R²]

### Step 4

Compound (Id) can be prepared by reacting Compound (Ie) with 1 to 30 equivalents of R^{2B}X² (wherein R^{2B} has the same meaning as that mentioned above, and X² represents each atom of chlorine, bromine and iodine), or (R¹²CO)₂O (wherein R¹² has the same meaning as those mentioned above) without solvent or in an appropriate solvent in the presence or absence of 0.01 to 50 equivalents of an appropriate base at a temperature between -50°C and the boiling point of the solvent used for 5 minutes to 48 hours.

Examples of the appropriate solvent include methanol, ethanol, dichloromethane, acetonitrile, toluene, ethyl acetate, THF, 1,4-dioxane, DMF, NMP and the like, and they can be used alone or as a mixture thereof. Examples of the appropriate base include sodium hydride, lithium hydroxide, cesium fluoride, triethylamine, diethylamine, sodium hydroxide, potassium hydroxide, sodium carbonate, potassium carbonate, triethylamine, diisopropylamine, 1,8-diazah,'_cyclo [5.4.0]uncec-7-ene (DBU), 4-dimethylaminopyridine and the like, and they can be used alone or as a mixture thereof.

### Preparing method 5

Among Compound (I), Compounds (If) and (Ig) wherein Z is a sulfur atom, and R¹ is -C(=W)R⁵ (wherein W and R⁵ have the same meanings as those mentioned above, respectively) can be prepared in accordance with the following step from Compound (Ia-a) obtained in the preparing method 1. 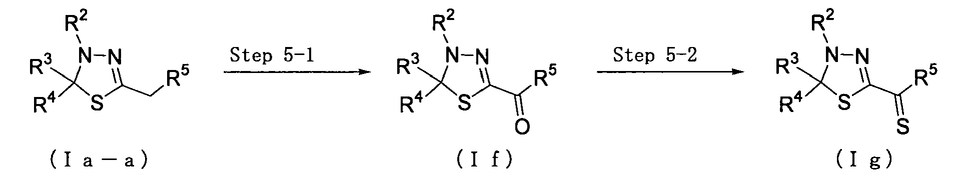 (wherein R², R³, R⁴ and R⁵ have the same meanings as those mentioned above, respectively)

### Step 5-1

Compound (If) can be prepared by treatment of Compound (Ia-a) with 1 to 100 equivalents of an appropriate oxidizing agent without solvent or in an appropriate solvent at a temperature between -30°C and 150°C for 5 minutes to 72 hours.

Examples of the appropriate solvent include methanol, dichloromethane, acetone, toluene, ethyl acetate, DMF, water and the like, and they can be used alone or as a mixture thereof. Examples of the appropriate oxidizing agent include chromium(IV) oxide, potassium permanganate, manganese dioxide, selenium dioxide, pyridinium chlorochromate (PCC), hydrogen peroxide, and the like.

### Step 5-2

Compound (Ig) can be prepared by treatment of Compound (If) obtained in Step 5-1 mentioned above with 1 to 100 equivalents of an appropriate sulfur compound in an appropriate solvent at a temperature between -30°C and the boiling point of the solvent used for 5 minutes to 72 hours.

Examples of the appropriate solvent include methanol, dichloromethane, toluene, xylene, ethyl acetate, THF, DMF, water and the like, and they can be used alone or as a mixture thereof. Examples of the appropriate sulfur compound include sodium sulfide, sodium hydrosulfide, 2,4-bis(4-methoxyphenyl)-1,3-dithia-2,4-diphosphetane 2,4-disulfide (Lawesson's reagent), sulfur, and the like.

Compound (Ig) can also be prepared from Compound (If) by the methods described in, for example, Shin-Jikken-Kagaku-Koza, Vol. 14, p. 1699 (1978), Maruzen, and WO03/051854, or the methods similar to the foregoing methods.

### Preparing method 6

Among Compound (I), Compound (Ih) wherein Z is a sulfur atom, R¹ is -C(OR^{c})=CR^{a}R^{b} [wherein R^{a} and R^{b} are the same or different, and represent a hydrogen atom, substituted or unsubstituted lower alkyl (said lower alkyl has the same meaning as that mentioned above, and the substituent in said substituted lower alkyl has the same meaning as that of the aforementioned substituent (xi) in the substituted lower alkyl), substituted or unsubstituted lower alkenyl (said lower alkenyl has the same meaning as that mentioned above, and the substituent in said substituted lower alkenyl has the same meaning as that of the aforementioned substituent (xi) in the substituted lower alkenyl), substituted or unsubstituted lower alkynyl (said lower alkynyl has the same meaning as that mentioned above, and the substituent in said substituted lower alkynyl has the same meaning as that of the aforementioned substituent (xi) in the substituted lower alkynyl), substituted or unsubstituted cycloalkyl (said cycloalkyl has the same meaning as that mentioned above, and the substituent in said substituted cycloalkyl has the same meaning as that of the aforementioned substituent (xi) in the aforementioned substituted cycloalkyl), substituted or unsubstituted aryl (said aryl has the same meaning as that mentioned above, and the substituent in said substituted aryl has the same meaning as that of the aforementioned substituent (xii) in the substituted aryl), or a substituted or unsubstituted aromatic heterocyclic group (said aromatic heterocyclic group has the same meaning as that mentioned above, and the substituent in said substituted aromatic heterocyclic group has the same meaning as that of the aforementioned substituent (xiii) in the substituted aromatic heterocyclic group), and R^{c} represents lower alkyl having the same meaning as that mentioned above], and Compound (Ii) wherein Z is a sulfur atom, R¹ is -COCHR^{a}R^{b} (wherein Rₐ and R^{b} have the same meanings as those mentioned above, respectively) can be prepared from Compound (III) obtained in Step 1-1 of the preparing method 1 in accordance with the following step. 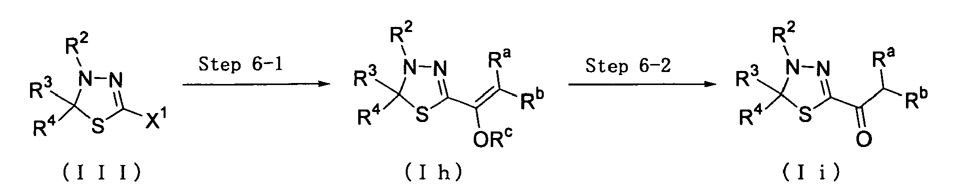 (wherein X¹, R², R³, R⁴, R^{a}, R^{b} and R^{c} have the same meanings as those mentioned above, respectively)

### Step 6-1

Compound (Ih) can be prepared from Compound (III) in the same manner as that of Step 1-2 of the preparing method 1.

### Step 6-2

Compound (Ii) can be prepared by treatment of Compound (Ih) obtained in Step 6-1 mentioned above with 0.1 to 500 equivalents of an acid without solvent or in an appropriate solvent at a temperature between -30°C and 150°C for 5 minutes to 72 hours.

Examples of the appropriate solvent include methanol, ethanol, dichloromethane, chloroform, acetonitrile, toluene, ethyl acetate, THF, 1,4-dioxane, DMF, NMP, dimethyl sulfoxide (DMSO), water and the like, and they can be used alone or as a mixture thereof. Examples of the acid include hydrochloric acid, sulfuric acid, acetic acid, trifluoroacetic acid, p-toluenesulfonic acid, and the like.

### Preparing method 7

Among Compound (I), Compound (Ik) wherein Z is a sulfur atom, R¹ is -CR^{e}HCR^{f}R^{g}NR³⁴R³⁵ [wherein R^{e}, R^{f} and R^{g} are the same or different, and represent a hydrogen atom, or substituted or unsubstituted lower alkyl (said lower alkyl has the same meaning as that mentioned above, and the substituent in said substituted lower alkyl has the same meaning as that of the aforementioned substituent (xi) in the substituted lower alkyl), and R³⁴ and R³⁵ have the same meanings as those mentioned above, respectively] can be prepared in accordance with the following step. 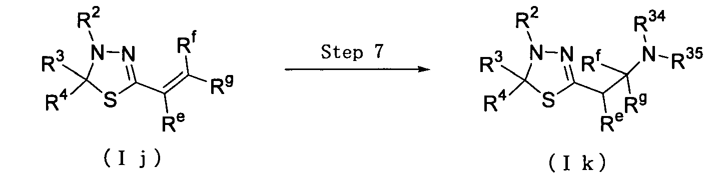 (wherein R², R³, R⁴, R^{e}, R^{f}, R^{g}, R³⁴ and R³⁵ have the same meanings as those mentioned above, respectively)

### Step 7

Compound (Ik) can be prepared by reacting Compound (Ij) obtained in the preparing method 1 with 1 to 300 equivalents of HNR³⁴R³⁵ (wherein R³⁴ and R³⁵ have the same meanings as those mentioned above, respectively) without solvent or in an appropriate solvent, if necessary, in the presence of 0.1 to 100 equivalents of an appropriate base at a temperature between -30°C and 200°C for 5 minutes to 100 hours.

Examples of the appropriate solvent include methanol, ethanol, dichloromethane, chloroform, acetonitrile, toluene, xylene, ethyl acetate, THF, 1,4-dioxane, DMF, NMP, DMSO, water and the like, and they can be used alone or as a mixture thereof. Examples of the appropriate base include sodium hydride, sodium hydroxide, potassium hydroxide, potassium carbonate, sodium hydrogencarbonate, sodium methoxide, triethylamine, diisopropylethylamine, DBU, 4-dimethylaminopyridine and the like, and they can be used alone or as a mixture thereof.

### Preparing method 8

Among Compound (I), Compound (Im) wherein Z is a sulfur atom, and R¹ is -CR^{e}HCR^{f}R^{g}SR⁴⁹ (wherein R^{e}, R^{f}, R^{g} and R⁴⁹ have the same meanings as those mentioned above, respectively) can also be prepared in accordance with the following step. 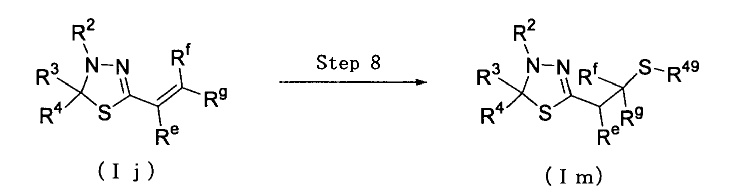 (wherein R², R³, R⁴, R^{e}, R^{f}, R^{g} and R⁴⁹ have the same meanings as those mentioned above, respectively)

### Step 8

Compound (Im) can be prepared by reacting Compound (Ij) obtained in the preparing method 1 with 1 to 200 equivalents of HSR⁴⁹ (wherein R⁴⁹ has the same meaning as that mentioned above) without solvent or in an appropriate solvent, if necessary, in the presence of 0.1 to 100 equivalents of an appropriate base at a temperature between -30°C and 200°C for 5 minutes to 100 hours.

Examples of the appropriate solvent include methanol, ethanol, dichloromethane, chloroform, acetonitrile, toluene, xylene, ethyl acetate, THF, 1,4-dioxane, DMF, NMP, DMSO, water and the like, and they can be used alone or as a mixture thereof. Examples of the appropriate base include sodium hydride, sodium hydroxide, potassium hydroxide, potassium carbonate, sodium methoxide, triethylamine, diisopropylethylamine, DBU, 4-dimethylaminopyridine and the like, and they can be used alone or as a mixture thereof.

### Preparing method 9

Among Compound (I), Compound (In) wherein R¹ is carboxyl group can be prepared in accordance with the following step. 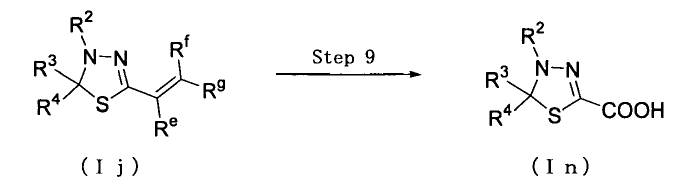 (wherein R², R³, R⁴, Re, R^{f} and R^{g} have the same meanings as those mentioned above, respectively)

### Step 9

Compound (In) can be prepared by treatment of Compound (Ij) obtained in the preparing method 1 with 0.1 to 50 equivalents of potassium permanganate, or the like in an appropriate solvent, if necessary, in the presence of 0.1 to 10 equivalents of a phase transfer catalyst and/or 0.1 to 50 equivalents of base at a temperature between -30°C and 150°C for 5 minutes to 72 hours.

Examples of the appropriate solvent include dichloromethane, ethyl acetate, toluene, DMF, acetone, methyl ethyl ketone (MEK), pyridine, acetic acid, water and the like, and they can be used alone or as a mixture thereof. Examples of the phase transfer catalyst include crown ethers such as 18-crown-6 and 16-crown-5, ammonium salts such as tetrabutylammonium chloride, and tetrabutylammonium bromide, and the like. Examples of the base include sodium hydroxide, potassium hydroxide and the like.

### Preparing method 10

Among Compound (I), Compound (Io) wherein R¹ is -COYR^{6a} (wherein Y has the same meaning as that mentioned above, and R^{6a} represents any one of the groups among the definitions of R⁶ except for a hydrogen atom) can be prepared in accordance with the following step. 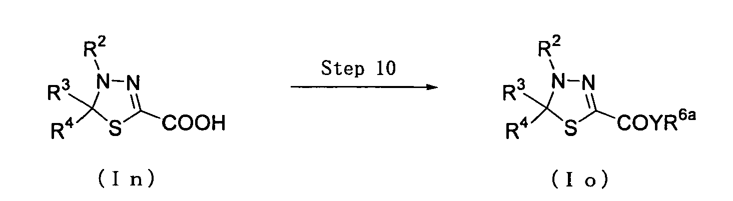 (wherein R², R³, R⁴, R^{6a} and Y have the same meanings as those mentioned above, respectively)

### Step 10

Compound (Io) can be prepared by reacting Compound (In) obtained in the preparing method 9, the after-mentioned preparing method 13, or the like with 1 to 200 equivalents of R^{6a}YH (wherein R^{6a} and Y have the same meanings as those mentioned above, respectively) without solvent or in an appropriate solvent in the presence of 1 to 50 equivalents of an appropriate chlorinating agent at a temperature between -30°C and 150°C for 5 minutes to 72 hours.

Examples of the appropriate solvent include dichloromethane, chloroform, acetonitrile, toluene, xylene, ethyl acetate, THF, 1,4-dioxane, DMF, NMP and the like, and they can be used alone or as a mixture thereof. Examples of the appropriate chlorinating agent include thionyl chloride, phosphorous oxychloride, cyanuric chloride and the like.

As an alternative method, Compound (Io) can also be prepared by treatment of Compound (In) with 1 to 200 equivalents of an appropriate chlorinating agent without solvent or in an appropriate solvent at a temperature between -30°C and 150°C for 5 minutes to 72 hours, and then reacting the resultant with 1 to 300 equivalents of R^{6a}YH (wherein R^{6a} and Y have the same meanings as those mentioned above, respectively) without solvent or in an appropriate solvent, if necessary, in the presence of an appropriate base at a temperature between -30°C and 150°C for 5 minutes to 72 hours.

Examples of the appropriate solvent used for each reaction include dichloromethane, chloroform, acetonitrile, toluene, xylene, ethyl acetate, THF, 1,4-dioxane, DMF, NMP and the like, and they can be used alone or as a mixture thereof. Examples of the appropriate chlorinating agent include thionyl chloride, phosphorous oxychloride, cyanuric chloride and the like. Examples of the appropriate base include pyridine, triethylamine, diisopropylethylamine, DBU, 4-dimethylaminopyridine and the like.

Further, as an alternative method, Compound (Io) can also be prepared by reacting Compound (In) with 1 to 300 equivalents of R^{6a}YH (wherein R^{6a} and Y have the same meanings as those mentioned above, respectively) without solvent or in an appropriate solvent in the presence of 1 to 30 equivalents of an appropriate condensing agent and, if necessary, in the presence of 0.1 to 30 equivalents of an additive, at a temperature between -30°C and 150°C for 5 minutes to 72 hours.

Examples of the appropriate solvent include dichloromethane, chloroform, acetonitrile, toluene, xylene, ethyl acetate, THF, 1,4-dioxane, DMF, NMP and the like, and they can be used alone or as a mixture thereof. Examples of the appropriate condensing agent include 1-(3-dimethylaminopropyl)-3-ethylcarbodiimide (EDC), 1-(3-dimethylaminopropyl)-3-ethylcarbodiimide hydrochloride (EDC • HCl), N,N'-dicyclohexylcarbodiimide (DCC), 1,1'-carboxyldiimidazole (CDI), and the like. Examples of the additive include N-hydroxysuccinimide, 4-dimethylaminopyridine, 1-hydroxybenzotriazole (HOBt), 1-hydroxybenzotriazole monohydrate (HOBt • H₂O) and the like.

### Preparing method 11

Among Compound (I), Compound (Ip) wherein R¹ is -CONR⁷R⁸ (wherein R⁷ and

R⁸ have the same meanings as those mentioned above, respectively) can be prepared in accordance with the following step. 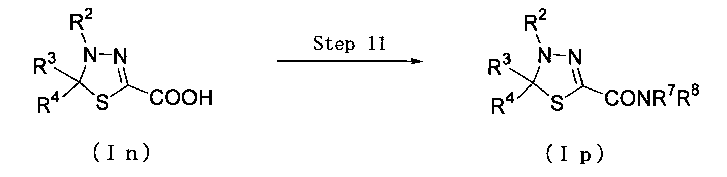 (wherein R², R³, R⁴, R⁷ and R⁸ have the same meanings as those mentioned above, respectively)

### Step 11

Compound (Ip) can be prepared by treating Compound (In) obtained in the preparing method 9, the after-mentioned preparing method 13, or the like with 1 to 200 equivalents of an appropriate chlorinating agent without solvent or in an appropriate solvent at a temperature between -30°C and 150°C for 5 minutes to 72 hours, and then reacting the resultant with 1 to 300 equivalents of HNR⁷R⁸ (wherein R⁷ and R⁸ have the same meanings as those mentioned above, respectively) without solvent or in an appropriate solvent, if necessary, in the presence of an appropriate base, at a temperature between -30°C and 150°C for 5 minutes to 72 hours.

Examples of the appropriate solvent used for each reaction include dichloromethane, chloroform, acetonitrile, toluene, xylene, ethyl acetate, THF, 1,4-dioxane, DMF, NMP, pyridine and the like, and they can be used alone or as a mixture thereof. Examples of the appropriate chlorinating agent include thionyl chloride, phosphorous oxychloride, cyanuric chloride and the like. Examples of the appropriate base include, for example, pyridine, triethylamine, diisopropylethylamine, DBU, 4-dimethylaminopyridine and the like.

As an alternative method, Compound (Ip) can also be prepared by reacting Compound (In) with 1 to 200 equivalents of HNR⁷R⁸ (wherein R⁷ and R⁸ have the same meanings as those mentioned above, respectively) without solvent or in an appropriate solvent in the presence of 1 to 20 equivalents of an appropriate condensing agent and, if necessary, in the presence of 0.1 to 30 equivalents of an additive, at a temperature between -30°C and 150°C for 5 minutes to 72 hours.

Examples of the appropriate solvent include dichloromethane, chloroform, acetonitrile, toluene, xylene, ethyl acetate, THF, 1,4-dioxane, DMF, NMP, water and the like, and they can be used alone or as a mixture thereof. Examples of the appropriate condensing agent include EDC, EDC • HCl, DCC, CDI and the like. Examples of the additive include N-hydroxysuccinimide, 4-dimethylaminopyridine, HOBt, HOBt • H₂O and the like.

### Preparing method 12

Among Compound (I), Compound (Iq) wherein R¹ is COOCH₃ can also be prepared in accordance with the following step. 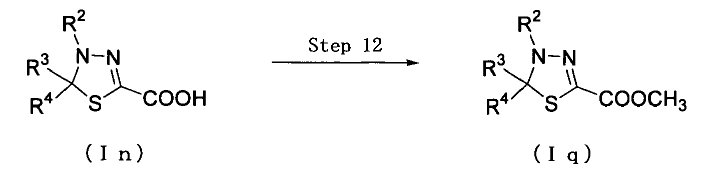 (wherein R², R³ and R⁴ have the same meaning as those mentioned above, respectively)

### Step 12

Compound (Iq) can also be prepared by reacting Compound (In) obtained in the preparing method 9, the after-mentioned preparing method 13, or the like with 1 to 100 equivalents of diazomethane, (trimethylsilyl)diazomethane, or the like in an appropriate solvent at a temperature between -30°C and 100°C for 5 minutes to 72 hours.

Examples of the appropriate solvent include methanol, ethanol, dichloromethane, chloroform, diethyl ether, THF, 1,4-dioxane, toluene, ethyl acetate, hexane, DMF and the like, and they can be used alone or as a mixture thereof.

As an alternative method, Compound (Iq) can also be prepared by reacting Compound (In) with 1 to 30 equivalents of methyl iodide in a solvent such as DMF, THF, and dichloromethane in the presence of 1 to 30 equivalents of potassium carbonate, or sodium hydride at a temperature between -30°C and 100°C for 5 minutes to 72 hours. Preparing method 13

Among Compound (I), Compound (In) wherein R¹ is -COOH can also be prepared in accordance with the following step. 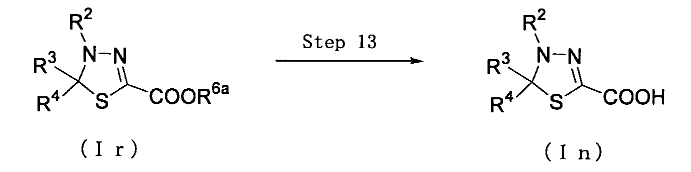 (wherein R², R³, R⁴ and R^{6a} have the same meanings as those mentioned above, respectively)

Compound (In) can be prepared by treatment of Compound (Ir) obtained in the preparing method 5, 10, or 12 with 1 to 100 equivalents of an appropriate base in water or in an appropriate solvent containing water at a temperature between -30°C and 150°C for 5 minutes to 72 hours.

Examples of the appropriate solvent containing water include a mixed solvent of at least one of methanol, ethanol, dichloromethane, acetonitrile, toluene, THF, 1,4-dioxane, DMF, NMP, or the like with water, and they can be used alone or as a mixture. Examples of the appropriate base include sodium hydroxide, potassium hydroxide, lithium hydroxide, sodium carbonate, potassium carbonate, aqueous ammonia, DBU and the like.

### Preparing method 14

Among Compound (I), Compound (Is) wherein Z is a sulfur atom can be prepared by the methods described in Chemistry of Heterocyclic Compounds, Vol. 35, p. 87 (1999), and the like, or the methods similar to those methods.

### Preparing method 15

Among Compound (I), Compound (It) wherein Z is -S (=O)- can be prepared from Compounds (Ia) - (Is) obtained in the preparing methods 1 to 14 in accordance with the following step. 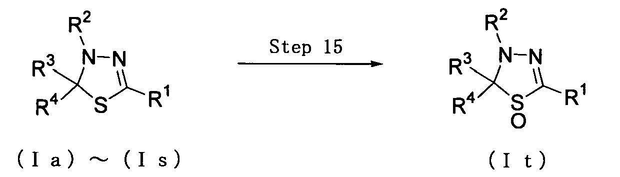 (wherein R¹, R², R³ and R⁴ have the same meanings as those mentioned above, respectively)

### Step 15

Compound (It) can be prepared from Compounds (Ia) - (Is) by the methods described in J. Chem. Soc. ,Chem. Commun., Vol. 16, p. 901 (1982) and the like, or the methods similar to those methods.

Specifically, Compound (It) can be prepared by treatment of Compounds (Ia) · (Is) obtained in the preparing methods 1 to 14 with 1 to 100 equivalents of an appropriate oxidizing agent in an appropriate solvent at a temperature between -30°C and 150°C for 5 minutes to 100 hours.

Examples of the appropriate solvent include methanol, ethanol, dichloromethane, chloroform, acetone, pyridine, acetic acid, water and the like, and they can be used alone or as a mixture thereof. Examples of the appropriate oxidizing agent include m-chloroperbenzoic acid, hydrogen peroxide, potassium permanganate, and the like.

In Compound (I), conversion of the functional groups contained in R¹, R², R³, or R⁴ can be carried out by the other known methods [e.g., Comprehensive Organic Transformations, R. C. Larock (1989) and the like], or the methods similar to the known methods, as well as by the aforementioned steps.

Compound (I) having the desired functional group at the desired position can be prepared by carrying out the aforementioned methods in appropriate combination.

The intermediates and the desired compounds in the aforementioned preparation methods can be isolated and purified by conducting separation and purification methods ordinarily used in the organic synthetic chemistry such as filtration, extraction, washing, drying, concentration, recrystallization, various chromatography and the like. The intermediates can also be subjected to the next reaction without particular purification.

Among Compounds (I), stereoisomers such as regioisomers, geometrical isomers, optical isomers, tautomers and the like may be existed, and including these isomers, all possible isomers and the mixtures thereof can be used for the antitumor agent, and the like of the present invention.

To obtain a salt of Compound (I), when Compound (I) is obtained as a salt form, it may be purified as it is. When Compound (I) is obtained as a free form, it may be dissolved or suspended in an appropriate solvent, and added an appropriate acid or base to form a salt and then be isolated and purified.

In addition, Compound (I) or a pharmacologically acceptable salt thereof may exist in the form of adducts with water or various solvents, which can also be used for the antitumor agent, and the like of the present invention.

Specific examples of Compounds (I) and (IA) used for the present invention, or obtained by the present invention are shown in Tables 1 and 2. However, the compounds used for the present invention, or the compounds of the present invention are not limited to these examples.

**Table 1**

| | | | | |
|---|---|---|---|---|
| 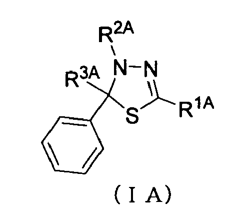 | | | | |

| Example No. | Compound No. | R^{1A} | R^{2A} | R^{3A} |
|---|---|---|---|---|
| 1 | 1 | 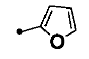 | -COCH₃ | -CH₃ |
| 2 | 2 | 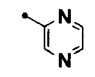 | -COCH₃ | -CH₃ |
| 3 | 3 | 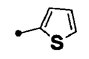 | -COCH₃ | -CH₃ |
| 4 | 4 | 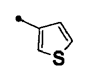 | -COCH₃ | -CH₃ |
| 5 | 5 | 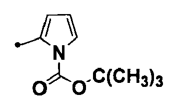 | -COCH₃ | -CH₃ |
| 6 | 6 | 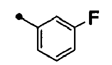 | -COCH₃ | -CH₂NHSO₂CH₃ |
| 7 | 7 | 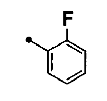 | -COC(CH₃)₃ | -CH₂NHSO₂CH₃ |
| 8 | 8 | 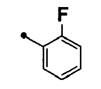 | -COCH₃ | -CH₂NHSO₂CH₃ |
| 9 | 9 | 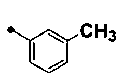 | -COCH₃ | -CH₂NHSO₂CH₃ |
| 10 | 10 | 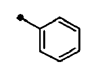 | -COCH₃ | -CH₂NHSO₂CH₃ |
| 11 | 11 | 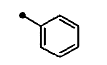 | -COC(CH₃)₃ | -CH₂NHSO₂CH₃ |
| 12 | 12 | 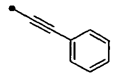 | -COCH₃ | -CH₃ |
| 13 | 13 | 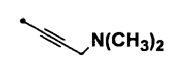 | -COCH₃ | -CH₃ |
| 14 | 14 | 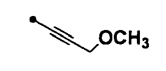 | -COCH₃ | -CH₃ |
| 15 | 15 | 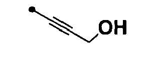 | -COCH₃ | -CH₃ |
| 16 | 16 | 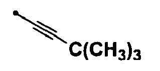 | -COCH₃ | -CH₃ |
| 17 | 17 | 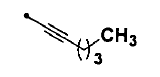 | -COCH₃ | -CH₃ |
| 18 | 18 | 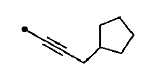 | -COCH₃ | -CH₃ |
| 19 | 19 | 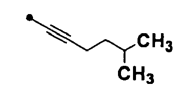 | -COCH₃ | -CH₃ |
| 20 | 20 | 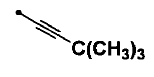 | -COCH₃ | -CH₂NHSO₂CH₃ |
| 21 | 21 | 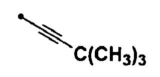 | -COC(CH₃)₃ | -CH₂NHSO₂CH₃ |
| 22 | 35 | -CH=CH₂ | -COCH₃ | -CH₃ |
| 23 | 36 | -COCH₃ | -COCH₃ | -CH₃ |
| 24 | 37 | 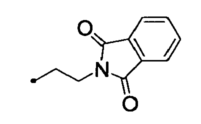 | -COCH₃ | -CH₃ |
| 25 | 38 | -(CH₂)₂NH₂ | -COCH₃ | -CH₃ |
| 26 | 39 | -(CH₂)₂NHCH₂CH₃ | -COCH₃ | -CH₃ |
| 27 | 40 | -(CH₂)₂NHSO₂CH₃ | -COCH₃ | -CH₃ |
| 28 | 41 | -CO₂CH₃ | -COCH₃ | -CH₃ |
| 29 | 42 | -CO₂H | -COCH₃ | -CH₃ |
| 30 | 43 | -CON(CH₃)₂ | -COCH₃ | -CH₃ |
| 31 | 44 | -CONHC(CH₃)₃ | -COCH₃ | -CH₃ |
| 32 | 45 | 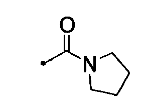 | -COCH₃ | -CH₃ |
| 33 | 46 | 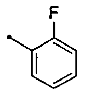 | -COCH₃ | 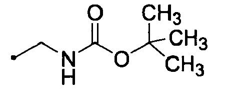 |
| 34 | 47 | 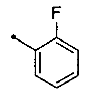 | -COCH₃ | 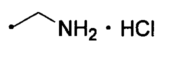 |
| 35 | 48 | 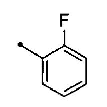 | -COCH₃ | 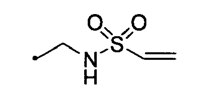 |
| 36 | 49 | 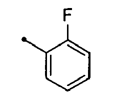 | -COCH₃ | 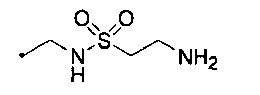 |
| 37 | 50 | 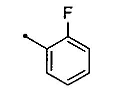 | -COCH₃ | 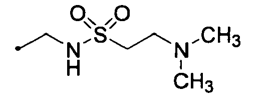 |
| 38 | 51 | 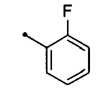 | -COCH₃ | 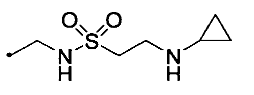 |
| 39 | 52 | 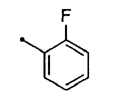 | -COCH₃ | 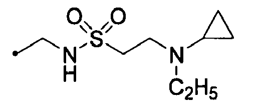 |
| 40 | 53 | 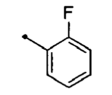 | -COCH₃ | 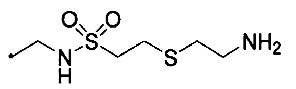 |
| 41 | 54 | 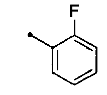 | -COCH₃ | 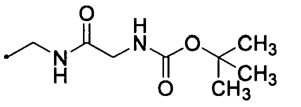 |
| 42 | 55 | 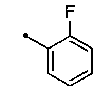 | -COCH₃ | 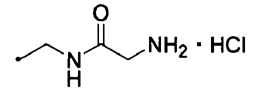 |
| 43 | 56 | 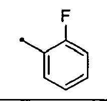 | -COCH₃ | 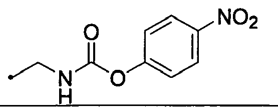 |
| 44 | 57 | 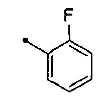 | -COCH₃ | 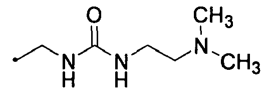 |
| 45 | 58 | 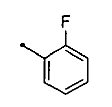 | -COCH₃ | 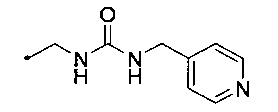 |
| 46 | 59 | 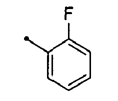 | -COCH₃ | 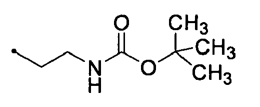 |
| 47 | 60 | 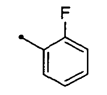 | -COCH₃ | 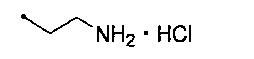 |
| 48 | 61 | 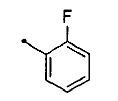 | -COCH₃ | 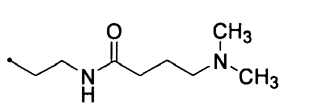 |
| 49 | 62 | 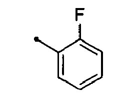 | -COCH₃ | 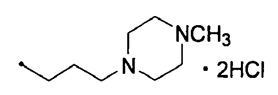 |
| 50 | 63 | -CH=CH₂ | -COCH₃ | -CH₂NHCO₂C(CH₃)₃ |
| 51 | 64 | -CO₂H | -COCH₃ | -CH₂NHCO₂C(CH₃)₃ |
| 52 | 65 | -CONHC(CH₃)₃ | -COCH₃ | -CH₂NHCO₂C(CH₃)₃ |
| 53 | 66 | -CONHC(CH₃)₃ | -COCH₃ | -CH₂NH₂ HCl |
| 54 | 67 | -CONHC(CH₃)₃ | -COCH₃ | -CH₂NHSO₂CH=CH₂ |
| 55 | 68 | -CONHC(CH₃)₃ | -COCH₃ | -CH₂NHSO₂(CH₂)₂NMe₂ |
| 56 | 69 | -CON[CH(CH₃)₃]₂ | -COCH₃ | -CH₂NHCO₂C(CH₃)₃ |
| 57 | 70 | -CON[CH(CH₃)₃]₂ | -COCH₃ | -CH₂NHSO₂CH=CH₂ |
| 58 | 71 | -CON[CH(CH₃)₃]₂ | -COCH₃ | -CH₂NHSO₂(CH₂)₂N·HCl |
| 59 | 72 | -CO₂CH₃ | -COCH₃ | -CH₂NHSO₂CH=CH₂ |
| 60 | 73 | -CON(CH₃)₂ | -COCH₃ | -CH₂NHSO₂(CH₂)₂NMe₂ |
| 61 | 74 | -CH=CH₂ | -COC(CH₃)₃ | -CH₂NHCO₂C(CH₃)₃ |
| 62 | 75 | -CONHC(CH₃)₃ | -COC(CH₃)₃ | -CH₂NHSO₂(CH₂)₂NMe₂·HCl |

**Table 2**

| | | | | |
|---|---|---|---|---|
| 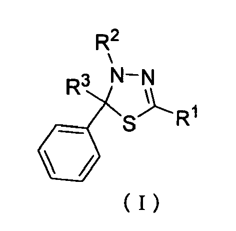 | | | | |

| Ref. Ex. No. | Compound No. | R¹ | R² | R³ |
|---|---|---|---|---|
| 6 | 22 | 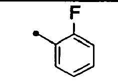 | -COCH₃ | -CH₃ |
| 7 | 23 | 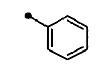 | -COCH₃ | -CH₃ |
| 8 | 24 | 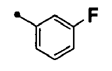 | -COCH₃ | -CH₃ |
| 9 | 25 | 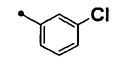 | -COCH₃ | -CH₃ |
| 10 | 26 | 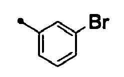 | -COCH₃ | -CH₃ |
| 11 | 27 | 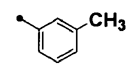 | -COCH₃ | -CH₃ |
| 12 | 28 | 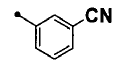 | -COCH₃ | -CH₃ |
| 13 | 29 | 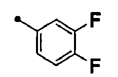 | -COCH₃ | -CH₃ |
| 14 | 30 | 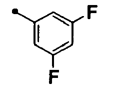 | -COCH₃ | -CH₃ |
| 15 | 31 | 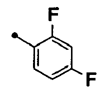 | -COCH₃ | -CH₃ |
| 16 | 32 | 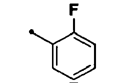 | -COCH₃ | 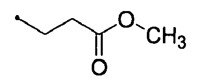 |
| 17 | 33 | 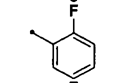 | -COCH₃ | 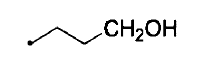 |
| 18 | 34 | 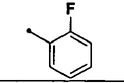 | -COCH₃ | 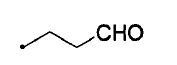 |

Pharmacological activities of typical Compound (I) will be specifically explained by the following test examples. Test example 1: Antiproliferative activity against HCT 116 human colon carcinoma cells

HCT 116 cells (ATCC No.: CCL-247) were placed on a 96-well microtiter plate (Nunc, 167008) at a density of 1x10³ cells/well. The plate was incubated in a 5% CO₂ incubator at 37°C for 24 hours, and then to the plate was added test compounds diluted stepwise to 100 µL/well in total, and the plate was further incubated in a 5% CO₂ incubator at 37°C for 72 hours. To the culture medium, the XTT (sodium 3'-[1-(phenylaminocarbonyl)-3,4-tetrazolium]-bis(4-methoxy-6-nitro)benzenesulfonic acid hydrate) labeling mixture (Roche Diagnostics, 1465015) was dispensed in 50 µL/well portions, then the plate was incubated in a 5% CO₂ incubator at 37°C for 1 hour, and the absorbance was measured at 490 nm and 655 nm with a microplate spectrophotometer (Bio-Rad, Model 550). The inhibitory activity against cell proliferation was shown as GI₅₀, a concentration of compound at which induces 50% inhibition of cell proliferation.

GI₅₀ calculation method: The value (difference in absorbance) was calculated by subtracting the absorbance at 655 nm from the absorbance at 490 nm of each well. The difference in absorbance obtained from the cells untreated with a test compound was defined as 100%, and compared with the difference in absorbance obtained from the cells treated with the solution of the compound in the known concentration, and thereby the concentration of the compound of 50% inhibition against cell proliferation was calculated to obtain GI₅₀.

The results were shown in Table 3.

**Table 3**

| Compound No. | GI₅₀ (µ mol/L) |
|---|---|
| 1 | 0.53 |
| 5 | 0.17 |
| 10 | 0.18 |
| 20 | 0.20 |
| 22 | 0.083 |
| 24 | 0.22 |
| 44 | 0.41 |
| 45 | 0.47 |
| 49 | < 0.1 |
| 55 | < 0.1 |
| 61 | < 0.1 |
| 62 | 0.16 |
| 68 | 0.19 |

### Test Example 2: Eg5 enzyme inhibition test (1)

A full length recombinant human Eg5 protein is prepared by referring to the literature [Cell, Vol. 83, p.1159 (1995)]. The *Spodoptera frugiperda* (Sf) 9 insect cells are infected with a baculovirus expressing a full length human Eg5 protein fused with a His tag at the N-terminus, and cultured. Then the culture medium is centrifuged to collect cell pellets. The cell pellets are suspended in a buffer, and the suspension is centrifuged to recover the supernatant. The supernatant is passed through a nickel agarose column to obtain the Eg5 protein fused with a His tag at the N-terminus as a partially purified sample.

Measurement of the ATPase activity of Eg5 is carried out by referring to the literature [EMBO Journal, Vol. 13, p.751 (1994); Proc. Natl. Acad. Sci. USA, Vol. 89, p.4884 (1992)]. A reaction solution is prepared which consisted of 25 mmol/L piperazine N,N'-bis(ethanesulfonate) (PIPES)/KOH (pH 6.8), 1 mmol/L ethylene glycol-bis(2-aminoethyl ether)tetraacetic acid (EGTA), 2 mmol/L MgCl₂, 1 mmol/L dithiothreitol (DTT), 100 µg/mL bovine serum albumin (BSA), 5 µmol/L paclitaxel, 25 µg/mL tubulin (Cytoskeleton, Catalog No. TL238), 200 µmol/L MESG substrate (2-amino-6-mercapto-7-methylpurine riboside) (Molecular Probes, Catalog Number E-6646), 1 U/mL purine nucleoside phosphorylase (Molecular Probe, Catalog No. E-6646) and the partially purified sample of full length human Eg5. The reaction solution containing serially diluted test compound is added to each well of a 96-well plate. The enzymatic reaction is performed at 30°C for 30 minutes. Absorbance at 360 nm is measured using a plate reader (Molecular Device, SpectraMax 340PC³⁸⁴) as an index of the ATPase activity. The absorbance observed in the presence of Eg5 and absence of the test compound is defined 100%, and the absorbance observed in the absence of both Eg5 and the test compound is defined 0%. The relative activity is calculated to determine the IC₅₀ value.

The inhibitory activity against Eg5 enzyme of Compound (I) can be confirmed by the test mentioned above.

### Test Example 3: Eg5 enzyme inhibition test (2)

A recombinant human Eg5 motor domain protein was prepared by referring to the literature [Biochemistry, Vol. 35, p.2365 (1996)]. A plasmid expressing the motor domain of human Eg5 was constructed, and transformed into *Escherichia coli* BL21 (DE3). The transformant was cultured at 25°C, and when the OD₆₀₀ reached 0.74, isopropyl-B-D-thiogalactoside was added at a final concentration of 0.5 mmol/L. The transformant was further cultured for 4 hours, and then the culture medium was centrifuged to collect the cells. The cells were suspended in a buffer and ultrasonicated, and then the sonicated solution was centrifuged to recover the supernatant. The supernatant was purified by cation exchange column chromatography to obtain a partially purified sample. Furthermore, the partially purified sample was purified by gel filtration column chromatography to obtain a finally purified sample.

Measurement of the ATPase activity of Eg5 was carried out by referring to the literatures [EMBO Journal, Vol. 13, p.751 (1994); Proc. Natl. Acad. Sci. USA, Vol. 89, p.4884 (1992)]. The following two kinds of solutions were prepared: Solution A consisting of 25 mmol/L piperazine N,N'-bis(ethanesulfonate) (PIPES)/KOH (pH 6.8), 1 mmol/L ethylene glycol-bis(2-aminoethyl ether)tetraacetic acid (EGTA), 2 mmol/L MgCl₂, 1 mmol/L dithiothreitol (DTT), 5 µmol/L paclitaxel, 167 µg/mL bovine serum albumin (BSA), 41.7 µg/mL tubulin (Cytoskeleton, Catalog No. TL238), 333 µmol/L MESG substrate (2-amino-6-mercapto-7-methylpurine riboside) (Molecular Probes, Catalog Number E-6646), 1.67 U/mL purine nucleoside phosphorylase (Molecular Probe, Catalog No. E-6646) and 1.33 µg/mL of the human Eg5 motor domain purified sample, and Solution B consisting of 25 mmol/L piperazine N,N'-bis(ethanesulfonate) (PIPES)/KOH (pH 6.8), 1 mmol/L ethylene glycol-bis(2-aminoethyl ether)tetraacetic acid (EGTA), 2 mmol/L MgCl₂, 1 mmol/L dithiothreitol (DTT), 5 µmol/L paclitaxel and 2.5 mmol/L ATP. Solution A was dispensed into each well of a 96-well plate as 45 µL portions. Solution B was used to serially dilute a test compound. The diluted test compound solutions in a volume of 30 µL were mixed with Solution A added beforehand in each well of the 96-well plate to start the enzymatic reaction. The enzymatic reaction was performed at 30°C for 30 minutes. Absorbance at 360 nm, which serves as an index of the ATPase activity, was measured using a plate reader (Molecular Device, SpectraMax 340PC³⁸⁴). The absorbance observed in the presence of Eg5 and absence of the test compound was defined 100%, and the absorbance observed in the absence of both Eg5 and the test compound was defined 0%. The relative activity was calculated to calculate IC₅₀ value.

Compounds 1, 5, 10, 20, 22, 24, 44, 49, 55, 61, 62, and 68 inhibited the ATPase activity of Eg5 in a concentration-dependent manner, and IC₅₀ values of the compounds were found to be 10 µmol/L or lower.

Compound (I) or a pharmaceutically acceptable salt thereof can be administered alone. However, usually, Compound (I) or a pharmaceutically acceptable salt thereof is preferably provided in various pharmaceutical preparations. Furthermore, these pharmaceutical preparations are used for animals and humans.

The pharmaceutical preparations according to the present invention may comprise Compound (I) or a pharmaceutically acceptable salt thereof alone as an active ingredient. Alternatively, the pharmaceutical preparations may comprise a mixture of Compound (I) or a pharmaceutically acceptable salt thereof with any effective ingredient used for another treatment. Furthermore, these pharmaceutical preparations are prepared by mixing the active ingredient(s) with one or more pharmaceutically acceptable carrier(s) and then employing any method well-known in the technical field of pharmaceutics.

As for administration routes, it is preferred to select the most effective route of administration. Examples of the administration routes include oral administration and parenteral administration such as intravenous administration and the like.

As for the dosage form, for example, tablets, injections and the like are included.

For example, the tablet suitable for oral administration can be prepared with, for example, excipients such as lactose and mannitol; disintegrants such as starch; lubricants such as magnesium stearate; binders such as hydroxypropylcellulose; surfactants such as a fatty acid ester; plasticizers such as glycerol; and the like.

Preparations suitable for parenteral administration preferably comprise a sterilized aqueous preparation containing the active compound and being isotonic to blood of a recipient. For example, when an injection is prepared, a solution for injection is prepared by using a carrier consisting of a salt solution, glucose solution, a mixture of salt solution and glucose solution, or the like.

Also in these parenteral preparations, one or more kinds of auxiliary components selected from excipients, disintegrants, lubricants, binders, surfactants, plasticizers, diluents which are exemplified for the oral administration, preservatives, flavors and the like may be added.

Compound (I) or a pharmacologically acceptable salt thereof is generally administered systemically or locally in the form of an oral or parenteral preparation when used for the aforementioned purpose. The dose and the frequency of administration may vary depending on the administration form, the age and body weight of a patient, nature and severity of the condition to be treated, and the like. When oral administration is performed, generally 0.01 to 1,000 mg/kg, preferably 0.05 to 500 mg/kg per single administration for an adult may be administered once a day or a few times a day. When parenteral administration such as intravenous administration is performed, 0.001 to 1,000 mg/kg, preferably 0.01 to 300 mg/kg, per single administration for an adult may be administered once a day or a few times a day, or may be continuously administered intravenously for 1 to 24 hours a day. However, the dose and the frequency of administration may vary depending on the aforementioned various conditions and the like.

### Examples

The present invention will be explained in detail with reference to the following examples and formulation examples.

The spectra of proton nuclear magnetic resonance (¹H NMR) used in Examples were measured at 270 or 300 MHz, and exchangeable hydrogen may not always be clearly observed depending on the compound and the measurement conditions. For the descriptions of the multiplicity of signals, those generally applied are used, and the symbol "br" represents an apparent broad signal.

### Example 1 (Compound 1)

Compound A (1.50 g, 5.01 mmol) prepared in Reference Example 1 was dissolved in toluene (30 mL). To the solution was added 2-(tributylstanyl)furan (2.37 mL, 7.52 mmol) and tetrakis(triphenylphosphine)palladium (289 mg, 0.251 mmol), and the mixture was stirred at 100°C for 5 hours. To the reaction mixture was added 10% aqueous ammonium fluoride, and the mixture was extracted with ethyl acetate. The organic layer was washed with brine, and then dried over anhydrous sodium sulfate. The solvent was evaporated under reduced pressure, and the residue was purified by silica gel column chromatography (n-hexane/ethyl acetate = 9/1 → 6/1, then chloroform/methanol = 500/1) to give Compound 1 (1.14 g, yield: 79%). APCI-MS m/z: 287 [M+H]⁺; ¹H-NMR (CDCl₃) δ (ppm): 2.40 (s, 3H), 2.46 (s, 3H), 6.50 (dd, J = 1.8, 3.5 Hz, 1H), 6.76 (d, J = 3.6 Hz, 1H), 7.31 (m, 3H), 7.50 (m, 2H), 7.51 (dd, J = 1.8, 3.8 Hz, 1H).

### Example 2 (Compound 2)

In accordance with the method described in Example 1, Compound 2 (61 mg, yield: 76%) was obtained from Compound A (80 mg, 0.27 mmol) prepared in Reference Example 1, and 2-(tributylstanyl)pyrazine (148 mg, 0.401 mmol). APCI-MS m/z: 299 [M+H]⁺; ¹H-NMR (CDCl₃) δ (ppm): 2.45 (s, 3H), 2.49 (s, 3H), 7.25-7.39 (m, 3H), 7.50 (m, 2H), 8.55 (m, 2H), 9.29 (s, 1H).

### Example 3 (Compound 3)

In accordance with the method described in Example 1, Compound 3 (56 mg, yield: 79%) was obtained from Compound A (70 mg, 0.23 mmol), and 2-(tributylstanyl)thiophene (0.11 mL, 0.35 mmol). APCI-MS m/z: 303 [M+H]⁺; ¹H-NMR (CDCl₃) δ (ppm): 2.40 (s, 3H), 2.47 (s, 3H), 7.03 (dd, J = 3.8, 5.1 Hz, 1H), 7.17 (dd, J = 1.2, 3.8 Hz, 1H), 7.24-7.38 (m, 3H), 7.42 (dd, J = 1.2, 5.1 Hz, 1H), 7.50 (m, 2H).

### Example 4 (Compound 4)

In accordance with the method described in Reference Example 4, Compound 4 (1.84 g, yield: 91%) was obtained from Compound A (2.00 g, 6.68 mmol) prepared in Reference Example 1, and 3-thienylboronic acid (1.71 g, 13.4 mmol). APCI-MS m/z: 303 [M+H]⁺; ¹H-NMR (CDCl₃) δ (ppm): 2.40 (s, 3H), 2.47 (s, 3H), 7.27-7.38 (m, 4H), 7.45-7.50 (m, 4H).

### Example 5 (Compound 5)

In accordance with the method described in Reference Example 4, Compound 5 (11 mg, yield: 41%) was obtained from Compound A (20 mg, 0.067 mmol) prepared in Reference Example 1, and 1-(tert-butoxycarboxyl)pyrrole-2-boronic acid (28.2 mg, 0.134 mmol).
FAB-MS m/z: 386 [M+H]⁺; ¹H-NMR (CDCl₃) δ (ppm): 1.61 (s, 9H), 2.33 (s, 3H), 2.47 (s, 3H), 6.20 (dd, J = 3.3, 3.5 Hz, 1H), 6.58 (dd, J = 1.6, 3.4 Hz, 1H), 7.26-7.38 (m, 4H), 7.52 (m, 2H).

### Example 6 (Compound 6)

In accordance with the method described in Reference Example 4, Compound 6 (56 mg, yield: 67%) was obtained from Compound B (80 mg, 0.20 mmol) prepared in Reference Example 2, and 3-fluorophenylboronic acid (57 mg, 0.41 mmol).
FAB-MS m/z: 406 [M-H]; ¹H-NMR (CDCl₃) δ (ppm): 2.48 (s, 3H), 2.95 (s, 3H), 4.11 (dd, J = 6.4, 14.0 Hz, 1H), 4.70 (dd, J = 7.3, 14.2 Hz, 1H), 5.41 (t, J = 6.9 Hz, 1H), 7.15 (m, 1H), 7.29-7.52 (m, 8H).

### Example 7 (Compound 7)

In accordance with the method described in Reference Example 4, Compound 7 (4.9 mg, yield: 12%) was obtained from Compound C (40 mg, 0.092 mmol) prepared in Reference Example 3, and 2-fluorophenylboronic acid (26 mg, 0.18 mmol).
APCI-MS m/z: 450 [M+H]⁺; ¹H-NMR (CDCl₃) δ (ppm): 1.42 (s, 9H), 3.00 (s, 3H), 4.12 (dd, J = 5.4, 13.7 Hz, 1H), 4.75 (dd, J = 8.2, 13.5 Hz, 1H), 4.97 (dd, J = 5.3, 7.9 Hz, 1H), 7.12-7.48 (m, 8H), 7.75 (ddd, J = 1.7, 7.6, 7.6 Hz, 1H).

### Example 8 (Compound 8)

### Step 1

2-Fluorobenzoic acid (5.00 g, 35.7 mmol) was dissolved in DMF (75 mL). To the solution was added EDC • HCl (8.21 g, 42.8 mmol), tert-butyl carbazate (5.66 g, 42.8 mmol) and 4-dimethylaminopyridine (436 mg, 3.57 mmol), and the mixture was stirred at 0°C to room temperature for 24 hours. To the reaction mixture was added water, and the mixture was extracted with ethyl acetate. The organic layer was washed with brine, and then dried over anhydrous sodium sulfate. The solvent was evaporated under reduced pressure, and the residue was purified by silica gel column chromatography (hexane/ethyl acetate = 2/1) to give tert-butyl N'-(2-fluorobenzoyl)carbazate (6.23 g, yield: 69%).
APCI-MS m/z: 253 [M-H]-; ¹H-NMR (CDCl₃) δ (ppm): 1.50 (s, 9H), 6.75 (br s, 1H), 7.15 (ddd, J = 1.0, 8.4, 12.0 Hz, 1H), 7.28 (ddd, J = 1.0, 7.6, 7.6 Hz, 1H), 7.52 (m, 1H), 8.11 (ddd, J = 1.8, 7.7, 7.7 Hz, 1H), 8.37 (br d, J = 10.9 Hz, 1H).

### Step 2

tert-Butyl N'-(2-fluorobenzoyl)carbazate (8.29 g, 32.6 mmol) prepared in Step 1 mentioned above was dissolved in THF (166 mL). To the solution was added the Lawesson's regent (13.5 g, 33.3 mmol), and the mixture was stirred at 45°C for 3.2 hours. Then, the solvent was evaporated under reduced pressure, and the residue was purified by silica gel column chromatography (hexane/ethyl acetate = 4/1→3/1) to give tert-butyl N'-(2-fluorothiobenzoyl)carbazate (8.13 g, yield: 92%).
¹H-NMR (CDCl₃) δ (ppm): 1.53 (s, 9H), 7.12 (ddd, J = 1.0, 8.3, 12.5 Hz, 1H), 7.25 (m, 1H), 7.46 (m, 1H), 8.29 (ddd, J = 1.8, 8.1, 8.1 Hz, 1H), 9.21 (br s, 1H), 10.44 (br s, 1H).

### Step 3

tert-Butyl N'-(2-fluorothiobenzoyl)carbazate (8.13 g, 30.1 mmol) prepared in Step 2 mentioned above was dissolved in dichloromethane (180 mL). To the solution was added trifluoroacetic acid (50 mL), and the mixture was stirred at room temperature for 2 hours. Then, the solvent was evaporated under reduced pressure to give (2-fluorothiobenzoyl)hydrazine trifluoroacetate (7.86 g, yield: 92%).

### Step 4

2-(Methylsulfonylamino)acetophenone (1.00 g, 4.69 mmol) was dissolved in ethanol (30 mL). To the solution was added (2-fluorothiobenzoyl)hydrazine trifluoroacetate (1.47 g, 5.16 mmol) prepared in Step 3 mentioned above, and the mixture was stirred under reflux for 8 hours. Then, the solvent was evaporated under reduced pressure, and the residue was purified by silica gel column chromatography (chloroform/acetone = 30/1 → 15/1) to give N-[5-(2-fluorophenyl)-2-phenyl-2,3-dihydro[1,3,4]thiadiazol-2-ylmethyl]methanesulfonamide (1.17 g, yield: 68%).
¹H-NMR (CDCl₃) δ (ppm): 2.91 (s, 3H), 3.73 (dd, J = 5.6, 13.8 Hz, 1H), 3.86 (dd, J = 7.7, 14.0 Hz, 1H), 4.76 (dd, J = 5.6, 7.6 Hz, 1H), 6.74 (s, 1H), 7.06-7.19 (m, 2H), 7.31-7.45 (m, 4H), 7.51 (m, 2H), 7.76 (ddd, J = 1.6, 7.6, 7.6 Hz, 1H).

### Step 5

N-[5-(2-Fluorophenyl)-2-phenyl-2,3-dihydro[1,3,4]thiadiazol-2-ylmethyl]methanesulfonamide (1.17 g, 3.19 mmol) prepared in Step 4 mentioned above was dissolved in THF (35 mL). To the solution was added 4-dimethylaminopyridine (585 mg, 4.79 mmol) and acetyl chloride (0.340 mL, 4.79 mmol), and the mixture was stirred at room temperature for 1 hour. To the reaction mixture was added water, and the mixture was extracted with ethyl acetate. The organic layer was washed with brine, and then dried over anhydrous sodium sulfate. The solvent was evaporated under reduced pressure, and the residue was purified by silica gel column chromatography (chloroform/methanol = 50/1), and then by preparative thin layer chromatography (chloroform/acetonitrile = 9/1) to give Compound 8 (762 mg, yield: 59%).
ESI-MS m/z: 408 [M+H]⁺; ¹H-NMR (CDCl₃) δ (ppm): 2.50 (s, 3H), 2.99 (s, 3H), 4.10 (dd, J = 5.6, 14.2 Hz, 1H), 4.71 (dd, J = 8.1, 14.0 Hz, 1H), 5.20 (m, 1H), 7.10-7.36 (m, 2H), 7.39-7.46 (m, 6H), 7.83 (ddd, J = 1.6, 7.6, 7.6 Hz, 1H).
Example 9 (Compound 9)

### Step 1

Carbon disulfide (2.7 mL, 45 mmol) was dissolved in THF (30 mL). To the solution was added dropwise 3-tolyl magnesium bromide (1 mol/L solution in THF, 30 mL, 30 mmol) at 0°C, and then stirred at 0°C to room temperature for 1 hour. To the reaction mixture was added water (5 mL), and then the solvent was evaporated under reduced pressure. Then, to the residue was added water (30 mL), chloroacetic acid (3.40 g, 36 mmol) and sodium hydrogencarbonate (2.39 g, 28.5 mmol), and the mixture was stirred at 70°C for 4.5 hours. To the reaction mixture was added saturated aqueous sodium hydrogencarbonate, and the mixture was washed with ethyl acetate. The aqueous layer was made acidic by addition of 20% aqueous sulfuric acid, and then extracted with ethyl acetate. The organic layer was washed with brine, and then dried over anhydrous sodium sulfate. The solvent was evaporated under reduced pressure to give S-[3-methyl(thiobenzoyl)]thioglycolic acid (5.77 g, yield: 85%). FAB-MS m/z: 227 [M+H]⁺; ¹H-NMR (CDCl₃) δ (ppm): 2.39 (s, 3H), 4.27 (s, 2H), 7.30 (m, 1H), 7.38 (m, 1H), 7.83 (m, 2H).

### Step 2

To S-[3-methyl(thiobenzoyl)]thioglycolic acid (2.55 g, 11.3 mmol) prepared in Step 1 mentioned above was added water (35 mL) and sodium hydroxide (473 mg, 11.8 mmol), and dissolved. Then, to the solution was added hydrazine monohydrate (1.09 mL, 22.5 mmol), and the mixture was stirred at 0°C for 2.5 hours. The deposited solid was collected by filtration, washed with water, and then dried under reduced pressure to give [3-methyl(thiobenzoyl)lhydrazine (1.19 g, yield: 64%).

APCI-MS m/z: 165 [M-H]-; ¹H-NMR (CDCl₃) δ (ppm): 2.35 (s, 3H), 5.01 (br s, 2H), 7.29 (m, 2H), 7.46 (m, 1H), 7.56 (m, 1H), 8.75 (br s, 1H).

### Steps 3 and 4

In accordance with the methods described in Example 8, Steps 4 and 5, Compound 9 (55 mg, yield: 38%, for 2 steps) was obtained from [3-methyl(thiobenzoyl)]hydrazine (59 mg, 0.36 mmol) prepared in Step 2 mentioned above and 2-(methylsulfonylamino)acetophenone (84 mg, 0.40 mmol).
APCI-MS m/z: 402 [M-H]-; ¹H-NMR (CDCl₃) δ (ppm): 2.38 (s, 3H), 2.49 (s, 3H), 2.94 (s, 3H), 4.11 (dd, J = 6.2, 13.9 Hz, 1H), 4.70 (dd, J = 7.3, 13.9 Hz, 1H), 5.43 (m, 1H), 7.24-7.40 (m, 7H), 7.44 (m, 2H).

### Example 10 (Compound 10)

### Step 1

S-(Thiobenzoyl)thioglycolic acid (2.00 g, 9.42 mmol) was suspended in water (20 mL). To the suspension was added sodium hydroxide (396 mg, 9.89 mmol), and dissolved. To the resulting solution was added dropwise hydrazine monohydrate (0.914 mL, 18.8 mmol) at 0°C, and the mixture was further stirred at 0°C for 2 hours. The deposited solid was collected by filtration, washed with water, and then dried under reduced pressure to give thiobenzoylhydrazine (469 mg, yield: 33%).

APCI-MS m/z: 151 [M-H]⁻; ¹H-NMR (CDCl₃) δ (ppm): 5.01 (br s, 2H), 7.27-7.52 (m, 3H), 7.71 (m, 1H), 8.02 (m, 1H), 8.72 (br s, 1H).

### Steps 2 and 3

In accordance with the methods of described in Steps 4 and 5 of Example 8, Compound 10 (20 mg, yield: 31%, for 2 steps) was obtained from thiobenzoylhydrazine (26 mg, 0.17 mmol), 2-(methylsulfonylamino)acetophenone (73 mg, 0.34 mmol) and acetyl chloride (0.0094 mL, 0.13 mmol).
ESI-MS m/z: 390 [M+H]⁺; ¹H-NMR (CDCl₃) δ (ppm): 2.50 (s, 3H), 2.96 (s, 3H), 4.12 (dd, J = 6.1, 13.9 Hz, 1H), 4.71 (dd, J = 7.4, 14.0 Hz, 1H), 5.34 (dd, J = 6.3, 6.6 Hz, 1H), 7.29-7.49 (m, 8H), 7.65 (m, 2H).

### Example 11 (Compound 11)

In accordance with the methods described in Steps 4 and 5 of Example 8, Compound 11 (19 mg, yield: 15%, 2 steps) was obtained from thiobenzoylhydrazine (44 mg, 0.29 mmol), 2-(methylsulfonylamino)acetophenone (124 mg, 0.582 mmol) and pivaloyl chloride (0.0187 mL, 0.152 mmol).
APCI-MS m/z: 432 [M+H]⁺; ¹H-NMR (CDCl₃) δ (ppm): 1.43 (s, 9H), 2.97 (s, 3H), 4.14 (dd, J = 5.5, 13.6 Hz, 1H), 4.75 (dd, J = 8.1, 13.7 Hz, 1H), 5.01 (dd, J = 5.8, 7.8 Hz, 1H), 7.28-7.50 (m, 8H), 7.65 (m, 2H).

### Example 12 (Compound 12)

Compound A (50 mg, 0.17 mmol) prepared in Reference Example 1 was dissolved in toluene (1.5 mL). To the solution was added phenylacetylene (0.092 mL, 0.84 mmol), triethylamine (0.17 mL, 0.84 mmol), dichlorobis(triphenylphosphine)-palladium (5.9 mg, 0.0084 mmol) and copper iodide (6.4 mg, 0.033 mol), and the mixture was stirred at room temperature for 2.8 hours under an argon atmosphere. To the reaction mixture was added water, and the mixture was extracted with ethyl acetate. The organic layer was washed with brine, and then dried over anhydrous sodium sulfate. The solvent was evaporated under reduced pressure, and the residue was purified by preparative thin layer chromatography (hexane/ethyl acetate = 5/1) to give Compound 12 (39 mg, yield: 72%).
APCI-MS m/z: 321 [M+H]⁺; ¹H-NMR (CDCl₃) δ (ppm): 2.37 (s, 3H), 2.46 (s, 3H), 7.26-7.42 (m, 6H), 7.48-7.56 (m, 4H).

### Example 13 (Compound 13)

In accordance with the method described in Example 12, Compound 13 (50 mg, yield: 70%) was obtained from Compound A (70 mg, 0.23 mmol) prepared in Reference Example 1, and 1-dimethylamino-2-propyne (0.126 mL, 1.17 mmol).
APCI-MS m/z: 302 [M+H]⁺; ¹H-NMR (CDCl₃) δ (ppm): 2.32 (s, 3H), 2.33 (s, 6H), 2.41 (s, 3H), 3.48 (s, 2H), 7.24-7.38 (m, 3H), 7.44 (m, 2H).

### Example 14 (Compound 14)

In accordance with the method described in Example 12, Compound 14 (51 mg, yield: 75%) was obtained from Compound A (70 mg, 0.23 mmol) prepared in Reference Example 1, and methyl propargyl ether (0.099 mL, 1.2 mmol).
APCI-MS m/z: 289 [M+H]⁺; ¹H-NMR (CDCl₃) δ (ppm): 2.40 (s, 3H), 2.42 (s, 3H), 3.42 (s, 3H), 4.29 (s, 2H), 7.27-7.38 (m, 3H), 7.44 (m, 2H).

### Example 15 (Compound 15)

In accordance with the method described in Example 12, Compound 15 (34 mg, yield: 46%) was obtained from Compound A (80 mg, 0.27 mmol) prepared in Reference Example 1, and propargyl alcohol (0.078 mL, 1.3 mmol).
FAB-MS m/z: 275 [M+H]⁺; ¹H-NMR (CDCl₃) δ (ppm): 2.34 (s, 3H), 2.42 (s, 3H), 2.51 (m, 1H), 4.36 (d, J = 5.6 Hz, 2H), 7.26-7.39 (m, 3H), 7.45 (m, 2H).

### Example 16 (Compound 16)

In accordance with the method described in Example 12, Compound 16 (65 mg, yield: 81%) was obtained from Compound A (80 mg, 0.27 mmol) prepared in Reference Example 1, and 3,3-dimethyl-1-butyne (0.314 mL, 2.55 mmol).
FAB-MS m/z: 301 [M+H]⁺; ¹H-NMR (CDCl₃) δ (ppm): 1.30 (s, 9H), 2.32 (s, 3H), 2.40 (s, 3H), 7.23-7.43 (m, 3H), 7.46 (m, 2H).

### Example 17 (Compound 17)

In accordance with the method described in Example 12, Compound 17 (46 mg, yield: 57%) was obtained from Compound A (80 mg, 0.27 mmol) prepared in Reference Example 1, and 1-hexyne (0.307 mL, 2.67 mmol).
APCI-MS m/z: 301 [M+H]⁺; ¹H-NMR (CDCl₃) δ (ppm): 0.93 (t, J = 7.2 Hz, 3H), 1.44 (m, 2H), 1.56 (m, 2H), 2.32 (s, 3H), 2.40 (s, 3H), 2.41 (t, J = 6.9 Hz, 2H), 7.23-7.37 (m, 3H), 7.44 (m, 2H).

### Example 18 (Compound 18)

In accordance with the method described in Example 12, Compound 18 (62 mg, yield: 71%) was obtained from Compound A (80 mg, 0.27 mmol) prepared in Reference Example 1, and 3-cyclopentyl-1-propyne (0.349 mL, 2.67 mmol).
APCI-MS m/z: 327 [M+H]⁺; ¹H-NMR (CDCl₃) δ (ppm): 1.27 (m, 2H), 1.59 (m, 4H), 1.81 (m, 2H), 2.11 (m, 1H), 2.32 (s, 3H), 2.40 (s, 3H), 2.41 (d, J = 6.3 Hz, 2H), 7.23-7.37 (m, 3H), 7.44 (m, 2H).

### Example 19 (Compound 19)

In accordance with the method described in Example 12, Compound 19 (45 mg, yield: 53%) was obtained from Compound A (80 mg, 0.27 mmol) prepared in Reference Example 1, and 5-methyl-1-hexyne (0.348 mL, 2.67 mmol).
FAB-MS m/z: 315 [M+H]⁺; ¹H-NMR (CDCl₃) δ (ppm): 0.91 (d, J = 6.6 Hz, 6H), 1.49 (dd, J = 7.2, 14.5 Hz, 2H), 1.69 (m, 1H), 2.32 (s, 3H), 2.40 (s, 3H), 2.41 (t, J = 7.5 Hz, 2H), 7.24-7.38 (m, 3H), 7.44 (m, 2H).

### Example 20 (Compound 20)

In accordance with the method described in Example 12, Compound 20 (5.1 mg, yield: 13%) was obtained from Compound B (40 mg, 0.10 mmol) prepared in Reference Example 2, and 3,3-dimethyl-1-butyne (0.126 mL, 1.02 mmol).
FAB-MS m/z: 394 [M+H]⁺; ¹H-NMR (CDCl₃) δ (ppm): 1.29 (s, 9H), 2.40 (s, 3H), 3.00 (s, 3H), 4.02 (dd, J = 5.7, 14.0 Hz, 1H), 4.66 (dd, J = 7.9, 13.9 Hz, 1H), 5.11 (t, J = 6.3 Hz, 1H), 7.26-7.42 (m, 5H).

### Example 21 (Compound 21)

In accordance with the method described in Example 12, Compound 21 (21 mg, yield: 52%) was obtained from Compound C (40 mg, 0.092 mmol) prepared in Reference Example 3, and 3,3-dimethyl-1-butyne (0.113 mL, 0.920 mmol).
FAB-MS m/z: 436 [M+H]⁺; ¹H-NMR (CDCl₃) δ (ppm): 1.30 (s, 9H), 1.34 (s, 9H), 3.01 (s, 3H), 4.02 (dd, J = 5.4, 13.6 Hz, 1H), 4.69 (dd, J = 8.3, 13.6 Hz, 1H), 4.92 (m, 1H), 7.26-7.37 (m, 5H).

### Example 22 (Compound 35)

### Step 1

Copper bromide (17.1 g, 76.5 mmol) was dissolved in acetonitrile (225 mL). To the solution was added tert-butyl nitrite (12.1 mL, 102 mmol) at 0°C. After the mixture was stirred for 10 minutes, to the mixture was added 3-acetyl-5-amino-2,3-dihydro-2-methyl-2-phenyl-1,3,4-thiadiazole (15.0 g, 63.8 mmol) prepared in accordance with the method described in WO03/051854, and the mixture was stirred at 0°C to room temperature for 4.8 hours. To the reaction mixture was added water, and the mixture was extracted with ethyl acetate. The organic layer was washed with brine, and then dried over anhydrous sodium sulfate. The solvent was evaporated under reduced pressure, and the residue was purified by silica gel column chromatography (n-hexane/ethyl acetate = 8/1 → 6/1) to give 3-acetyl-5-bromo-2,3-dihydro-2-methyl-2-phenyl-1,3,4-thiadiazole (15.4 g, yield: 81%).
FAB-MS m/z: 299 [M+H]⁺; ¹H-NMR (CDCl₃) δ (ppm): 2.29 (s, 3H), 2.44 (s, 3H), 7.32 (m, 3H), 7.46 (m, 2H).

### Step 2

3-Acetyl-5-bromo-2,3-dihydro-2-methyl-2-phenyl-1,3,4-thiadiazole (2.00 g, 6.68 mmol) prepared above was dissolved in toluene (40 mL). To the solution was added tributyl(vinyl)tin (2.92 mL, 9.99 mol) and tetrakis(triphenylphosphine)palladium (0.384 g, 0.332 mmol), and the mixture was stirred at 100°C for 7 hours. To the reaction mixture was added 5% aqueous ammonium fluoride, and the mixture was extracted with ethyl acetate. The organic layer was washed with brine, then dried over anhydrous sodium sulfate, and concentrated under reduced pressure. The residue was purified by silica gel column chromatography (n-hexane/ethyl acetate = 6/4, and then n-hexane/ethyl acetate = 96/4) to give Compound 35 (1.14 g, yield: 69%).
APCI-MS m/z: 247 [M+H]⁺; ¹H-NMR (CDCl₃) δ (ppm): 2.33 (s, 3H), 2.41 (s, 3H), 5.45 (d, J = 17.5 Hz, 1H), 5.65 (d, J = 10.7 Hz, 1H), 6.63 (dd, J = 10.9, 17.5 Hz, 1H), 7.30 (m, 3H), 7.44 (m, 2H).

### Example 23 (Compound 36)

3-Acetyl-5-bromo-2,3-dihydro-2-methyl-2-phenyl-1,3,4-thiadiazole (100 mg, 0.334 mmol) prepared in Step 1 of Example 22 mentioned above was dissolved in toluene (3 mL). To the solution was added tributyl(1-ethoxyvinyl)tin (0.169 mL, 0.501 mmol) and bis(triphenylphosphine)palladium chloride (12 mg, 0.017 mmol), and the mixture was stirred at 100°C for 2 hours. To the reaction mixture was added 5% aqueous ammonium fluoride, and the mixture was extracted with ethyl acetate. Then, to the organic layer was added 2 mol/L hydrochloric acid (10 mL), and the mixture was stirred at room temperature for 80 minutes. The organic layer was washed with brine, then dried over anhydrous sodium sulfate, and concentrated under reduced pressure. The residue was purified by preparative thin layer chromatography (n-hexane/ethyl acetate = 3/1, and then chloroform/methanol = 300/1) to give Compound 36 (74 mg, yield: 84%).
FAB-MS m/z: 263 [M+H]⁺; ¹H-NMR (CDCl₃) δ (ppm): 2.39 (s, 3H), 2.41 (s, 3H), 2.55 (s, 3H), 7.25-7.45 (m, 5H).

### Example 24 (Compound 37)

Compound 35 (53 mg, 0.21 mmol) prepared in Example 22 and phthalimide (31 mg, 0.21 mmol) were dissolved in DMSO (0.4 mL). To the solution was added sodium methoxide (0.029 mL, 28% solution in methanol), and the mixture was stirred at room temperature for 18 hours. To the mixture was added sodium methoxide (0.82 mL, 28% solution in methanol), and the mixture was further stirred at room temperature for 46 hours. Then, to the mixture was added water, and the mixture was extracted with ethyl acetate. The organic layer was washed with brine, then dried over anhydrous sodium sulfate, and concentrated under reduced pressure. The residue was purified by preparative thin layer chromatography (n-hexane/ethyl acetate = 2/1) to give Compound 37 (23 mg, yield: 27%).
ESI-MS m/z: 394 [M+H]⁺; ¹H-NMR (CDCl₃) δ (ppm): 2.09 (s, 3H), 2.35 (s, 3H), 2.88 (m, 2H), 4.04 (t, J = 6.9 Hz, 2H), 7.19-7.45 (m, 5H), 7.69-7.82 (m, 2H), 7.83-7.94 (m, 2H).

### Example 25 (Compound 38)

Compound 37 (15 mg, 0.038 mmol) prepared in Example 24 was dissolved in ethanol (0.6 mL). To the solution was added hydrazine monohydrate (0.006 mL, 0.1 mmol), and the mixture was stirred at 60°C for 2.5 hours. The deposited precipitates were filtrated off, and then the filtrate was concentrated under reduced pressure.

The residue was purified by preparative thin layer chromatography (chloroform/methanol = 6/1) to give Compound 38 (5.0 mg, yield: 50%).
ESI-MS m/z: 264 [M+H]⁺; ¹H-NMR (CD₃OD) δ (ppm): 2.30 (s, 3H), 2.37 (s, 3H), 2.71 (t, J = 6.9 Hz, 2H), 3.04 (t, J = 6.9 Hz, 2H), 7.23-7.54 (m, 5H).

### Example 26 (Compound 39)

Compound 35 (30 mg, 0.12 mmol) prepared in Example 22 was dissolved in THF (0.4 mL). To the solution was added a 70% aqueous solution of ethylamine (0.197 mL), and the mixture was stirred at room temperature for 18.5 hours, and then at 60°C for 8.5 hours. The reaction mixture was concentrated under reduced pressure, and the residue was purified by preparative thin layer chromatography (chloroform/methanol = 9/1) to give Compound 39 (2.4 mg, yield: 7%).
ESI-MS m/z: 292 [M+H]⁺; ¹H-NMR (DMSO-d₆) δ (ppm): 1.01 (t, J = 7.1 Hz, 3H), 2.21 (s, 3H), 2.29 (s, 3H), 2.59 (m, 2H), 2.64 (t, J = 7.6 Hz, 2H), 2.79 (t, J = 7.1 Hz, 2H), 7.19-7.45 (m, 5H).

### Example 27 (Compound 40)

Compound 35 (34 mg, 0.14 mmol) prepared in Example 22 and methanesulfonamide (26 mg, 0.27 mmol) was dissolved in DMF (0.5 mL). To the solution was added sodium hydride (11 mg, 0.28 mmol, 60% oil), and the mixture was stirred at 90°C for 3.5 hours. To the reaction mixture was added water and 1 mol/L hydrochloric acid, and the mixture was extracted with ethyl acetate. The organic layer was washed with brine, then dried over anhydrous sodium sulfate, and concentrated under reduced pressure. The residue was purified by preparative thin layer chromatography (n-hexane/ethyl acetate = 1/2) to give Compound 40 (15 mg, yield: 31%).
ESI-MS m/z: 340 [M-H]⁻; ¹H-NMR (CDCl₃) δ (ppm): 2.31 (s, 3H), 2.40 (s, 3H), 2.74 (t, J = 6.3 Hz, 2H), 2.96 (s, 3H), 3.46 (dt, J = 6.3, 6.3 Hz, 2H), 4.83 (brt, J = 6.0 Hz, 1H), 7.25-7.46 (m, 5H).

### Example 28 (Compound 41)

Compound 35 (823 mg, 3.34 mmol) prepared in Example 22 was dissolved in a mixed solvent of pyridine (10 mL) and DMF (5 mL). To the solution was added potassium permanganate (1.06 g, 6.71 mmol) dissolved in water (16 mL) at 0°C over 10 minutes with stirring. To the reaction mixture was added pyridine (50 mL), and the mixture was further stirred at 0°C for 20 minutes. Then, to the mixture was successively added 10% aqueous sodium sulfite and 20% sulfuric acid, and the mixture was extracted with chloroform. The organic layer was washed with brine, then dried over anhydrous sodium sulfate, and concentrated under reduced pressure. Then, the residue was dissolved in a mixed solvent of dichloromethane (20 mL) and methanol (20 mL). To the solution was added (trimethylsilyl)diazomethane (6.0 mL, 12 mmol, 2.0 mol/L solution in n-hexane), and the mixture was stirred at room temperature for 40 minutes. The reaction mixture was concentrated under reduced pressure, and the residue was purified by silica gel column chromatography (chloroform/methanol = 50/1) and then purified by preparative thin layer chromatography (n-hexane/ethyl acetate = 1/1) to give Compound 41 (75 mg, yield: 8%).
ESI-MS m/z: 279 [M+H]⁺; ¹H-NMR (CDCl₃) δ (ppm): 2.39 (s, 3H), 2.43 (s, 3H), 3.92 (s, 3H), 7.27-7.50 (m, 5H).

### Example 29 (Compound 42)

Compound 41 (63 mg, 0.23 mmol) prepared in Example 28 was dissolved in a mixed solvent of methanol (2 mL) and water (1 mL). To the solution was added lithium hydroxide (54 mg, 2.3 mmol), and the mixture was stirred at room temperature for 2 hours. To the reaction mixture was added 1 mol/L hydrochloric acid, and the mixture was extracted with ethyl acetate. The organic layer was washed with brine, then dried over anhydrous sodium sulfate, and concentrated under reduced pressure. The residue was triturated with diisopropyl ether to give Compound 42 (23 mg, yield: 38%).
ESI-MS m/z: 263 [M-H]⁻; ¹H-NMR (CDCl₃) δ (ppm): 2.40 (s, 3H), 2.45 (s, 3H), 6.92 (br, 1H), 7.26-7.51 (m, 5H).

### Example 30 (Compound 43)

Compound 42 (6.0 mg, 0.023 mmol) prepared in Example 29 was dissolved in DMF (0.5 mL). To the solution was added EDC • HCl (5.2 mg, 0.028 mmol) and HOBt • H₂O (4.2 mg, 0.027 mmol) at 0°C, and the mixture was stirred at 0°C for 20 minutes. Then, to the mixture was added 50% aqueous dimethylamine (0.004 mL), and the mixture was stirred at room temperature for 9 hours. Then, the reaction mixture was concentrated under reduced pressure. The residue was purified by preparative thin layer chromatography (chloroform/methanol = 20/1) to give Compound 43 (3.0 mg, yield: 45%).
APCI-MS m/z : 292 [M+H]⁺; ¹H-NMR (CDCl₃) δ (ppm): 2.31 (s, 3H), 2.41 (s, 3H), 3.10 (s, 3H), 3.39 (s, 3H), 7.24-7.48 (m, 5H).

### Example 31 (Compound 44)

In accordance with the method described in Example 30, Compound 44 (19 mg, yield: 48%) was obtained from Compound 42 (33 mg, 0.13 mmol), EDC • HCl (29 mg, 0.15 mmol), HOBt • H₂O (23 mg, 0.15 mmol), and tert-butylamine (0.020 mL, 0.19 mmol).
ESI-MS m/z: 320 [M+H]⁺; ¹H-NMR (CDCl₃) δ (ppm): 1.44 (s, 9H), 2.33 (s, 3H), 2.41 (s, 3H), 6.43 (brs, 1H), 7.25-7.41 (m, 3H), 7.46 (d, J = 7.3 Hz, 2H).

### Example 32 (Compound 45)

In accordance with the method described in Example 30, Compound 45 (11 mg, yield: 59%) was obtained from Compound 42 (16 mg, 0.061 mmol), EDC • HCl (15 mg, 0.078 mmol), HOBt • H₂O (12 mg, 0.078 mmol), and pyrrolidine (0.008 mL, 0.1 mmol). APCI-MS m/z: 318 [M+H]⁺; ¹H-NMR (CDCl₃) δ (ppm): 1.93 (m, 2H), 2.02 (m, 2H), 2.32 (s, 3H), 2.41 (s, 3H), 3.62 (t, J = 6.8 Hz, 2H), 3.89 (t, J = 6.8 Hz, 2H), 7.23-7.50 (m, 5H).

### Example 33 (Compound 46)

### Step 1

N-tert-Butoxycarboxylglycine (10.0 g, 57.1 mmol) was dissolved in THF (100 mL). To the solution was added CDI (14.3 g, 59.0 mmol) at room temperature, and the mixture was stirred for 30 minutes. To the reaction mixture was added N,O-dimethylhydroxylamine hydrochloride (6.2 g, 64.0 mmol), and the mixture was further stirred at room temperature for 12 hours. To the reaction mixture was added water, and the mixture was extracted with ethyl acetate. The organic layer was washed with brine, and then dried over anhydrous sodium sulfate, and the solvent was evaporated under reduced pressure to give [(methoxymethylcarbamoyl)methyl]carbamic acid tert-butyl ester (9.55 g, yield: 77%). APCI-MS m/z: 218 [M+H]⁺.

### Step 2

[(Methoxymethylcarbamoyl)methyl]carbamic acid tert-butyl ester (9.55g, 43.8 mmol) prepared in Step 1 mentioned above was dissolved in THF (300 mL). To the solution was added a 2.0 mol/L solution of isopropyl magnesium chloride in THF (18.4 mL, 36.8 mmol) at -12°C, and the mixture was stirred for 15 minutes. Then, to the mixture was added a 2.0 mol/L solution of for phenyl magnesium chloride in THF (21.3 mL, 42.7 mmol) at -10°C, and the mixture was stirred at room temperature for 3 hours. To the reaction mixture was added acetic acid (5.6 mL), and the solvent was evaporated under reduced pressure. To the residue was added water, and the mixture was extracted with ethyl acetate. The organic layer was washed with brine, and then dried over anhydrous sodium sulfate, and the solvent was evaporated under reduced pressure. The residue was purified by silica gel column chromatography (hexane/ethyl acetate = 6/1 → 4/1) to give (2-oxo-2-phenylethyl)carbamic acid tert-butyl ester (3.95 g, yield: 39%).
APCI-MS m/z: 235 [M+H]⁺.

### Step 3

In accordance with the method described in Step 4 of Example 8, [5-(2-fluorophenyl)-2-phenyl-2,3-dihydro[1,3,4]thiadiazol-2-ylmethyl]carbamic acid tert-butyl ester (1.63 g, yield: 45%) was obtained from (2-oxo-2-phenylethyl)-carbamic acid tert-butyl ester (2.20g, 9.36 mmol) prepared in Step 2 mentioned above, and (2-fluorothiobenzoyl)hydrazine trifluoroacetate (4.21 g, 14.8 mmol) prepared in Step 3 of Example 18.
APCI-MS m/z: 388 [M+H]⁺.

### Step 4

In accordance with the method described in Step 5 of Example 8, Compound 46 (0.43 g, yield: 28%) was obtained from [5-(2-fluorophenyl)-2-phenyl-2,3-dihydro[1,3,4]thiadiazol-2-ylmethyl]carbamic acid tert-butyl ester (1.36g, 3.51 mmol) prepared in Step 3 mentioned above.
APCI-MS m/z 430 [M+H]⁺; ¹H-NMR (270 MHz, CDCl₃) δ (ppm): 1.40 (s, 9H), 2.51 (s, 3H), 4.14 (dd, J = 6.1, 14.8 Hz, 1H), 4.68 (dd, J = 6.7, 14.8 Hz, 1H), 5.42 (m, 1H), 7.07-7.45 (m, 8H), 7.79 (ddd, J = 0.9, 7.2, 7.4 Hz, 1H).

### Example 34 (Compound 47)

Compound 46 (0.330 g, 0.768 mmol) prepared in Example 33 was dissolved in a 4 mol/L solution of hydrogen chloride in ethyl acetate (30 mL), and the solution was stirred at room temperature for 30 minutes. Then, the solvent was evaporated under reduced pressure, and the residue was reslurried in diisopropyl ether to give Compound 47 (0.28 g, yield: 100%).
APCI-MS m/z: 330 [M+H]⁺; ¹H-NMR (270 MHz, CDCl₃) δ (ppm): 2.53 (s, 3H), 3.83 (d, J = 14.3 Hz 1H), 4.60 (d, J = 14.3 Hz, 1H), 7.04-7.18 (m, 2H), 7.28-7.45 (m, 6H), 7.87 (ddd, J = 1.6, 7.6, 7.6 Hz, 1H), 8.93 (br s, 2H).

### Example 35 (Compound 48)

Compound 47 (169 mg, 0.462 mmol) prepared in Example 34 was dissolved in dichloromethane (5 mL). To the solution was added triethylamine (322 mL, 2.31 mmol) and 2-chloroethylsulfonyl chloride (0.097 mL, 0.924 mmol) at room temperature, and the mixture was stirred for 30 minutes. To the reaction mixture was added water (5 mL), and the mixture was extracted with ethyl acetate. The organic layer was washed with brine, and then dried over anhydrous sodium sulfate, and the solvent was evaporated under reduced pressure. The residue was purified by preparative thin layer chromatography (hexane/ethyl acetate = 3/2) to give Compound 48 (186 mg, yield: 96%).
APCI-MS m/z: 420 [M+H]⁺; ¹H-NMR (270 MHz, CDCl₃) δ (ppm): 2.50 (s, 3H), 3.98 (dd, J = 5.5, 13.9 Hz, 1H), 4.58 (dd, J = 7.6, 13.9 Hz, 1H), 5.92 (d, J = 9.9 Hz, 1H), 6.27 (d, J = 16.7 Hz, 1H), 6.53 (dd , J = 9.9, 16.7 Hz, 1H), 7.09-7.49 (m, 8H), 7.83 (dd, J = 1.8, 7.8 Hz, 1H).

### Example 36 (Compound 49)

Compound 48 (120 mg, 0.286 mmol) prepared in Example 35 was dissolved in a 7 mol/L solution of ammonia in methanol (2 mL), and the solution was stirred at room temperature for 30 minutes. The solvent was evaporated under reduced pressure, and the residue was purified by preparative thin layer chromatography (chloroform/methanol = 6/1) to give Compound 49 (89 mg, yield: 72%).
APCI-MS m/z: 437 [M+H]⁺.

### Example 37 (Compound 50)

Compound 48 (78.3 mg, 0.187 mmol) prepared in Example 35 was dissolved in acetonitrile (3 mL). To the solution was added dimethylamine hydrochloride (76.0 mg, 0.933 mmol) at room temperature, and the mixture was stirred at room temperature for 30 minutes. The solvent was evaporated under reduced pressure, and the residue was purified by preparative thin layer chromatography (chloroform/methanol = 6/1) to give Compound 50 (79 mg, yield: 91%).
APCI-MS m/z: 465 [M+H]⁺; ¹H NMR (270 MHz, CDCl₃) δ (ppm): 2.21 (s, 6H), 2.49 (s, 3H), 2.77 (m, 2H), 3.17 (m, 2H), 4.14 (d, J = 13.7 Hz, 1H), 4.65 (d, J = 13.7 Hz, 1H), 7.08-7.47 (m, 8H), 7.83 (ddd, J = 1.8, 7.6, 7.6 Hz, 1H).

### Example 38 (Compound 51)

Compound 48 (108 mg, 0.257 mmol) prepared in Example 35 was dissolved in acetonitrile (3 mL). To the solution was added cyclopropylamine (0.090 mL, 1.3 mmol) at room temperature, and the mixture was stirred at room temperature for 10 hours. The solvent was evaporated under reduced pressure, and the residue was purified by preparative thin layer chromatography (chloroform/methanol = 9/1) to give Compound 51 (87 mg, yield: 67%).
APCI-MS m/z: 477 [M+H]⁺

### Example 39 (Compound 52)

Compound 51 (87.4 mg, 0.183 mmol) prepared in Example 38 was dissolved in a mixed solvent of 1,2-dichloroethane (3 mL) and acetic acid (0.5 mL). To the solution was added acetaldehyde (0.030 mL, 0.55 mmol) and triacetoxy sodium borohydride (0.194 g, 0.915 mmol), and the mixture was stirred at room temperature for 2 hours. Then, to the reaction mixture was added water (5 mL), and the mixture was extracted with chloroform. The organic layer was washed with brine, and then dried over anhydrous sodium sulfate, and the solvent was evaporated under reduced pressure. The residue was purified by preparative thin layer chromatography (chloroform/methanol = 9/1) to give Compound 52 (56 mg, yield: 61%).
APCI-MS m/z: 505 [M+H]⁺; ¹H-NMR (270 MHz, CDCl₃) δ (ppm): 0.45 (m, 4H), 1.04 (t, J = 7.2 Hz, 3H), 1.71 (m, 1H), 2.50 (s, 3H), 2.63 (m, 2H), 3.10 (m, 2H), 3.24 (m, 2H), 4.11 (dd, J = 6.1, 13.9Hz, 1H), 4.63 (dd, J = 7.2, 13.9 Hz, 1H), 5.59 (t, J = 7.2 Hz, 1H), 7.08-7.47 (m, 8H), 7.83 (ddd, J = 1.8, 7.6, 7.6 Hz, 1H).

### Example 40 (Compound 53)

Compound 48 (87.0 mg, 0.176 mmol) prepared in Example 35 was dissolved in a mixed solvent of acetonitrile (2 mL) and methanol (2 mL). To the solution was added 2-aminoethanethiol (200 mg, 1.76 mmol) and saturated aqueous sodium hydrogencarbonate (2 mL), and the mixture was stirred at room temperature for 2 hours. Then, to the reaction mixture was added water (5 mL), and the mixture was extracted with chloroform. The organic layer was washed with brine, and then dried over anhydrous sodium sulfate, and the solvent was evaporated under reduced pressure. The residue was purified by preparative thin layer chromatography (chloroform/methanol = 6/1) to give Compound 53 (45 mg, yield: 45%).
APCI-MS m/z: 497 [M+H]⁺; ¹H-NMR (270 MHz, CDCl₃) δ (ppm): 2.50 (s, 3H), 2.76-2.98 (m, 4H), 3.26-3.45 (m, 4H), 4.11 (d, J = 14.2 Hz, 1H), 4.69 (d, J = 14.2 Hz, 1H), 5.61 (br s, 1H), 7.08-7.47 (m, 8H), 7.83 (ddd, J = 1.8, 7.7, 7.7 Hz, 1H).

### Example 41 (Compound 54)

Compound 47 (149 mg, 0.409 mmol) prepared in Example 34 was dissolved in DMF (5 mL). To the solution was added N-tert-butoxycarboxylglycine (208 mg, 1.19 mmol), EDC • HCl (171 mg, 1.10 mmol) and HOBt • H₂O (282 mg, 1.84 mmol), and the mixture was stirred at room temperature for 12 hours. Then, to the reaction mixture was added water (5 mL), and the mixture was extracted with ethyl acetate. The organic layer was washed with brine, and then dried over anhydrous sodium sulfate, and the solvent was evaporated under reduced pressure. The residue was purified by preparative thin layer chromatography (hexane/ethyl acetate = 1/1) to give Compound 54 (144 mg, yield: 73%).
APCI-MS m/z: 487 [M+H]⁺; ¹H-NMR (270 MHz, CDCl₃) δ (ppm): 1.39 (s, 9H), 2.53 (s, 3H), 3.70 (d, J = 5.8 Hz, 2H), 4.23 (dd, J = 4.8, 14.8 Hz, 1H), 4.85 (dd, J = 7.1, 14.8 Hz, 1H), 4.97 (m, 1H), 7.07-7.46 (m, 8H), 7.79 (ddd, J = 1.6, 7.7, 7.7 Hz, 1H).

### Example 42 (Compound 55)

Compound 54 (109 mg, 0.224 mmol) prepared in Example 41 was dissolved in a 4 mol/L solution of hydrogen chloride in ethyl acetate (2 mL), and the solution was stirred at room temperature for 30 minutes. The solvent was evaporated under reduced pressure, and the residue was reslurried in diisopropyl ether to give Compound 55 (43 mg, yield: 46%).
APCI-MS m/z: 387 [M+H]⁺; ¹H-NMR (270 MHz, CDCl₃) δ (ppm): 2.27 (s, 3H), 3.83 (m, 2H), 4.20 (dd, J = 6.2, 14.0 Hz, 1H), 4.78 (dd, J = 5.6, 14.0 Hz, 1H), 4.97 (m, 1H), 7.07-7.46 (m, 8H), 7.79 (ddd, J = 1.6, 7.7, 7.7 Hz, 1H), 8.43 (m, 2H).

### Example 43 (Compound 56)

Compound 47 (153 mg, 0.418 mmol) prepared in Example 34 was dissolved in dichloromethane (4 mL). To the solution was added pyridine (0.079 mL, 0.92 mmol) and p-nitrophenyl chloroformate (101 mg, 0.502 mmol), and the mixture was stirred at room temperature for 1 hour. Then, to the reaction mixture was added water (5 mL), and the mixture was extracted with chloroform. The organic layer was washed with brine, and then dried over anhydrous sodium sulfate, and the solvent was evaporated under reduced pressure. The residue was purified by preparative thin layer chromatography (n-hexane/ethyl acetate = 2/1) to give Compound 56 (205 mg, yield: 100%).
APCI-MS m/z: 495 [M+H]⁺.

### Example 44 (Compound 57)

Compound 56 (96.0 mg, 0.194 mmol) prepared in Example 43 was dissolved in dichloromethane (2 mL). To the solution was added N,N-dimethylethylenediamine (107 mg, 0.971 mmol), and the mixture was stirred at room temperature for 7 hours. The solvent was evaporated under reduced pressure, and the residue was purified by preparative thin layer chromatography (chloroform/methanol = 9/1) to give Compound 57 (12.2 mg, yield: 14%).
APCI-MS m/z: 444 [M+H]⁺; ¹H-NMR (270 MHz, CDCl₃) δ (ppm): 2.20 (s, 6H), 2.39 (t, J = 5.8 Hz, 2H), 2.53 (s, 3H), 3.22 (q, J = 5.8 Hz, 2H), 4.27 (dd, J = 5.4, 15.1 Hz, 1H), 4.74 (dd, J = 6.7, 15.1 Hz, 1H), 5.08 (m, 1H), 7.06-7.45 (m, 8H), 7.78 (ddd, J = 1.8, 7.7, 7.7 Hz, 1H).

### Example 45 (Compound 58)

In accordance with the method described in Example 44, Compound 58 (21.1 mg, yield: 23%) was obtained from Compound 56 (100 mg, 0.202 mmol) prepared in Example 43, and 4-aminomethylpyridine (0.0103 mL, 1.01 mmol).
APCI-MS m/z: 464 [M+H]⁺; ¹H-NMR (270 MHz, CDCl₃) δ (ppm): 2.49 (s, 3H), 4.07-4.43 (m, 3H), 4.78-4.95 (m, 2H), 5.84 (s, 1H), 7.06-7.45 (m, 10H), 7.78 (ddd, J = 1.6, 7.4, 7.4 Hz, 1H), 12.9 (m, 2H).

### Example 46 (Compound 59)

In accordance with the method described in Reference Example 6, Compound 59 (2.09 g, yield: 79%) was obtained from Compound D (2.66 g, 6.21 mmol), and 2-fluorophenylboronic acid (1.74 g, 12.4 mmol).
APCI-MS m/z: 444 [M+H]⁺: ¹H-NMR (270 MHz, CDCl₃) δ (ppm): 1.43 (s, 9H), 2.45 (s, 3H), 2.64 (m, 1H), 3.29 (m, 2H), 3.74 (m, 1H), 4.68 (m, 1H), 7.08-7.46 (m, 8H), 7.81 (ddd, J = 1.6, 7.4, 7.4 Hz, 1H).

### Example 47 (Compound 60)

In accordance with the method described in Example 34, Compound 60 (1.65 g, yield: 92%) was obtained from Compound 59 (2.09 g, 4.71 mmol) prepared in Example 46.
APCI-MS m/z: 344 [M+H]⁺; ¹H-NMR (270 MHz, DMSO-d₆) δ (ppm): 2.40 (s, 3H), 2.86 (m, 2H), 3.25 (m, 2H), 7.28-7.45 (m, 7H), 7.59 (m, 1H), 7.87 (ddd, J = 1.5, 8.1, 8.1 Hz, 1H).

### Example 48 (Compound 61)

In accordance with the method described in Example 41, Compound 61 (1.07 g, yield: 65%) was obtained from Compound 60 (1.37 g, 3.61 mmol) prepared in Example 47, and 4-(dimethylamino)lactic acid hydrochloride (1.80 g, 10.8 mmol).
APCI-MS m/z: 457 [M+H]⁺; ¹H-NMR (270 MHz, CDCl₃) δ (ppm): 2.22 (s, 6H), 2.20-2.35 (m, 5H), 2.72 (m, 1H), 3.28 (m, 2H), 3.91 (m, 2H), 6.66 (m, 1H), 7.08-7.47 (m, 8H).

### Example 49 (Compound 62)

Compound 34 (120 mg, 0.337 mmol) prepared in Reference Example 18 was dissolved in a mixed solution of 1,2-dichloroethane (4 mL) and acetic acid (0.67 mL). To the solution was added N-methylpiperazine (0.187 mL, 1.68 mmol) and triacetoxy sodium borohydride (0.428 g, 2.02 mmol), and the mixture was stirred at room temperature for 12 hours. To the reaction mixture was added water (5 mL), and the mixture was extracted with chloroform. The organic layer was washed with brine, and then dried over anhydrous sodium sulfate, and the solvent was evaporated under reduced pressure. The residue was purified by preparative thin layer chromatography (chloroform/methanol = 9/1) to give free base of Compound 62 (35 mg). The obtained free base of Compound 62 was dissolved in ethyl acetate (1 mL). To the solution was added a 4 mol/L solution of hydrogen chloride in ethyl acetate (1 mL), and the mixture was stirred for 1 hour. Then, to the mixture was added diethyl ether (5 mL), and the resulting solid was collected by filtration, and dried under reduced pressure to give Compound 62 (29 mg, yield: 20%).

APCI-MS m/z: 441 [M+H]⁺; ¹H NMR (270 MHz, CDCl₃) δ (ppm): 2.01 (m, 1H), 2.28 (s, 3H), 2.35 (m, 3H), 2.43 (s, 3H), 2.40-2.58 (m, 9H), 3.14 (m, 1H), 7.08-7.46 (m, 8H), 7.81 (ddd, J = 1.6, 7.4, 7.4 Hz, 1H).

### Example 50 (Compound 63)

### Step 1

2-Aminoacetophenone hydrochloride (2.93 g, 17.1 mmol) was dissolved in acetonitrile (100 mL). To the solution was added di-tert-butyl dicarbonate (5.09 g, 22.9 mmol) and 4-dimethylaminopyridine (2.21 g, 18.1 mmol), and the mixture was stirred at room temperature for 10 hours. To the reaction mixture was added saturated aqueous ammonium chloride, and the mixture was extracted with ethyl acetate. The organic layer was washed with brine, and then dried over anhydrous sodium sulfate, and the solvent was evaporated under reduced pressure. The residue was purified by silica gel column chromatography (hexane/ethyl acetate = 9/1 → 4/1) to give 2-(tert-butoxycarboxylamino)acetophenone (865 mg, 21%).

### Step 2

2-(tert-Butoxycarboxylamino)acetophenone (851 mg, 3.62 mmol) prepared in Step 1 mentioned above was dissolved in methanol (20 mL). To the solution was added thiosemicarbazide hydrochloride (1.03 g, 8.04 mmol), and the mixture was stirred at room temperature for 15 hours. To the reaction mixture was added water, and the mixture was extracted with ethyl acetate. The organic layer was washed with brine, and then dried over anhydrous sodium sulfate, and the solvent was evaporated under reduced pressure to give 2-(tert-butoxycarboxylamino)acetophenone=thiosemicarbazone.

### Step 3

To 2-(tert-butoxycarboxylamino)acetophenone=thiosemicarbazone (2.91 g, 9.44 mmol) prepared in Step 2 mentioned above was added acetic anhydride (30 mL), and the mixture was stirred at 130°C for 5 minutes, and then at 70°C for 1 hour.

The reaction mixture was left to cool, and then triturated with a mixed solvent of diisopropyl ether and hexane to give 3-acetyl-5-acetylamino-2-[(tert-butoxycarboxylamino)methyl]-2,3-dihydro-2-phenyl-1,3,4-thiadiazole (2.06 g, 56%).
APCI-MS m/z: 393 (M+H)⁺.

### Step 4

3-Acetyl-5-acetylamino-2-[(tert-butoxycarboxylamino)methyl]-2,3-dihydro-2-phenyl-1,3,4-thiadiazole (2.01 g, 5.12 mmol) prepared in Step 3 mentioned above was dissolved in acetonitrile (20 mL). To the solution was added hydrazine monohydrate (8.0 mL, 0.16 mol), and the mixture was stirred at room temperature for 6 hours. To the reaction mixture was added water, and the mixture was extracted with ethyl acetate. The organic layer was washed with brine, then dried over anhydrous sodium sulfate, and concentrated under reduced pressure. The residue was purified by using a 12-system parallel preparative chromatography (Hi-Flash™ column, Yamazen, hexane/ethyl acetate = 2/3) to give 5-amino-3-acetyl-2-[(tert-butoxycarboxylamino)methyl]-2,3-dihydro-2-phenyl-1,3,4-thiadiazole (1.42 g, 79%).
APCI-MS m/z: 351 (M+H)+.

### Step 5

5-Amino-3-acetyl-2-[(tert-butoxycarboxylamino)methyl]-2,3-dihydro-2-phenyl-1,3,4-thiadiazole (4.30 g, 12.3 mmol) prepared in Step 4 mentioned above, potassium iodide (2.45 g, 14.8 mmol) and copper iodide (2.81 g, 14.8 mmol) were suspended in acetonitrile (86 mL), to the suspension was added iodine (3.75 g, 14.8 mmol), and then added tert-butyl nitrite (4.40 mL, 37.0 mmol) under ice cooling, and the mixture was stirred at 50°C for 2 hours. The reaction mixture was cooled to room temperature, then to the mixture was added water, and the mixture was extracted with ethyl acetate. The organic layer was washed with brine, then dried over anhydrous sodium sulfate, and concentrated under reduced pressure. The residue was purified by silica gel column chromatography (ethyl acetate/n-hexane = 1/6) to give 3-acetyl-2-[(tert-butoxycarboxylamino)methyl]-2,3-dihydro-5-iodo-2-phenyl-1,3,4-thiadiazole (4.04 g, yield: 71%).
ESI-MS m/z: 462 [M+H]⁺; ¹H-NMR (CDCl₃) δ (ppm): 1.45 (s, 9H), 2.40 (s, 3H), 4.13 (dd, J = 6.6, 14.9 Hz, 1H), 4.61 (dd, J = 6.6, 14.9 Hz, 1H), 5.26 (m, 1H), 7.27-7.41 (m, 5H). Step 6

3-Acetyl-2-[(tert-butoxycarboxylamino)methyl]-2,3-dihydro-5-iodo-2-phenyl-1,3,4-thiadiazole (1.93 g, 4.18 mmol) prepared in Step 5 mentioned above was dissolved in toluene (57 mL). To the solution was added tributylvinylsilane (1.83 mL, 6.26 mmol) and tetrakis(triphenylphosphine)palladium (242 mg, 0.209 mmol), and the mixture was stirred at 100°C for 3 hours. To the reaction mixture was added 5% aqueous ammonium fluoride, and the mixture was extracted with ethyl acetate. The organic layer was successively washed with water and brine, then dried over anhydrous sodium sulfate, and concentrated under reduced pressure. The residue was purified by silica gel column chromatography (ethyl acetate/n-hexane = 2/3) to give Compound 63 (1.33 g, yield: 88%).

ESI-MS m/z: 362 [M+H]⁺; ¹H-NMR (CDCl₃) δ (ppm): 1.42 (s, 9H), 2.41 (s, 3H), 4.13 (dd, J = 6.2, 14.9 Hz, 1H), 4.62 (dd, J = 6.6, 14.9 Hz, 1H), 5.35 (m, 1H), 5.51 (d, J = 17.4 Hz, 1H), 5.67 (d, J = 10.7 Hz, 1H), 6.58 (dd, J = 10.7, 17.4 Hz, 1H), 7.26-7.39 (m, 5H).

### Example 51 (Compound 64)

Compound 63 (1.10 g, 3.04 mmol) prepared in Example 50 was dissolved in acetone (20 mL). To the solution was added water (3.0 mL), then added potassium permanganate (1.68 g, 10.6 mmol) under ice cooling, and the mixture was stirred at room temperature for 3 hours. To the reaction mixture was added sodium hydrogensulfite (1.03 g), 1 mol/L hydrochloric acid (20 mL) and ethyl acetate (20 mL), and the mixture was stirred at room temperature for 30 minutes, and then extracted with ethyl acetate. The organic layer was washed with brine, then dried over anhydrous sodium sulfate, and concentrated under reduced pressure. The residue was crystallized from a mixed solvent of ethyl acetate and n-hexane to give

### Compound 64 (965 mg, yield: 81%).

ESI-MS m/z: 380 [M+H]⁺; ¹H-NMR (DMSO-d₆) δ (ppm): 1.39 (s, 3H), 2.25 (s, 9H), 4.03 (dd, J = 7.8, 14.4 Hz, 1H), 4.33 (dd, J = 4.7, 14.4 Hz, 1H), 7.30 (m, 1H), 7.32-7.42 (m, 5H).

### Example 52 (Compound 65)

Compound 64 (116 mg, 0.306 mmol) prepared in Example 51, HOBt • H₂O (48.0 mg, 0.313 mmol) and EDCI (66.0 mg, 0.344 mmol) were dissolved in DMF (1.2 mL), and the solution was stirred at room temperature for 10 minutes. Then, to the solution was added tert-butylamine (0.163 mL, 1.55 mmol) at the same temperature, and the mixture was stirred at room temperature for 20 hours. To the reaction mixture was added saturated aqueous sodium hydrogencarbonate and water, and the mixture was extracted with ethyl acetate. The organic layer was successively washed with water, 1 mol/L hydrochloric acid, and brine, then dried over anhydrous sodium sulfate, and concentrated under reduced pressure to give Compound 65 (96.1 mg, yield: 72%).
ESI-MS m/z: 435 [M+H]⁺; ¹H-NMR (CDCl₃) δ (ppm): 1.43 (s, 18H), 2.38 (s, 3H), 3.95 (dd, J = 5.5, 14.7 Hz, 1H), 4.71 (dd, J = 7.6, 14.7 Hz, 1H), 5.31 (m, 1H), 6.38 (br s, 1H), 7.25-7.39 (m, 5H).

### Example 53 (Compound 66)

Compound 65 (96.1 mg, 0.221 mmol) prepared in Example 52 was dissolved in a 4 mol/L solution of hydrogen chloride in ethyl acetate (2.0 mL). The solution was stirred at room temperature for 30 minutes. The reaction mixture was concentrated under reduced pressure, and the residue was crystallized from a mixed solvent of ethyl acetate and n-hexane to give Compound 66 (80.4 mg, yield: 98%).
ESI-MS m/z: 335 [M+H]⁺; ¹H-NMR (DMSO-d₆) δ (ppm): 1.34 (s, 9H), 2.41 (s, 3H), 4.16 (d, J = 13.8 Hz, 1H), 4.25 (d, J = 13.8 Hz, 1H), 7.31-7.46 (m, 5H), 7.81 (br s, 1H), 8.49 (br s, 3H).

### Example 54 (Compound 67)

To a solution of Compound 66 (78.3 mg, 0.211 mmol) prepared in Example 53 in dichloromethane (2.0 mL) was added triethylamine (0.147 mL, 1.05 mmol) and 2-chloroethylsulfonyl chloride (0.033 mL, 0.32 mmol), and the mixture was stirred at room temperature for 30 minutes. To the reaction mixture was added water, and the mixture was extracted with ethyl acetate. The organic layer was successively washed with 1 mol/L hydrochloric acid, and brine, then dried over anhydrous sodium sulfate, and concentrated under reduced pressure. The residue was purified by preparative thin layer chromatography (methanol/ethyl acetate = 1/10) to give Compound 67 (59.3 mg, yield: 66%).
APCI-MS m/z: 425 [M+H]⁺; ¹H-NMR (CDCl₃) δ (ppm): 1.44 (s, 9H), 2.38 (s, 3H), 3.82 (dd, J = 5.3, 13.8 Hz, 1H), 4.58 (dd, J = 8.5, 13.8 Hz, 1H), 5.24 (m, 1H), 5.96 (d, J = 9.9 Hz, 1H), 6.29 (d, J = 16.5 Hz, 1H), 6.40 (br s, 1H), 6.55 (dd, J = 9.9, 16.5 Hz, 1H), 7.25-7.40 (m, 5H).

### Example 55 (Compound 68)

Compound 67 (58.3 mg, 0.137 mmol) prepared in Example 54 was dissolved in acetonitrile (1.2 mL). To the solution was added triethylamine (0.134 mL, 0.961 mmol) and dimethylamine hydrochloride (56.0 mg, 0.687 mmol), and the mixture was stirred at room temperature for 1 hour. To the reaction mixture was added water, and the mixture was extracted with ethyl acetate. The organic layer was washed with brine, then dried over anhydrous sodium sulfate, and concentrated under reduced pressure. The residue was crystallized from a mixed solvent of ethyl acetate, n-hexane and methanol to give Compound 68 (42.5 mg, yield: 66%).
ESI-MS m/z: 470 [M+H]⁺; ¹H-NMR (CDCl₃) δ (ppm): 1.43 (s, 9H), 2.27 (s, 6H), 2.38 (s, 3H), 2.77-2.84 (m, 2H), 3.14-3.20 (m, 2H), 3.93 (d, J = 13.8 Hz, 1H), 4.69 (d, J = 13.8 Hz, 1H), 6.13 (m, 1H), 6.39 (br s, 1H), 7.27-7.42 (m, 5H).

### Example 56 (Compound 69)

Compound 64 (100 mg, 0.264 mmol) prepared in Example 51 was dissolved in N,N-dimethylacetamide (1.0 mL). To the solution was added thionyl chloride (0.039 mL, 0.54 mmol) under ice cooling, and the mixture was stirred for 30 minutes. Then, to the mixture was added diisopropylamine (0.370 mL, 2.64 mmol) at the same temperature, and the mixture was stirred at room temperature for 20 hours. To the reaction mixture was added water, and the mixture was extracted with ethyl acetate. The organic layer was successively washed with 1 mol/L hydrochloric acid, water, and brine, then dried over anhydrous sodium sulfate, and concentrated under reduced pressure. The residue was purified by preparative thin layer chromatography (ethyl acetate/n-hexane = 1/1) to give Compound 69 (122 mg, yield: 100%).
ESI-MS m/z: 463 [M+H]⁺; ¹H-NMR (CDCl₃) δ (ppm): 1.16-1.32 (m, 6H), 1.35-1.56 (m, 6H), 1.42 (s, 9H), 2.35 (s, 3H), 3.55 (m, 1H), 3.97 (dd, J = 5.4, 14.8 Hz, 1H), 4.54 (m, 1H), 4.72 (dd, J = 7.7, 14.8 Hz, 1H), 5.43 (m, 1H), 7.25-7.42 (m, 5H).

### Example 57 (Compound 70)

In accordance with the methods described in Example 53 and Example 54, Compound 69 (122 mg, 0.264 mmol) prepared in Example 56 was treated with a 4 mol/L solution of hydrogen chloride in ethyl acetate (1.0 mL), followed by reacting with 2-chloroethylsulfonyl chloride (0.055 mL, 0.53 mmol) in the presence of triethylamine (0.184 mL, 1.32 mmol) to give Compound 70 (75.1 mg, yield: 62%). ESI-MS m/z: 453 [M+H]⁺; ¹H-NMR (CDCl₃) δ (ppm): 1.22-1.35 (m, 6H), 1.40-1.52 (m, 6H), 2.37 (s, 3H), 3.58 (m, 1H), 3.84 (dd, J = 5.3, 14.2 Hz, 1H), 4.57 (dd, J = 8.3, 14.2 Hz, 1H), 4.69 (m, 1H), 5.19 (dd, J = 5.3, 8.3 Hz, 1H), 5.97 (d, J = 9.7 Hz, 1H), 6.29 (d, J = 16.5 Hz, 1H), 6.56 (dd, J = 9.7, 16.5 Hz, 1H), 7.26-7.42 (m, 5H).

### Example 58 (Compound 71)

In accordance with the method described in Example 55, Compound 70 (72.0 mg, 0.159 mmol) prepared in Example 57 was reacted with dimethylamine hydrochloride (65.0 mg, 0.797 mmol) in the presence of triethylamine (0.155 mL, 1.11 mmol). Then the obtained product was dissolved in ethyl acetate (3.0 mL). To the solution was added a 4 mol/L solution of hydrogen chloride in ethyl acetate (0.30 mL), and the mixture was stirred at room temperature for 30 minutes. To the reaction mixture was added n-hexane, and the resulting crystals were collected by filtration to give Compound 71 (66.0 mg, yield: 72%).
ESI-MS m/z: 498 [M+H]⁺; ¹H-NMR (DMSO-d₆) δ (ppm): 1.21 (d, J = 6.6 Hz, 6H), 1.34 (d, J = 6.6 Hz, 6H), 2.22 (s, 3H), 2.80 (s, 6H), 3.35-3.50 (m, 3H), 3.58-3.70 (m, 3H), 3.92 (dd, J = 7.6, 14.2 Hz, 1H), 4.41 (dd, J = 5.6, 14.2 Hz, 1H), 4.64 (m, 1H), 7.27-7.47 (m, 5H), 8.09 (br s, 1H), 10.18 (br s, 1H).

### Example 59 (Compound 72)

Compound 64 (100 mg, 0.264 mmol) prepared in Example 51 was dissolved in DMF (1.0 mL). To the solution was added methyl iodide (0.040 mL, 0.64 mmol) and potassium carbonate (40.0 mg, 0.289 mmol) at room temperature, and the mixture was stirred for 2 hours. To the reaction mixture was added water, and the mixture was extracted with ethyl acetate. The organic layer was washed with brine, then dried over anhydrous sodium sulfate, and concentrated under reduced pressure. The residue was dissolved in methanol (3.0 mL). To the solution was added concentrated hydrochloric acid (0.30 mL), and the mixture was stirred at 50°C for 1 hour. The reaction mixture was concentrated under reduced pressure, and then the residue was reacted with 2-chloroethylsulfonyl chloride (0.110 mL, 1.05 mmol) in the presence of triethylamine (0.368 mL, 2.64 mmol) in accordance with the method described in Example 54 to give Compound 72 (46.6 mg, yield: 46%).
ESI-MS m/z: 384 [M+H]⁺; ¹H-NMR (CDCl₃) δ (ppm): 2.42 (s, 3H), 3.89 (dd, J = 6.0, 14.1 Hz, 1H), 4.57 (dd, J = 7.8, 14.1 Hz, 1H), 5.37 (dd, J = 6.0, 7.8 Hz, 1H), 5.95 (d, J = 9.8 Hz, 1H), 6.27 (d, J = 16.5 Hz, 1H), 6.55 (dd, J = 9.8, 16.5 Hz, 1H), 7.25-7.42 (m, 5H).

### Example 60 (Compound 73)

In accordance with the method described in Example 55, Compound 72 (46.0 mg, 0.120 mmol) prepared in Example 59 was reacted with dimethylamine hydrochloride (48.9 mg, 0.600 mmol) in the presence of triethylamine (0.117 mL, 0.839 mmol) to give Compound 73 (29 mg, yield: 56%).
APCI-MS m/z: 442 [M+H]⁺; ¹H-NMR (CD₃OD) δ (ppm): 2.22 (s, 3H), 2.80 (s, 6H), 3.35-3.50 (m, 2H), 3.58-3.70 (m, 2H), 3.92 (dd, J = 7.6, 14.2 Hz, 1H), 4.41 (dd, J = 5.6, 14.2 Hz, 1H), 4.64 (m, 1H), 7.27-7.47 (m, 5H), 8.09 (br s, 1H), 10.18 (br s, 1H).

### Example 61 (Compound 74)

### Step 1

2-Aminoacetophenone hydrochloride (2.93 g, 17.1 mmol) was dissolved in acetonitrile (100 mL). To this solution was successively added di-tert-butyl dicarbonate (5.09 g, 22.9 mmol) and 4-dimethylaminopyridine (2.21 g, 18.1 mmol), and the mixture was stirred at room temperature for 10 hours. To the reaction mixture was added saturated aqueous ammonium chloride, and the mixture was extracted with ethyl acetate. The organic layer was washed with brine, and dried over anhydrous sodium sulfate, and the solvent was evaporated under reduced pressure. The residue was purified by silica gel column chromatography (hexane/ethyl acetate = 9/1 → 4/1) to give 2-(N-tert-butoxycarboxylamino)acetophenone (865 mg, 21%).

### Step 2

2-(N-tert-Butoxycarboxylamino)acetophenone (851 mg, 3.62 mmol) prepared in Step 1 mentioned above was dissolved in methanol (20 mL). To the solution was added thiosemicarbazide hydrochloride (1.03 g, 8.04 mmol), and the mixture was stirred at room temperature for 15 hours. To the reaction mixture was added water, and the mixture was extracted with ethyl acetate. The organic layer was washed with brine, and dried over anhydrous sodium sulfate, and the solvent was evaporated under reduced pressure. The residue was dissolved in dichloromethane (50 mL). To the solution was added pyridine (1.75 mL, 21.7 mmol) and trimethylacetyl chloride (2.23 mL, 18.1 mmol), and the mixture was stirred at room temperature for 16 hours. To the reaction mixture was added saturated aqueous sodium hydrogencarbonate, and the mixture was further stirred at room temperature for 1 hour, and then extracted with ethyl acetate. The organic layer was washed with brine, and dried over anhydrous sodium sulfate, and the solvent was evaporated under reduced pressure. The residue was purified by silica gel column chromatography (hexane/ethyl acetate = 9/1 → 4/1) to give 2-[(tert-butoxycarboxylamino)methyl]-2,3-dihydro-3-(2,2-dimethylpropionyl)-5-[(2,2-dimethylpropionyl)amino]-2-phenyl-1,3,4-thiadiazole (910 mg, 53%).
APCI-MS m/z 477 [M+H]⁺.

### Step 3

2-[(tert-Butoxycarboxylamino)methyl]-2,3-dihydro-3-(2,2-dimethylpropionyl)-5-[(2,2-dimethylpropionyl)amino]-2-phenyl-1,3,4-thiadiazole (3.72 g, 9.48 mmol) prepared in Step 2 mentioned above was dissolved in a mixed solvent of tert-butanol (150 mL) and an aqueous solution of hydrochloric acid/sodium acetate (pH = 3, 50 mL).

To the solution was added sodium borohydride (3.6 g, 94.8 mmol) at room temperature, and the mixture was stirred at 50°C for 1 hour. To the reaction mixture was added acetic acid (5.4 mL), and the mixture was stirred at room temperature for 30 minutes. Then, to the reaction mixture was added water, and the mixture was extracted with ethyl acetate. The organic layer was washed with brine, and dried over anhydrous sodium sulfate, and the solvent was evaporated under reduced pressure. The residue was reslurried in hexane to give 5-amino-2-[(tert-butoxycarboxylamino)methyl]-2,3-dihydro-3-(2,2-dimethylpropionyl)-2-phenyl-1,3,4-thiadiazole (3.10g, 99%).
APCI-MS m/z: 393 [M+H]⁺.

### Step 4

In accordance with the method described in Step 1 of Example 50, 2-[(tert-butoxycarboxylamino)methyl]-2,3-dihydro-3-(2,2-dimethylpropionyl)-5-iodo-2-phenyl-1,3,4-thiadiazole (986 mg, yield: 77%) was obtained from 5-amino-2-[(tert-butoxycarboxylamino)methyl]-2,3-dihydro-3-(2,2-dimethylpropionyl)-2-phenyl-1,3,4-thiadiazole (1.00 g, 2.55 mmol) prepared in Step 3 mentioned above, potassium iodide (580 mg, 3.04 mmol), copper iodide (510 mg, 3.07 mmol), iodine (780 mg, 3.07 mmol), and tert-butyl nitrite (0.980 mL, 8.24 mmol).

### Step 5

2-[(tert-Butoxycarboxylamino)methyl]-2,3-dihydro-3-(2,2-dimethylpropionyl)-5-iodo-2-phenyl-1,3,4-thiadiazole (500 mg, 0.993 mmol) prepared in Step 4 mentioned above was dissolved in THF (10 mL). To the solution was added triethoxyvinylsilane (0.420 mL, 1.99 mmol), bis(dibenzylideneacetone)palladium (86 mg, 0.15 mmol) and tetrabutylammonium fluoride (1.0 mol/L solution in THF, 2.00 mL, 2.00 mmol), and the mixture was stirred at 50°C for 6 hours. The reaction mixture was cooled to room temperature, then to the mixture was added water, and the mixture was extracted with ethyl acetate. The organic layer was washed with brine, then dried over anhydrous sodium sulfate, and concentrated under reduced pressure. The residue was purified by silica gel column chromatography (ethyl acetate/n-hexane = 1/9) to give Compound 74 (343 mg, yield: 85%).

ESI-MS m/z: 404 [M+H]⁺; ¹H-NMR (CDCl₃) δ (ppm): 1.36 (s, 9H), 1.41 (s, 9H), 4.12 (dd, J = 5.9, 14.8 Hz, 1H), 4.64 (dd, J = 6.6, 14.8 Hz, 1H), 5.23 (m, 1H), 5.49 (d, J = 17.4 Hz, 1H), 5.66 (d, J = 10.7 Hz, 1H), 6.62 (dd, J = 10.7, 17.4 Hz, 1H), 7.22-7.38 (m, 5H).

### Example 62 (Compound 75)

Compound 74 (314 mg, 0.778 mmol) prepared in Example 61 was used and treated in accordance with the methods described in Example 51 to Example 54, and then the obtained product was treated in accordance with the method described in Example 58 to give Compound 75 (yield: 32% (for 5 steps)). APCI-MS m/z: 512 [M+H]⁺; ¹H-NMR (DMSO-d₆) δ (ppm): 1.25 (s, 9H), 1.36 (s, 9H), 2.79 (s, 6H), 3.35-3.53 (m, 2H), 3.59-3.69 (m, 2H), 4.05 (dd, J = 5.6, 14.0 Hz, 1H), 4.44 (dd, J = 8.2, 14.0 Hz, 1H), 7.04 (br s, 1H), 7.26-7.41 (m, 5H), 7.86 (dd, J = 5.6, 8.2 Hz, 1H), 10.38 (br s, 1H).

The structures of the Compounds A to E obtained in Reference Examples 1 to 5 are shown in Table 4 below.

**Table 4**

| 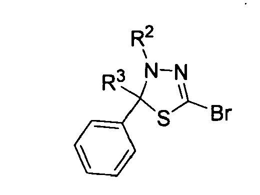 | | | |
|---|---|---|---|
| Ref. Ex. No. | Compound No. | R² | R³ |
| 1 | A | -COCH₃ | -CH₃ |
| 2 | B | -COCH₃ | -CH₂NHSO₂CH₃ |
| 3 | C | -COC(CH₃)₃ | -CH₂NHSO₂CH₃ |
| 4 | D | -COCH₃ | -CH₂CH₂NHCO₂C(CH₃)₃ |
| 5 | E | -COCH₃ | -CH₂CH₂CO₂CH₃ |

### Reference Example 1 (Compound A)

### Step 1

Thiosemicarbazide (12.0 g, 132 mmol) was suspended in methanol (200 mL), to the suspension was added acetophenone (15.4 mL, 132 mmol) and concentrated hydrochloric acid (1.2 mL), and the mixture was stirred under reflux for 5.7 hours. Then, the solvent was evaporated under reduced pressure, to the residue was added a mixed solution of hexane and diethyl ether (1/1, 150 mL), and the deposited solid was reslurried. The resulting white solid was collected by filtration, washed with hexane/diethyl ether, and then dried under reduced pressure to give acetophenone=thiosemicarbazone (25.4 g, yield: 96%).
¹H-NMR (DMSO-d₆) δ (ppm): 2.30 (s, 3H), 7.37-7.40 (m, 3H), 7.91-7.94 (m, 3H), 8.27 (br s, 1H), 10.21 (br s, 1H).

### Step 2

Acetophenone=thiosemicarbazone (8.44 g, 43.7 mmol) prepared in Step 1 mentioned above was suspended in acetone (170 mL), to the suspension was added pyridine (7.1 mL, 87 mmol) and acetic anhydride (8.3 mL, 87 mmol), and the mixture was stirred at room temperature for 25 hours. To the reaction mixture was added 2 mol/L aqueous sodium hydroxide, and the mixture was extracted with ethyl acetate. The organic layer was washed with brine, and then dried over anhydrous sodium sulfate. The solvent was evaporated under reduced pressure, and the residue was purified by silica gel column chromatography (hexane/ethyl acetate = 1/1 → 1/2) to give 1-(5-amino-2-methyl-2-phenyl-2,3-dihydro[1,3,4]thiadiazol-3-yl)-ethanone (7.67 g, yield: 75%).
¹H-NMR (DMSO-d₆) δ (ppm): 2.12 (s, 3H), 2.31 (s, 3H), 6.49 (br s, 2H), 7.21-7.41 (m, 5H).

### Step 3

Copper bromide (17.1 g, 76.5 mmol) was dissolved in acetonitrile (225 mL). To the solution was added tert-butyl nitrite (12.1 mL, 102 mmol) at 0°C. After stirring for 10 minutes, to the reaction mixture was added 1-(5-amino-2-methyl-2-phenyl-2,3-dihydro[1,3,4]thiadiazol-3-yl)-ethanone (15.0 g, 63.8 mmol) prepared in Step 2 mentioned above, and the mixture was stirred at 0°C to room temperature for 4.8 hours. To the reaction mixture was added water, and the mixture was extracted with ethyl acetate. The organic layer was washed with brine, and then dried over anhydrous sodium sulfate. The solvent was evaporated under reduced pressure, and the residue was purified by silica gel column chromatography (hexane/ethyl acetate = 8/1 → 6/1) to give Compound A (15.4 g, yield: 81%).
FAB-MS m/z: 299 [M+H]⁺; ¹H-NMR (CDCl₃) δ (ppm): 2.29 (s, 3H), 2.44 (s, 3H), 7.32 (m, 3H), 7.46 (m, 2H).

### Reference Example 2 (Compound B)

### Step 1

In accordance with the method described in Step 1 of Reference Example 1, 2-(methylsulfonylamino)acetophenone=thiosemicarbazone (14.5 g, yield: 77%) was obtained from 2-(methylsulfonylamino)acetophenone (14.1 g, 66.0 mmol), and thiosemicarbazide (6.00 g, 66.0 mmol).

### Step 2

In accordance with the method described in Step 2 of Reference Example 1, N-(4-acetyl-2-amino-5-phenyl-4,5-dihydro[1,3,4]thiadiazol-5-ylmethyl)methanesulfonamide (302 mg, yield: 26%) was obtained from 2-(methylsulfonylamino)acetophenone=thiosemicarbazone (1.00 g, 3.49 mmol) prepared in Step 1 mentioned above.

APCI-MS m/z: 329 [M+H]⁺; ¹H-NMR (CDCl₃) δ (ppm): 2.29 (s, 3H), 2.99 (s, 3H), 4.04 (d, J = 14.0 Hz, 1H), 4.55 (d, J = 14.0 Hz, 1H), 7.30-7.41 (m, 5H).

### Step 3

In accordance with the method described in Step 3 of Reference Example 1, Compound B (176 mg, yield: 61%) was obtained from N-(4-acetyl-2-amino-5-phenyl-4,5-dihydro[1,3,4]thiadiazol-5-ylmethyl)methanesulfonamide (240 mg, 0.732 mmol) prepared in Step 2 mentioned above.
FAB-MS m/z: 392 [M+H]⁺; ¹H-NMR (CDCl₃) δ (ppm): 2.38 (s, 3H), 3.01 (s, 3H), 4.07 (dd, J = 6.3, 14.2 Hz, 1H), 4.65 (dd, J = 7.4, 14.3 Hz, 1H), 5.15 (t, J = 6.9 Hz, 1H), 7.26-7.44 (m, 5H).

### Reference Example 3 (Compound C)

### Step 1

2-(Methylsulfonylamino)acetophenone=thiosemicarbazone (300 mg, 1.05 mmol) prepared in Step 1 of Reference Example 2 was dissolved in THF (18 mL). To the solution was added dropwise 4-dimethylaminopyridine (641 mg, 5.25 mmol) and pivaloyl chloride (0.13 mL, 1.1 mmol) at room temperature with stirring. One and two hours after completion of the addition of pivaloyl chloride, to the mixture was added pivaloyl chloride (0.065 mL, 0.53 mmol), and the mixture was further stirred for 1.6 hours. To the reaction mixture was added water, and the mixture was extracted with ethyl acetate. The organic layer was washed with brine, and then dried over anhydrous sodium sulfate. The solvent was evaporated under reduced pressure, and the residue was purified by preparative thin layer chromatography (chloroform/methanol = 20/1) to give N-[5-amino-3-(2,2-dimethylpropionyl)-2-phenyl-2,3-dihydro[1,3,4]thiadiazol-2-ylmethyl]methanesulfonamide (88 mg, yield: 22%).
APCI-MS m/z: 371 [M+H]⁺; ¹H-NMR (CDCl₃) δ (ppm): 1.34 (s, 9H), 2.96 (s, 3H), 4.06 (dd, J = 6.2, 13.7 Hz, 1H), 4.19 (br s, 2H), 4.58 (dd, J = 7.0, 13.7 Hz, 1H), 5.20 (t, J = 6.4 Hz, 1H), 7.27-7.55 (m, 5H).

### Step 2

In accordance with the method described in Step 3 of Reference Example 1, Compound C (164 mg, yield: 78%) was obtained from N-[5-amino-3-(2,2-dimethylpropionyl)-2-phenyl-2,3-dihydro[1,3,4]thiadiazol-2-ylmethyl]methanesulfonamide (180 mg, 0.486 mmol) prepared in Step 1 mentioned above.

APCI-MS m/z: 434 [M+H]⁺; ¹H-NMR (CDCl₃ + CD₃OD) δ (ppm): 1.32 (s, 9H), 3.03 (s, 3H), 4.08 (d, J = 14.0 Hz, 1H), 4.63 (d, J = 14.0 Hz, 1H), 7.32-7.42 (m, 5H).

### Reference Example 4 (Compound D)

### Step 1

N-tert-Butoxycarboxyl-β-alanine (10.0 g, 52.9 mmol) was dissolved in THF (150 mL). To the solution was added phenylboronic acid (7.73 g, 63.4 mmol), pivalic anhydride (16.1 mL, 79.3 mmol), palladium acetate (593 mg, 2.64 mmol), triphenylphosphine (1.39 g, 5.29 mmol) and water (2.28 mL), and the mixture was stirred at 60°C for 22.5 hours under an argon atmosphere. The reaction mixture was filtered through Celite, to the filtrate was added saturated aqueous sodium hydrogencarbonate, and the mixture was extracted with ethyl acetate. The organic layer was washed with brine, and then dried over anhydrous sodium sulfate. The solvent was evaporated under reduced pressure, and the residue was purified by silica gel column chromatography (hexane/ethyl acetate = 6/1 → 2/1) to give (3-oxo-3-phenylpropyl)carbamic acid tert-butyl ester (7.85 g, yield: 60%).
APCI-MS m/z: 250 [M+H]⁺.

### Step 2

(3-Oxo-3-phenylpropyl)carbamic acid tert-butyl ester (7.80 g, 31.3 mmol) prepared in Step 1 mentioned above was dissolved in a mixed solvent of methanol (240 mL) and water (60 mL). To the solution was added thiosemicarbazide hydrochloride (7.98 g, 62.6 mmol), and the mixture was stirred at room temperature for 4 hours. To the reaction mixture was added water, and the mixture was extracted with ethyl acetate. The organic layer was washed with brine, and then dried over anhydrous sodium sulfate. The solvent was evaporated under reduced pressure, and to the residue was added a hexane/chloroform solution (10/1, 200 mL) to reslurry the solid product. The white solid was collected by filtration, washed with hexane, and then dried under reduced pressure to give (3-oxo-3-phenylpropyl)carbamic acid tert-butyl ester=thiosemicarbazone (9.23 g, yield: 91%). APCI-MS m/z: 323 [M+H]⁺.

### Step 3

(3-Oxo-3-phenylpropyl)carbamic acid tert-butyl ester=thiosemicarbazone (4.07 g, 12.6 mmol) prepared in Step 2 mentioned above was dissolved in acetone (100 mL). To the solution was added pyridine (5.4 mL, 63.1 mmol) and acetic anhydride (6.0 mL, 63.1 mmol), and the mixture was stirred at room temperature for 6 hours. Then, to the reaction mixture was added saturated aqueous sodium hydrogencarbonate (50 mL), and the mixture was stirred at room temperature for 30 minutes. The reaction mixture was extracted with ethyl acetate. The organic layer was washed with brine, and then dried over anhydrous sodium sulfate. The solvent was evaporated under reduced pressure, to the residue was added methanol (20 mL) and hydrazine monohydrate (20 mL), and the mixture was stirred at room temperature for 2 hours. To the reaction mixture was added water, and the mixture was extracted with ethyl acetate. The organic layer was washed with brine, and then dried over anhydrous sodium sulfate. The solvent was evaporated under reduced pressure, and to the residue was added a hexane-diethyl ether (10/1) solution (100 mL) to reslurry the solid product. The white solid was collected by filtration, washed with hexane, and then dried under reduced pressure to give [2-(3-acetyl-5-amino-2-phenyl-2,3-dihydro[1,3,4]thiadiazol-2-yl)ethyl]carbamic acid tert-butyl ester (4.38 g, yield: 91%).
APCI-MS m/z: 365 [M+H]⁺.

### Step 4

In accordance with the method described in Step 3 of Reference Example 1, Compound D (2.66 g, yield: 81%) was obtained from [2-(3-acetyl-5-amino-2-phenyl-2,3-dihydro[1,3,4]thiadiazol-2-yl)ethyl]carbamic acid tert-butyl ester (2.80 g, 7.67 mmol) prepared in Step 3 mentioned above.
APCI-MS m/z: 429 [M+H]⁺.

### Reference Example 5 (Compound E)

### Step 1

In accordance with the method described in Step 2 of Reference Example 4, 3-benzoylpropionic acid methyl ester=thiosemicarbazone (5.79 g, yield: 73%) was obtained from 3-benzoylpropionic acid methyl ester (5.70 g, 29.7 mmol) and thiosemicarbazide hydrochloride (5.70 g, 44.5 mmol).
APCI-MS m/z: 266 [M+H]⁺.

### Step 2

In accordance with the method described in Step 2 of Reference Example 1, 3-(3-acetyl-5-amino-2-phenyl-2,3-dihydro[1,3,4]thiadiazol-2-yl)propionic acid methyl ester (4.52 g, yield: 78%) was obtained from 3-benzoylpropionic acid methyl ester=thiosemicarbazone (5.00 g, 18.8 mmol) prepared in Step 1 mentioned above.

APCI-MS m/z: 307 [M+H]⁺.

### Step 3

In accordance with the method described in Step 3 of Reference Example 1, Compound E (4.39 g, yield: 80%) was obtained from 3-(3-acetyl-5-amino-2-phenyl-2,3-dihydro[1,3,4]thiadiazol-2-yl)propionic acid methyl ester (4.52 g, 14.7 mmol) prepared in Step 2 mentioned above.
APCI-MS m/z: 372 [M+H]⁺.

### Reference Example 6 (Compound 22)

Compound A (2.50 g, 8.36 mmol) was dissolved in toluene (30 mL). To the solution was added 2-fluorophenylboronic acid (2.34 g, 16.7 mmol), tetrakis(triphenylphosphine)palladium (773 mg, 0.669 mmol) and a 4 mol/L cesium fluoride aqueous solution (4.18 mL), and the mixture was stirred at 100°C for 4.8 hours under an argon atmosphere. To the reaction mixture was added water, and the mixture was extracted with ethyl acetate. The organic layer was washed with brine, and then dried over anhydrous sodium sulfate. The solvent was evaporated under reduced pressure, and the residue was purified by silica gel column chromatography (hexane/ethyl acetate = 7/1 → 6/1, and then chloroform/methanol = 500/1 → 300/1) to give Compound 22 (2.42 g, yield: 92%).
APCI-MS m/z: 315 [M+H]⁺; ¹H-NMR (CDCl₃) δ (ppm): 2.43 (s, 3H), 2.47 (s, 3H), 7.09-7.45 (m, 6H), 7.49 (m, 2H), 7.84 (ddd, J = 1.8, 7.5, 7.7 Hz, 1H).

### Reference Example 7 (Compound 23)

In accordance with the method described in Reference Example 6, Compound 23 (11 mg, yield: 15%) was obtained from Compound A (70 mg, 0.23 mmol), and phenylboronic acid (57 mg, 0.47 mmol).
FAB-MS m/z: 297 [M+H]⁺; ¹H-NMR (CDCl₃) δ (ppm): 2.44 (s, 3H), 2.48 (s, 3H), 7.26-7.52 (m, 8H), 7.60 (m, 2H).

### Reference Example 8 (Compound 24)

In accordance with the method described in Reference Example 6, Compound 24 (1.76 g, yield: 84%) was obtained from Compound A (2.00 g, 6.68 mmol), and 3-fluorophenylboronic acid (1.87 g, 13.4 mmol).

APCI-MS m/z: 313 [M-H]-; ¹H-NMR (CDCl₃) δ (ppm): 2.43 (s, 3H), 2.48 (s, 3H), 7.14 (m, 1H), 7.28-7.51 (m, 8H).

### Reference Example 9 (Compound 25)

In accordance with the method described in Reference Example 6, Compound 25 (1.92 g, yield: 87%) was obtained from Compound A (2.00 g, 6.68 mmol), and 3-chlorophenylboronic acid (2.09 g, 13.4 mmol).
APCI-MS m/z: 329 [M-H]; ¹H-NMR (CDCl₃) δ (ppm): 2.43 (s, 3H), 2.48 (s, 3H), 7.28-7.50 (m, 8H), 7.69 (dd, J = 1.5, 1.6 Hz, 1H).

### Reference Example 10 (Compound 26)

In accordance with the method described in Reference Example 6, Compound 26 (4.9 mg, yield: 20%) was obtained from Compound A (20 mg, 0.067 mmol), and 3-bromophenylboronic acid (26.8 mg, 0.134 mmol).
APCI-MS m/z: 373 [M-H]-; ¹H-NMR (CDCl₃) δ (ppm): 2.43 (s, 3H), 2.48 (s, 3H), 7.27-7.46 (m, 4H), 7.49-7.58 (m, 4H), 7.84 (dd, J = 1.6, 1.6 Hz, 1H).

### Reference Example 11 (Compound 27)

In accordance with the method described in Reference Example 6, Compound 27 (1.73 g, yield: 84%) was obtained from Compound A (2.00 g, 6.68 mmol), and 3-methylphenylboronic acid (1.82 g, 13.4 mmol).
APCI-MS m/z: 311 [M+H]⁺; ¹H-NMR (CDCl₃) δ (ppm): 2.39 (s, 3H), 2.44 (s, 3H), 2.47 (s, 3H), 7.24-7.38 (m, 6H), 7.48 (m, 3H).

### Reference Example 12 (Compound 28)

In accordance with the method described in Reference Example 6, Compound 28 (5.5 mg, yield: 26%) was obtained from Compound A (20 mg, 0.067 mmol), and 3-cyanophenylboronic acid (19.6 mg, 0.134 mmol).
APCI-MS m/z: 320 [M-H]⁻; ¹H-NMR (CDCl₃) δ (ppm): 2.44 (s, 3H), 2.49 (s, 3H), 7.29-7.39 (m, 3H), 7.48 (m, 2H), 7.55 (d, J = 7.7 Hz, 1H), 7.71 (d, J = 7.7 Hz, 1H), 7.82 (d, J = 7.9 Hz, 1H), 7.98 (br s, 1H).

### Reference Example 13 (Compound 29)

In accordance with the method described in Reference Example 6, Compound 29 (8.4 mg, yield: 38%) was obtained from Compound A (20 mg, 0.067 mmol), and 3,4-difluorophenylboronic acid (21.1 mg, 0.134 mmol).
APCI-MS m/z: 331 [M-H] ; ¹H-NMR (CDCl₃) δ (ppm): 2.42 (s, 3H), 2.48 (s, 3H), 7.19 (m, 1H), 7.28-7.39 (m, 4H), 7.48 (m, 2H), 7.56 (m, 1H).

### Reference Example 14 (Compound 30)

In accordance with the method described in Reference Example 6, Compound 30 (9.9 mg, yield: 45%) was obtained from Compound A (20 mg, 0.067 mmol), and 3,5-difluorophenylboronic acid (21.1 mg, 0.134 mmol).
APCI-MS m/z: 331 [M-H]⁻; ¹H-NMR (CDCl₃) δ (ppm): 2.42 (s, 3H), 2.48 (s, 3H), 6.88 (m, 1H), 7.18, (m, 2H), 7.28-7.39 (m, 3H), 7.47 (m, 2H).

### Reference Example 15 (Compound 31)

In accordance with the method described in Reference Example 6, Compound 31 (9.1 mg, yield: 41%) was obtained from Compound A (20 mg, 0.067 mmol), and 2,4-difluorophenylboronic acid (21.1 mg, 0.134 mmol).
APCI-MS m/z: 331 [M-H]-; ¹H-NMR (CDCls) δ (ppm): 2.41 (s, 3H), 2.46 (s, 3H), 6.86 (m, 1H), 6.95 (m, 1H), 7.26-7.39 (m, 3H), 7.48 (m, 2H), 7.83 (m, 1H).

### Reference Example 16 (Compound 32)

In accordance with the method described in Reference Example 6, Compound 32 (1.21 g, yield: 72%) was obtained from Compound E (2.88 g, 7.76 mmol), and 2-fluorophenylboronic acid (2.17 g, 15.5 mmol).
APCI-MS m/z: 387 [M+H]⁺; ¹H-NMR (270 MHz, CDCl₃) δ (ppm): 2.33 (s, 3H), 2.47 (m, 1H), 2.76 (m, 2H), 3.47 (m, 1H), 3.72 (s, 3H), 7.08-7.46 (m, 8H), 7.81 (ddd, J = 1.6, 7.4, 7.4 Hz, 1H).

### Reference Example 17 (Compound 33)

Compound 32 (1.02 g, 2.64 mmol) prepared in Reference Example 16 was dissolved in THF (30 mL). To the solution was added lithium aluminum hydride (50.0 mg, 1.32 mmol) under ice cooling, and the mixture was stirred at room temperature for 1 hour. Then, to the reaction mixture was added sodium sulfate decahydrate (0.5 g), and the mixture was stirred at room temperature for 3 hours. The reaction mixture was filtered, and then the solvent was evaporated under reduced pressure from the filtrate. The residue was purified by column chromatography (hexane/ethyl acetate = 1/1) to give Compound 33 (238 mg, yield: 25%).
APCI-MS m/z: 359 [M+H]⁺.

### Reference Example 18 (Compound 34)

Compound 33 (238 mg, 0.664 mmol) prepared in Reference Example 17 was dissolved in dichloromethane (10 mL). To the solution was added Dess-Martin periodic acid (0.422 g, 0.996 mmol), and the mixture was stirred at room temperature for 45 minutes. Then, to the reaction mixture was added aqueous sodium thiosulfate, and the mixture was extracted with chloroform. The organic layer was washed with brine, and then dried over anhydrous sodium sulfate, and the solvent was evaporated under reduced pressure. The residue was purified by preparative thin layer chromatography (hexane/ethyl acetate = 2/1) to give Compound 34 (120 mg, yield: 51%).

### Example 63 (Formulation Example 1)

### Tablets (Compound 1)

Tablets having the following composition are prepared in a conventional manner. Compound 1 (40 g), lactose (286.8 g) and potato starch (60 g) are mixed, and 10% aqueous solution of hydroxypropylcellulose (120 g) is added to the mixture. This mixture is kneaded, granulated and dried in a conventional manner, and then the granules are sized to obtain granules for tablet pressing. Magnesium stearate (1.2 g) is added to the granules for tablet pressing and mixed. Tableting is performed with a tableting machine having a pestle of 8 mm a diameter (Kikusui, RT-15) to obtain tablets (containing 20 mg/tablet of active ingredient).

### Formulation

| | | |
|---|---|---|
| Compound 1 | 20 | mg |
| Lactose | 143.4 | mg |
| Potato starch | 30 | mg |
| Hydroxypropylcellulose | 6 | mg |
| Magnesium stearate | 0.6 | mg |
| | 200 | mg |

### Example 64 (Formulation Example 2)

### Tablets (Compound 22)

The tablets (containing 20 mg/tablet of active ingredient) are obtained by using Compound 22 (40 g) in the same manner as that in Example 22.

### Formulation

| | | |
|---|---|---|
| Compound 22 | 20 | mg |
| Lactose | 143.4 | mg |
| Potato starch | 30 | mg |
| Hydroxypropylcellulose | 6 | mg |
| Magnesium stearate | 0.6 | mg |
| | 200 | mg |

### Example 65 (Formulation Example 3)

### Injection (Compound 24)

Injection having the following composition is prepared in a conventional manner. Compound 24 (1 g) and D-mannitol (5 g) are added to distilled water for injection and mixed, and hydrochloric acid and aqueous sodium hydroxide are added to the mixture to adjust the mixture to pH 6, and then the total volume is made 1000 mL with distilled water for injection. The resulting mixture is aseptically filled in glass vials in a volume of 2 mL each to obtain injection (containing 2 mg/vial of the active ingredient).

### Formulation

| | | |
|---|---|---|
| Compound 24 | 2 | mg |
| D-Mannitol | 10 | mg |
| Hydrochloric acid | Optimum | amount |
| Aqueous sodium hydroxide | Optimum | amount |
| Distilled water for injection | Optimum | amount |
| | 2.00 | mL |

### Example 66 (Formulation Example 4)

### Tablets (Compound 35)

Tablets having the following composition are prepared in a conventional manner. Compound 35 (40 g), lactose (286.8 g) and potato starch (60 g) are mixed, and 10% aqueous solution of hydroxypropylcellulose (120 g) is added to the mixture. This mixture is kneaded, granulated and dried in a conventional manner, and then the granules are sized to obtain granules for tablet pressing. Magnesium stearate (1.2 g) is added to the granules for tablet pressing and mixed. Tableting is performed with a tableting machine having a pestle of 8 mm a diameter (Kikusui, RT-15) to obtain tablets (containing 20 mg/tablet of active ingredient).

### Formulation

| | | |
|---|---|---|
| Compound 35 | 20 | mg |
| Lactose | 143.4 | mg |
| Potato starch | 30 | mg |
| Hydroxypropylcellulose | 6 | mg |
| Magnesium stearate | 0.6 | mg |
| | 200 | mg |

### Example 67 (Formulation Example 5)

### Tablets (Compound 37)

The tablets (containing 20 mg/tablet of active ingredient) are obtained by using Compound 37 (40 g) in the same manner as that in Formulation Example 1.

### Formulation

| | | |
|---|---|---|
| Compound 37 | 20 | mg |
| Lactose | 143.4 | mg |
| Potato starch | 30 | mg |
| Hydroxypropylcellulose | 6 | mg |
| Magnesium stearate | 0.6 | mg |
| | 200 | mg |

### Example 68 (Formulation Example 6)

### Injection (Compound 39)

Injection having the following composition is prepared in a conventional manner. Compound 39 (1 g) and D-mannitol (5 g) are added to distilled water for injection and mixed, and hydrochloric acid and aqueous sodium hydroxide are added to the mixture to adjust the mixture to pH 6, and then the total volume is made 1000 mL with distilled water for injection. The resulting mixture is aseptically filled in glass vials in a volume of 2 mL each to obtain injection (containing 2 mg/vial of the active ingredient).

### Formulation

| | | |
|---|---|---|
| Compound 39 | 2 | mg |
| D-Mannitol | 10 | mg |
| Hydrochloric acid | Optimum | amount |
| Aqueous sodium hydroxide | Optimum | amount |
| Distilled water for injection | Optimum | amount |
| | 2.00 | mL |

### Industrial Applicability

The present invention provides a therapeutic and/or preventive agent for diseases involving cell proliferation such as tumors, restenosis, cardiac hypertrophy, and immunologic diseases, which comprises a thiadiazoline derivative or a pharmacologically acceptable salt thereof as an active ingredient. A thiadiazoline derivative or a pharmacologically acceptable salt thereof which is useful for therapeutic treatment of the aforementioned diseases involving cell proliferation is also provided.

## Claims

1. An antitumor agent comprising a thiadiazoline derivative represented by the general formula (I), or a pharmacologically acceptable salt thereof as an active ingredient: 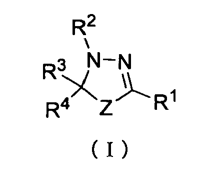 <wherein Z represents a sulfur atom or -S(=O)-, R¹ represents substituted or unsubstituted lower alkyl, substituted or unsubstituted lower alkenyl, substituted or unsubstituted lower alkynyl, substituted or unsubstituted aryl, a substituted or unsubstituted aromatic heterocyclic group, or -C(=W)R⁵ {wherein W represents an oxygen atom or a sulfur atom, and R⁵ represents a hydrogen atom, substituted or unsubstituted lower alkyl, substituted or unsubstituted lower alkenyl,
-YR⁶ (wherein Y represents an oxygen atom or a sulfur atom, and R⁶ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group), or
-NR⁷R⁸ [wherein R⁷ and R⁸ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, - OR⁹ (wherein R⁹ has the same meaning as that of the aforementioned R⁶), or -NR¹⁰R¹¹ (wherein R¹⁰ and R¹¹ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group, or R¹⁰ and R¹¹ are combined together with the adjacent nitrogen atom to form a substituted or unsubstituted heterocyclic group), or R⁷ and R⁸ are combined together with the adjacent nitrogen atom to form a substituted or unsubstituted heterocyclic group]},
R² represents a hydrogen atom, substituted or unsubstituted lower alkyl, or
-C(=W¹)R¹² [wherein W¹ represents an oxygen atom or a sulfur atom, R¹² represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, -Y¹R¹³ (wherein Y¹ represents an oxygen atom or a sulfur atom, and R¹³ represents substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group), or -NR¹⁴R¹⁵ (wherein R¹⁴ and R¹⁵ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group, or R¹⁴ and R¹⁵ are combined together with the adjacent nitrogen atom to form a substituted or unsubstituted heterocyclic group)],
R³ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group, and
R⁴ represents substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group,
or R³ and R⁴ are combined together to represent
-(CR^{16A}R^{16B})ₘ₁-Q-(CR^{16C}R^{16D})ₘ₂- {wherein Q represents a single bond, substituted or unsubstituted phenylene, or cycloalkylene, m1 and m2 are the same or different, and each represents an integer of 0 to 4, with the proviso that m1 and m2 are not 0 at the same time,
R^{16A}, R^{16B}, R^{16C} and R^{16D} are the same or different, and represent a hydrogen atom, halogen, substituted or unsubstituted lower alkyl, -OR¹⁷ [wherein R¹⁷ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, -CONR¹⁸R¹⁹ (wherein R¹⁸ and R¹⁹ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group, or R¹⁸ and R¹⁹ are combined together with the adjacent nitrogen atom to form a substituted or unsubstituted heterocyclic group),
-SO₂NR²⁰R²¹ (wherein R²⁰ and R²¹ have the same meanings as those of the aforementioned R¹⁸ and R¹⁹, respectively), or -COR²² (wherein R²² represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group)], -NR²³R²⁴ [wherein R²³ and R²⁴ are the same or different, and represent a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, -COR²⁵ (wherein R²⁵ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, a substituted or unsubstituted heterocyclic group, substituted or unsubstituted lower alkoxy, substituted or unsubstituted aryloxy, amino, substituted or unsubstituted lower alkylamino, di-(substituted or unsubstituted lower alkyl)amino, or substituted or unsubstituted arylamino), or -SO₂R²⁶ (wherein R²⁶ represents substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group), or R²³ and
R²⁴ are combined together with the adjacent nitrogen atom to form a substituted or unsubstituted heterocyclic group], or -CO₂R²⁷ (wherein R²⁷ represents a hydrogen atom, substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group), or R^{16A} and R^{16B}, or R^{16C} and R^{16D} are combined together to represent an oxygen atom, and when m1 or m2 is an integer of 2 or more, any of R^{16A}, R^{16B}, R^{16C} and R^{16D} may be the same or different, and any two of R^{16A}, R^{16B}, R^{16C} and R^{16D} which are bound to the adjacent two carbon atoms may combine together to form a bond}>.

2. The antitumor agent according to claim 1, wherein R¹ is substituted or unsubstituted lower alkynyl, substituted or unsubstituted aryl, or a substituted or unsubstituted aromatic heterocyclic group.

3. The antitumor agent according to claim 1, wherein R¹ is substituted or unsubstituted lower alkyl, substituted or unsubstituted lower alkenyl, or -C(=W)R⁵ (wherein W and R⁵ have the same meanings as those mentioned above).

4. The antitumor agent according to claim 1, wherein R¹ is substituted or unsubstituted aryl, or a substituted or unsubstituted aromatic heterocyclic group.

5. The antitumor agent according to claim 1, wherein R¹ is substituted or unsubstituted aryl.

6. The antitumor agent according to claim 1, wherein R¹ is substituted or unsubstituted lower alkynyl.

7. The antitumor agent according to claim 1, wherein R¹ is substituted or unsubstituted lower alkyl, or substituted or unsubstituted lower alkenyl.

8. The antitumor agent according to any one of claims 1 to 7, wherein R² is a hydrogen atom, substituted or unsubstituted lower alkyl, or -C(=W¹)R¹² (wherein W¹ and R¹² have the same meanings as those mentioned above, respectively).

9. The antitumor agent according to any one of claims 1 to 7, wherein R² is -C(=W¹)R¹² (wherein W¹ and R¹² have the same meanings as those mentioned above, respectively).

10. The antitumor agent according to claim 8 or 9, wherein R¹² is substituted or unsubstituted lower alkyl, substituted or unsubstituted lower alkenyl, substituted or unsubstituted lower alkynyl, or substituted or unsubstituted cycloalkyl.

11. The antitumor agent according to claim 8 or 9, wherein R¹² is substituted or unsubstituted lower alkyl.

12. The antitumor agent according to claim 8 or 9, wherein R¹² is lower alkyl.

13. The antitumor agent according to any one of claims 8 to 12, wherein W¹ is an oxygen atom.

14. The antitumor agent according to any one of claims 1 to 13, wherein R³ is substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group.

15. The antitumor agent according to any one of claims 1 to 13, wherein R³ is substituted or unsubstituted lower alkyl.

16. The antitumor agent according to any one of claims 1 to 13, wherein R³ is substituted lower alkyl.

17. The antitumor agent according to any one of claims 1 to 16, wherein R⁴ is substituted or unsubstituted cycloalkyl, substituted or unsubstituted aryl, or a substituted or unsubstituted heterocyclic group.

18. The antitumor agent according to any one of claims 1 to 16, wherein R⁴ is substituted or unsubstituted aryl, or a substituted or unsubstituted heterocyclic group.

19. The antitumor agent according to any one of claims 1 to 16, wherein R⁴ is substituted or unsubstituted phenyl, or substituted or unsubstituted thienyl.

20. The antitumor agent according to any one of claims 1 to 13, wherein R³ and R⁴ are combined together to represent -(CR^{16A}R^{16B})ₘ₁-Q-(CR^{16C}R^{16D})ₘ₂- (wherein Q, R^{16A}, R^{16B}, R^{16C}, R^{16D}, m1 and m2 have the same meanings as those mentioned above, respectively).

21. The antitumor agent according to any one of claims 1 to 13, wherein R³ and R⁴ are combined together to represent -(CH₂)ₘ₁-Q-(CH₂)ₘ₂- (wherein Q, m1 and m2 have the same meanings as those mentioned above, respectively).

22. The antitumor agent according to claim 20 or 21, wherein Q is substituted or unsubstituted phenylene.

23. A mitotic kinesin Eg5 inhibitor comprising the thiadiazoline derivative or a pharmacologically acceptable salt thereof according to any one of claims 1 to 22 as an active ingredient.

24. A thiadiazoline derivative represented by the formula (IA) or a pharmacologically acceptable salt thereof: 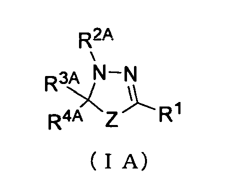 {wherein Z has the same meaning as that mentioned above, R¹ has the same meaning as that mentioned above,
(A) when R¹ is substituted or unsubstituted lower alkyl, substituted or unsubstituted lower alkenyl, or -C(=W)R⁵ (wherein W and R⁵ have the same meanings as those mentioned above, respectively), R^{2A}, R^{3A} and R^{4A} have the same meanings as those of the aforementioned R², R³ and R⁴ (with proviso that Z^{A} is a sulfur atom, R¹ is benzyl, R^{2A} is acetyl, one of R³ and R^{4A} is methyl, and the other of R³ and R^{4A} is not 2-oxopropyl), respectively
(B) when R¹ is substituted or unsubstituted lower alkynyl, or a substituted or unsubstituted aromatic heterocyclic group, R^{2A} and R^{3A} have the same meanings as those of the aforementioned R² and R³, respectively, and R^{4A} represents substituted or unsubstituted aryl, or a substituted or unsubstituted heterocyclic group, and
(C) when R¹ is substituted or unsubstituted aryl, R^{2A} represents -C(=W)R¹² (wherein W and R¹² have the same meanings as those mentioned above, respectively), R^{3A} represents -(CH₂)ₖNHSO₂R^{3B} [wherein k represents an integer of 1 to 6, and R^{3B} represents substituted or unsubstituted lower alkyl, substituted or unsubstituted lower alkenyl, substituted or unsubstituted lower alkynyl, or -NR^{7B}R^{8B} (wherein R^{7B} and R^{8B} have the same meanings as those of the aforementioned R⁷ and R⁸, respectively)], -(CH₂)ₖNR^{7C}R^{8C} (wherein k has the same meaning as that mentioned above, and R^{7C} and R^{8C} have the same meanings as those of the aforementioned R⁷ and R⁸, respectively), or -(CH₂)ₖNHC(=O)R^{7D} (wherein k has the same meaning as that mentioned above, and R^{7D} has the same meaning as that of the aforementioned R⁷), and R^{4A} has the same meaning as that of the aforementioned R⁴}.

25. The thiadiazoline derivative or a pharmacologically acceptable salt thereof according to claim 24, wherein Z is a sulfur atom.

26. The thiadiazoline derivative or a pharmacologically acceptable salt thereof according to claim 24 or 25, wherein R¹ is substituted or unsubstituted lower alkynyl, substituted or unsubstituted aryl, or a substituted or unsubstituted aromatic heterocyclic group.

27. The thiadiazoline derivative or a pharmacologically acceptable salt thereof according to claim 24 or 25, wherein R¹ is substituted or unsubstituted aryl.

28. The thiadiazoline derivative or a pharmacologically acceptable salt thereof according to claim 24 or 25, wherein R¹ is substituted or unsubstituted phenyl.

29. The thiadiazoline derivative or a pharmacologically acceptable salt thereof according to claim 24 or 25, wherein R¹ is substituted or unsubstituted lower alkynyl.

30. The thiadiazoline derivative or a pharmacologically acceptable salt thereof according to claim 24 or 25, wherein R¹ is substituted lower alkyl.

31. The thiadiazoline derivative or a pharmacologically acceptable salt thereof according to claim 24 or 25, wherein R¹ is -C(=W)R⁵ (wherein W and R⁵ have the same meanings as those mentioned above, respectively).

32. The thiadiazoline derivative or a pharmacologically acceptable salt thereof according to claim 31, wherein W is an oxygen atom.

33. The thiadiazoline derivative or a pharmacologically acceptable salt thereof according to claim 31 or 32, wherein R⁵ is -NR⁷R⁸ (wherein R⁷ and R⁸ have the same meanings as those mentioned above, respectively).

34. The thiadiazoline derivative or a pharmacologically acceptable salt thereof according to any one of claims 24 to 33, wherein R^{2A} is -C(=O)R¹² (wherein R¹² have the same meanings as those mentioned above).

35. The thiadiazoline derivative or a pharmacologically acceptable salt thereof according to claim 34, wherein R¹² is lower alkyl.

36. The thiadiazoline derivative or a pharmacologically acceptable salt thereof according to any one of claims 24 to 35, wherein R^{3A} is substituted or unsubstituted lower alkyl.

37. The thiadiazoline derivative or a pharmacologically acceptable salt thereof according to any one of claims 24 to 35, wherein R^{3A} is -(CH₂)ₖNHSO₂R^{3B} (wherein k and R^{3B} have the same meanings as those mentioned above, respectively), -(CH₂)ₖNR^{7C}R^{8C} (wherein k, R^{7C} and R^{8C} have the same meanings as those mentioned above, respectively), or -(CH₂)ₖNHC(=O)R^{7D} (wherein k and R^{7D} have the same meanings as those mentioned above, respectively).

38. The thiadiazoline derivative or a pharmacologically acceptable salt thereof according to any one of claims 24 to 35, wherein R^{3A} is -(CH₂)ₖNHSO₂R^{3B} (wherein k and R^{3B} have the same meanings as those mentioned above, respectively).

39. The thiadiazoline derivative or a pharmacologically acceptable salt thereof according to any one of claims 24 to 38, wherein R^{4A} is substituted or unsubstituted aryl, or a substituted or unsubstituted aromatic heterocyclic group.

40. The thiadiazoline derivative or a pharmacologically acceptable salt thereof according to any one of claims 24 to 38, wherein R^{4A} is substituted or unsubstituted aryl.

41. The thiadiazoline derivative or a pharmacologically acceptable salt thereof according to any one of claims 24 to 38, wherein R^{4A} is substituted or unsubstituted phenyl, or substituted or unsubstituted thienyl.

42. The thiadiazoline derivative or a pharmacologically acceptable salt thereof according to any one of claims 24 to 38, wherein R^{4A} is phenyl.

43. A medicament comprising the thiadiazoline derivative or a pharmacologically acceptable salt thereof according to any one of claims 24 to 42 as an active ingredient.

44. A mitotic kinesin Eg5 inhibitor comprising the thiadiazoline derivative or a pharmacologically acceptable salt thereof according to any one of claims 24 to 42 as an active ingredient.

45. A therapeutic agent for a disease involving cell proliferation comprising the thiadiazoline derivative or a pharmacologically acceptable salt thereof according to any one of claims 24 to 42 as an active ingredient.

46. An antitumor agent comprising the thiadiazoline derivative or a pharmacologically acceptable salt thereof according to any one of claims 24 to 42 as an active ingredient.

47. A method for therapeutic and/or preventive treatment of a malignant tumor which comprises administering an effective amount of the thiadiazoline derivative or a pharmacologically acceptable salt thereof according to any one of claims 1 to 22.

48. A method for inhibiting a mitotic kinesin Eg5 which comprises administering an effective amount of the thiadiazoline derivative or a pharmacologically acceptable salt thereof according to any one of claims 1 to 22.

49. Use of the thiadiazoline derivative or a pharmacologically acceptable salt thereof according to any one of claims 1 to 22 for the manufacture of an antitumor agent.

50. Use of the thiadiazoline derivative or a pharmacologically acceptable salt thereof according to any one of claims 1 to 22 for the manufacture of a mitotic kinesin Eg5 inhibitor.

51. A method for inhibiting a mitotic kinesin Eg5 which comprises administering an effective amount of the thiadiazoline derivative or a pharmacologically acceptable salt thereof according to any one of claims 24 to 42.

52. A method for therapeutic and/or preventive treatment of a disease involving cell proliferation which comprises administering an effective amount of the thiadiazoline derivative or a pharmacologically acceptable salt thereof according to any one of claims 24 to 42.

53. A method for therapeutic and/or preventive treatment of a malignant tumor which comprises administering an effective amount of the thiadiazoline derivative or a pharmacologically acceptable salt thereof according to any one of claims 24 to 42.

54. Use of the thiadiazoline derivative or a pharmacologically acceptable salt thereof according to any one of claims 24 to 42 for the manufacture of a mitotic kinesin Eg5 inhibitor.

55. Use of the thiadiazolirie derivative or a pharmacologically acceptable salt thereof according to any one of claims 24 to 42 for the manufacture of a therapeutic agent for a disease involving cell proliferation.

56. Use of the thiadiazoline derivative or a pharmacologically acceptable salt thereof according to any one of claims 24 to 42 for the manufacture of an antitumor agent.
